# EUROPEAN PATENT APPLICATION

(11) **EP 2 682 481 A2**
(43) Date of publication of application: **08.01.2014**
(21) Application number: 13179764.9
(22) Date of filing: 03.08.2006
(51) Int. Cl.: C12Q 1/68

(54) **Methods for characterizing and treating cognitive impairment in aging and disease**

(30) Priority: 03.08.2005 US 705642 P; 03.08.2005 US 705560 P; 03.08.2005 US 705559 P; 03.08.2005 US 705702 P; 03.08.2005 US 705691 P; 03.08.2005 US 705686 P; 03.08.2005 US 705700 P; 03.08.2005 US 705698 P; 03.08.2005 US 705662 P; 03.08.2005 US 705697 P; 03.08.2005 US 705659 P; 03.08.2005 US 705683 P; 03.08.2005 US 705699 P; 03.08.2005 US 705417 P; 03.08.2005 US 705418 P; 03.08.2005 US 705511 P; 03.08.2005 US 705701 P; 03.08.2005 US 705661 P; 03.08.2005 US 705594 P; 03.08.2005 US 705695 P; 03.08.2005 US 705512 P; 03.08.2005 US 705416 P; 03.08.2005 US 705591 P; 03.08.2005 US 705703 P
(62) Divisional of application: 06800760.8
(71) Applicant: THE JOHNS-HOPKINS UNIVERSITY, Baltimore, MD 21218 (US)
(72) Inventor: Gallagher, Michela, Baltimore, MD 21218 (US); Haberman, Rebecca, Baltimore, MD 21218 (US)
(74) Representative: Evenson, Jane Harriet

(57) **Abstract**

This invention provides methods for identifying genes associated with cognitive impairment and for identifying compounds useful in the treatment of cognitive impairment. The methods can in particular be used to identify genes associated with, and compounds useful in treating, cognitive impairment in ageing.

## Description

### Background of the Invention

A major focus of the field of aging and dementia is the investigation of the causes of cognitive impairment. Various conditions, such as dementias (e.g., Alzheimer's Disease, Lewy body dementia, vascular dementia, and HIV associated dementia), neurodegenerative diseases (e.g., Huntington's Disease, Parkinson's Disease, amyotrophic lateral sclerosis), neurological disorders (e.g., schizophrenia, depression), and age-related conditions (e.g., Mild Cognitive Impairment, Age-Related Cognitive Decline), are associated with cognitive impairment. As the understanding of cognitive impairment increases, so does the need to develop sensitive methods to both detect and to treat such impairment.

Accumulating evidence suggests that there is a neurogenetic component to cognitive impairment. For example, changes in the mammalian brain appear to parallel alterations in distinct learning and memory processes. *See*, e.g., Albert, Philos. Trans. R. Soc. Lond. B. Biol. Sci. 352(1362):1703-09 (1997). In particular, alterations in the hippocampal formation are among the most prominent and consistent features observed in age-related cognitive impairment. Gallagher, Philos. Trans. R. Soc. Lond. B. Biol. Sci. 352(1362):1711-7 (1997). Changes in the aged hippocampus also parallel, to some extent, the hippocampal changes observed in other conditions associated with cognitive impairment, such as Alzheimer's Disease. Whitehouse et al., Science. 215(4537):1237-39 (1982); Bartus et al., Science. 17(4558):408-14 (1982); Rapp and Heindel, Curr. Opin. Neurol. 7(4):294-8 (1994). Thus, the effects of aging on hippocampal function may be related at some level to disease processes in progressive neurodegenerative illnesses.

The mammalian hippocampus is a structure that contains distinct populations of neurons organized into separate anatomical subregions. These subregions include, for example, the dentate gyrus (DG) and the Cornu Ammonis (CA) subfields CA1 and CA3. Each hippocampal subregion is characterized by a unique molecular profile. Lein et al. J Neurosci. 24(15):3879-89 (2004). These different profiles may account for the differential vulnerability of these regions to various mechanisms of cognitive impairment. *See, e.g.,* Small et al. Proc. Natl. Acad. Sci. USA. 101(18):7181-6 (2004).

Considerable research has examined the correlation between changes in cognitive function and neuronal changes in specific hippocampal subregions. This research suggests that the specific nature and extent of hippocampal damage parallels in some cases the degree and type of cognitive impairment. See, Jarrard, Behav. Neural. Biol. 60(1):9-26 (1993). For example, synaptic alterations specific to the CA1 region correlate with age-related impairments in cellular responsiveness to glutamate receptor stimulation. Barnes et al., Hippocampus. 2(4):457-68 (1992). Likewise, individual differences in spatial learning ability correlate with expression levels of NR1, an N-methyl-D-Aspartate (NMDA) receptor subunit, selectively in CA3 neurons. Adams et al., J. Comp. Neurol. 432(2):230-43 (2001). And, levels of synaptic markers in the DG correlate with individual levels of cognitive impairment in spatial learning capacity among aged rats. Smith et al., J. Neurosci. 20(17):6587-93 (2000). These correlations do not, however, establish that the neuronal changes cause the observed functional changes. Just as, importantly, changes in the hippocampus and cognitive function do not occur solely as a result of aging *per se.* Thus, comparisons based on chronological age alone often fail to capture the hippocampal changes that correlate with individual levels of cognitive impairment.

Although some impairment in both cognitive function and hippocampal integrity may be a normal consequence of healthy aging, a significant population of elderly adults experiences a decline in cognitive ability that exceeds normal development. Thus, the effect of aging itself on cognition, in the absence of dementia or disease, is important for defining the boundary between illness and normal aging.

Heterogeneous patterns of progressive cognitive impairment are characteristic of mammalian test populations, e.g., aged humans and aged laboratory animals. The increasing preponderance of 'individual differences' in cognitive impairment has been used to characterize normal mammalian aging itself. Baxter and Gallagher, Neurobiol. Aging. 17(3):491-95 (1996).

There is, therefore, a need to elucidate the molecular basis of and to treat cognitive impairment, both in aging and in disease. There is also a need to understand the neurogenetic components of cognitive impairment and to develop a range of treatment options for individuals with varying levels of impaired cognition.

The examination of changes in expressed gene products in the hippocampus of the mammalian brain, as described in this invention, may serve to elucidate the genetic or molecular basis of cognitive impairment. Moreover, the examination of changes in expressed gene products in the aged hippocampus may serve to elucidate the genetic or molecular basis of cognitive impairment in aging. The identification of genes, or a plurality of genes, that are associated with cognitive impairment, and particularly that are associated with age-related cognitive impairment, would also allow the identification of compounds for treating such impairment.

### Summary of the Invention

This invention provides methods for identifying genes and their expressed gene products, e.g., RNAs, polypeptides, peptides and proteins, associated with cognitive impairment and for identifying compounds useful in the treatment of cognitive impairment. The methods of this invention can, in particular, be used to identify genes and expressed gene products associated with and compounds useful in treating cognitive impairment in aging.

This invention provides a method of identifying a gene or a plurality of genes associated with cognitive impairment by determining the abundance of expressed gene products in one or more of CA1, CA3 and DG hippocampal tissue of mammals in a population of aged mammals with cognitive impairment (aged impaired or "AI"), aged mammals without cognitive impairment (aged unimpaired or "AU") and young mammals ("Y"), and selecting the genes based on a significant changeincrease or decrease -- in the relative abundance of the gene's expressed gene product in the AI population relative to the combined AU and Y populations or based on a significant change -- increase or decrease -- in the relative abundance of the gene's expressed gene product in the AU population relative to the combined AI and Y populations. The AI genes and their expressed gene products may be related to and markers of cognitive impairment and unhealthy aging. Conversely the AU genes and their expressed genes products may be related to and markers of adaptive aging to preserve and protect cognitive function.

One preferred gene in accordance with this invention, the abundance of whose expressed gene product in the AI population relative to the expressed gene product in the combined AU and Y populations is decreased is the GABA-A α5 receptor gene (corresponding to GENBANK® accession number NM_017295), as shown in Example 36 and Table 36 (CA3 AI ANOVA decrease) and its homologues.

This invention provides a method of correlating the changed abundance -- increase or decrease -- of a selected gene's or its homologue's expressed gene product with the level of cognitive impairment or age-related cognitive impairment in each mammal of the AU and AI populations and selecting one or more genes, the changed abundance -- increase or decrease -- of whose expressed gene product(s) significantly correlates with the level of cognitive impairment or age-related cognitive impairment in the mammal. Preferably, one gene whose decreased expressed gene product significantly correlates with the level of cognitive impairment is the GABA-A α5 receptor gene (corresponding to GENBANK® accession number NM_017295), as shown in Example 10 and Table 10 (CA3 AI ANOVA negative correlation).

This invention also provides methods for identifying compounds useful for treating cognitive impairment by determining the abundance or function, either in a mammalian cell or in the hippocampus of a mammal, of the expressed gene product of at least one gene, identified by the above methods of the invention, or listed in Tables 1-48, or their homologues, in the presence or absence of a candidate compound and identifying a compound from among the candidate compounds that significantly changes that abundance or alters the function of those genes or expressed gene products in the appropriate direction as defined herein, either in the mammalian cell or in the hippocampus of the mammal, preferably in the CA1, CA3 or DG hippocampal tissue from which the gene was identified, to whom the candidate compound is administered. The same method may also be used for identifying compounds useful in treating age-related cognitive impairment by testing the candidate compounds in aged mammals. Preferably, compounds useful for treating cognitive impairment are (1) the GABA-A α5 receptor agonist QH-11-066 (1-methyl-7-acetyleno-5-phenyl-1,3-dihydro-benzo[e]-1,4-diazepin-2-one), see, Platt et al., Contribution of α1GABAA and α5GABAA Receptor Subtypes to the Discriminative Stimulus Effects of Ethanol in Squirrel Monkeys, J. Pharmacol. Exp. Ther. 313: 658-667 (2005); (2) S(CH₂)₃OH, corresponding to compound number 44 in Chambers et al., Identification of a Novel, Selective GABAA α5 Receptor Inverse Agonist Which Enhances Cognition, J. Med. Chem. 46:2227-2240 (2003); and (3) 8-ethylthio-3-methyl-5-(1-oxidopyridin-2-yl)-3,4-dihydronaphthalen-1(2H)-one, corresponding to compound number 19 in Szekeres et al., 3,4-Dihydronaphthalen-1(2H)-ones: novel ligands for the benzodiazepine site of alpha5-containing GABAA receptors. Bioorg. Med. Chem. Lett. 14:2871-2875 (2004).

This invention provides methods for identifying a compound useful in the treatment of cognitive impairment by determining the cognitive status of a mammal in the presence or absence of a candidate compound believed to change the abundance of the expressed gene product of at least one gene, identified by the method of this invention, or listed in Tables 1-48, or their homologues, or to alter the function of those genes or expressed gene products in the appropriate direction, as defined herein, and identifying a compound from among the candidate compounds that beneficially alters the cognitive status of the mammal. The same method may also be used to identify compounds useful for treating age-related cognitive impairment by testing candidate compounds in aged mammals.

### Detailed Description of the Invention

Unless otherwise defined herein, scientific and technical terms used in this application shall have the meanings that are commonly understood by those of ordinary skill in the art. Further, unless otherwise required by context, singular terms include pluralities and plural terms include the singular. Generally, nomenclatures used in connection with, and techniques of, cell and tissue culture, molecular biology, cell and cancer biology, neurobiology, neurochemistry, virology, immunology, microbiology, pharmacology, genetics and protein and nucleic acid chemistry, described herein, are those well known and commonly used in the art.

The methods and techniques of the present invention are generally performed, unless otherwise indicated, according to conventional methods well known in the art and as described in various general and more specific references that are cited and discussed throughout the present specification. See, e.g. Ausubel et al., "Current Protocols in Molecular Biology", Greene Publishing Associates (1992, and Supplements to 2003); Cooper and Hausman, "The Cell - A Molecular Approach, 2nd ed.", Sinauer Associates, Inc., Sunderland, MA (2000); Kandel et al., "Principles of Neural Science", McGraw-Hill Medical, New York, N.Y. (2000); Motulsky, "Intuitive Biostatistics", Oxford University Press, Inc. (1995); Lodish et al., "Molecular Cell Biology, 4th ed.", W. H. Freeman & Co., New York (2000); Griffiths et al., "Introduction to Genetic Analysis, 7th ed.", W. H. Freeman & Co., N.Y. (1999); Gilbert et al., "Developmental Biology, 6th ed.", Sinauer Associates, Inc., Sunderland, MA (2000); Sambrook et al., "Molecular Cloning: A Laboratory Manual, 2d ed.", Cold Spring Harbor Laboratory Press, Cold Spring Harbor, N.Y. (1989).

Chemistry terms used herein are used according to conventional usage in the art, as exemplified by "The McGraw-Hill Dictionary of Chemical Terms", Parker S., Ed., McGraw-Hill, San Francisco, C.A. (1985).

All of the above, and any other publications, patents and published patent applications, referred to in this application are specifically incorporated by reference herein. In case of conflict, the present specification, including its specific definitions, will control.

### The Specific Terms Used In The Methods Of This Invention

The following terms, unless otherwise indicated, shall be understood to have the following meanings.

"Abundance" refers to the level of expression, activity or amount of an expressed gene product of a gene or its homologue from another organism. The abundance of an expressed gene product includes, but is not limited to, the level of expression of the function that the gene performs and the way in which it does so, including chemical or structural differences and/or differences in binding or association with other factors. It also includes the amount of the product whatever its source, e.g., expression or direct addition to the tissue, cell or animal. Abundance can be detected by any method useful in quantitatively measuring the expressed gene product. These include, for example, enzymatic assays, binding assays, immunoassays, structural and chemical assays (e.g., which measure modifications such as phosphorylation status, conformational changes).

The methods for measuring the abundance of an expressed gene product according to this invention include, but are not limited to, microarray analysis, macroarray analysis, in situ hybridization histochemistry, fluorescent in situ hybridization (FISH), immunocytochemistry (ICC), immunofluorescence, enzyme linked immunosorbent assay (ELISA), fluorescence polarization immunoassay (FPIA), nephelometric inhibition immunoassay (NIA), immunoprecipitation, quantitative polymerase chain reaction (PCR), RNase protection assay, reverse-transcription PCR, competitive PCR, real-time quantitative PCR (i.e., TaqMan PCR), serial analysis of gene expression (SAGE) analysis, two-dimensional gel electrophoresis, mass spectrometry, MALDI-TOF mass spectrometry, radioimmunoassay (RIA) and blot analysis (e.g. Northern blot, Western blot, protein slot blot, immunoblot, dot blot). If desired, any of the expression and activity assays described above can be used in combination, either sequentially or simultaneously. Such assays can also be partially or completely automated, using methods known in the art.

"Aged" refers to a mammal advanced in years, preferably in or near the latter third of their average lifespan. For example, an aged human would be fifty or more years of age. An aged rat would be fifteen months of age or more.

"Age-related cognitive decline" or "ARCD", or an equivalent construct such as "age-associated memory impairment", refers to a diagnosis of mild memory deficit that is not expected to worsen considerably over time. As used herein, ARCD can also be defined as Stage 2 on the Global Deterioration Scale (GDS). The GDS is a seven-point rating system of cognitive and functional capabilities. It is widely used for rating cognitive performance in older adults, with scores ranging from normal aging (Stage 1) to severe dementia (Stage 7). Stage 2 is characterized, for example, by the following clinical characteristics: subjective cognitive complaints in the absence of clinically manifest deficit.

"AI" or "aged impaired" refers to one or more aged mammals with cognitive impairment relative to young mammals of the same species. The preferred species used as models of age impairment in this invention is rats. Most preferably, the rat is from an outbred strain. Aged rats naturally segregate into two populations, with about half of the aged rats being AI and about half being aged unimpaired (AU). To identify these populations, the cognitive ability of young and aged rats is measured. Methods for identifying populations of AI rats are known in the art, e.g., Gallagher and Burwell, Neurobiol. Aging 10:691-708 (1989) and an exemplary method is described in the Example. A number of methods for assessing rat cognitive ability are also known, e.g., the Barnes circular maze, the radial arm maze, the Morris water maze, delayed alternation (delayed nonmatch-to-sample), novel object recognition, conditioned avoidance, and fear conditioning.

"ANOVA" or "analysis of variance" refers to a statistical technique broadly applicable for analyzing data. An ANOVA compares the distribution of two or more populations to determine if one or more of the populations are significantly different from the others. The average variance within each of the populations is factored out from the variance between each of the populations before computing the probability of significant differences between the populations. When an ANOVA tests the difference between the means of two (and not more) populations, it is equivalent to a t-test. As used herein, for example, ANOVA is used to test the hypothesis that the abundance of an expressed gene product of a gene or a plurality of genes is different in mammals with cognitive impairment (e.g., the AI population) relative to mammals without cognitive impairment (e.g., the combined AU and Y populations), by computing the probability that significant differences among populations or treatments are too large to be due to chance.

The statistic calculated by an ANOVA is an *F-*ratio, which reveals the significance of the hypothesis that *Y* depends on *X*. It comprises the ratio of two mean-squares: MS[*X*] / MS[ε], which is the sum of squared deviations from the mean *X* or ε divided by the appropriate degrees of freedom. One of skill in the art would use standard tables to determine whether the observed *F*-ratio indicates a significant relationship. When there are two groups, the F-ratio is equivalent to the t as determined by a t-test. As used herein, when the mean abundance of an expressed gene product of a gene or a plurality of genes is being compared between two groups (e.g., between Aged Impaired and Aged Unimpaired with Young) by ANOVA, the F-ratio would be equivalent to the t test statistic, as determined by a t-test.

A "MAS5" statistical analysis produces absolute and comparison analysis results for microarrays and preferably GeneChip microarrays. It is an algorithm for deriving gene expression scores from microarrays based on present or absent calls, where a percentage of transcripts that are considered significantly hybridized to the chip are considered present. The MAS5 analysis also comprises an algorithm, not used in the present invention, that can deduce present, absent and also marginal calls.

A "gcRMA" statistical analysis is another algorithm for deriving gene expression scores from microarrays and preferably GeneChip microarrays. It is an open-source method that is based on robust averaging techniques and sequence-dependent affinity corrections. The robust averaging employed in gcRMA confers a strong immunity to outliers.

"Appropriate direction" refers to the direction of the change in the abundance of the expressed gene product(s) of at least one gene identified by the methods of this invention or listed in Tables 1-48 or their homologues or the change in the function of the gene or expressed gene product. For genes, whose gene products increase in the AI population relative to the combined AU and Y populations, the "appropriate direction" is a decrease or attenuation of function. For genes, whose gene products decrease in the AI population relative to the combined AU and Y population, the "appropriate direction" is an increase or enhancement in function. For genes, whose gene products increase in the AU population relative to the combined AI and Y populations, the "appropriate direction" is an increase or enhancement of function. And, for genes, whose gene products decrease in the AU population relative to the combined AI and Y populations, the "appropriate direction" is a decrease.

"AU" or "aged unimpaired" refers to one or more aged mammals without cognitive impairment relative to young mammals of the same species. The preferred species used as models of age impairment in this invention is rats. Most preferably, the rat is from an outbred strain. As noted above, aged rats naturally segregate into two populations, with about half of the aged rats being AI and about half being AU. To identify these populations, the cognitive ability of young and aged rats can be measured. Methods for identifying populations of AU rats are known in the art, e.g., Gallagher and Burwell, Neurobiol. Aging 10:691-708 (1989), and an exemplary method is described in the Example. A number of methods for assessing rat cognitive ability are known, e.g., the Barnes circular maze, the radial arm maze, the Morris water maze, delayed alternation (delayed nonmatch-to-sample), novel object recognition, conditioned avoidance, and fear conditioning.

"Beneficially alters" refers to the state of promoting, improving, or preserving cognitive function, or of alleviating or attenuating cognitive decline. A beneficial alteration in accordance with this invention also includes the alleviation or amelioration of one or more manifestations of cognitive impairment, or the delay of onset or slowing of the progression of cognitive impairment. A beneficial alteration of this invention includes, but is not limited to, a change in cognitive function sufficient to result in an improved score in a test of cognitive function; the improvement of cognitive function in a subject with impaired cognitive function so that it more closely resembles the function of a control subject, preferably, e.g., a young subject or an aged unimpaired subject; the improvement over an aged cognitively impaired subject or population; and the preservation of cognitive function over time such that it does not decline or fall below the level observed in the subject upon first presentation or diagnosis.

"Candidate compound" or "compound" means a pharmaceutical, chemical or other composition of matter with known or unknown physiological effects. A compound can be any natural or synthetic agent made up of one or more elements, including, but not limited to, a small molecule, peptide, polypeptide, peptidomimetic, carbohydrate, lipid, protein, glycoprotein, lipoprotein, nucleic acid, and antibody. A compound may or may not have been characterized for its target, or mode of action, in cells or animals prior to its use in the methods of this invention.

As used herein, the compound that is identified and selected from among one or more candidate compounds as being useful in treating cognitive impairment refers to a compound that is capable of altering the abundance -- increase or decrease -- of the expressed gene product of at least one gene identified in accordance with this invention, one or more genes listed in Tables 1-48, or their homologues in the appropriate direction, as defined herein, i.e., in a manner consistent with the beneficial treatment of cognitive function or that is capable of beneficially altering the cognitive status of a mammal to whom it is administered. Preferably, the compound crosses the blood-brain barrier. More preferably, the compound is orally bioavailable.

"Cognitive function" or "cognitive status" refers to any higher order intellectual brain process or brain state, respectively, involved in learning and memory including, but not limited to, attention, information acquisition, information processing, working memory, short-term memory, long-term memory, anterograde memory, retrograde memory, memory retrieval, discrimination learning, decision-making, inhibitory response control, attentional set-shifting, delayed reinforcement learning, reversal learning, the temporal integration of voluntary behavior, and expressing an interest in one's surroundings and self-care.

"Cognitive impairment" or "CI" or an equivalent construct, such as "impaired cognitive function" or "cognitive decline", refers to a deficit or reduction in cognitive status or cognitive function, as defined above, compared to that same function in an age-matched control subject or more usually a population. Cognitive impairment may be observed as a consequence of aging, as well as in various diseases and conditions, including but not limited to, Alzheimer's Disease, Lewy body dementia, vascular dementia, HIV associated dementia, Huntington's Disease, Parkinson's Disease, amyotrophic lateral sclerosis, schizophrenia, depression, MCI, and ARCD.

"Expressed gene product" refers to any form of expression of a gene that can be detected and measured, for example, RNA, amino acid, peptide, polypeptide or protein. It also includes the product itself, whether or not it was actually derived from expression in the cell or tissues of an animal.

"Hippocampal-dependent function" refers to a cognitive function, more specifically a learning or memory process, that includes the encoding and acquisition of memories for specific facts and events and episodes of experiences. Hippocampal-dependent function includes the processing of memory representations and the maintenance of memories. Hippocampal-dependent function in mammals includes, for example, spatial memory acquisition, long-term spatial memory, and spatial memory retrieval. A mammal with impaired hippocampal-dependent function may display, e.g., anterograde amnesia for newly acquired facts and events, including maze-specific information in a spatial water maze.

"Hippocampal formation" or "hippocampus" or "hippocampal tissue" refers to the whole or part of the hemispheric structure in the brain folded into the ventromedial surface of the temporal lobe, caudal to the amygdaloid complex. Hippocampal tissue comprises hippocampal cells from, without limitation, the CA1, CA3 and DG subregions. Hippocampal cells include, but are not limited to, pyramidal cells of the CA subregions and granule cells of the DG subregion.

"Homologue" refers to a gene that has the same origin and functions in two or more species. Preferably, a mammal's homologue of a gene, as identified by the method of the invention, refers to the mammal's equivalent of a gene identified in another mammalian species, e.g., the genes encoding human and rat growth hormones.

"Level of cognitive impairment" refers to a measure of the degree of cognitive impairment observed in a mammal. In humans, the level of cognitive impairment may be measured by various neuropsychological tests, alone or in combination, including, but not limited to, the Alzheimer's Disease Assessment Scale-cognitive subscale (ADAS-cog); Global Deterioration Scale (GDS); the clinical global impression of change scale (CIBIC-plus scale); the Alzheimer's Disease Cooperative Study Activities of Daily Living Scale (ADCS-ADL); the Mini Mental State Exam (MMSE); the Neuropsychiatric Inventory (NPI); the Clinical Dementia Rating Scale (CDR); the Rey Auditory Verbal Learning Test (AVLT), Logical Memory Subtest of the revised Wechsler Memory Scale (WMS-R); the New York University (NYU) Paragraph Recall Test the Cambridge Neuropsychological Test Automated Battery (CANTAB) or the Sandoz Clinical Assessment-Geriatric (SCAG).

In non-human mammalian models, for example, a rat or non-human primate model, the level of cognitive function may be measured by methods including, but not limited to, using a maze in which subjects use spatial information (e.g., Morris water maze, Barnes circular maze, elevated radial arm maze, elevated plus maze, T-maze and others), recognition tests using odor and novel objects, conditioning tests (e.g., fear conditioning, discrimination tasks, active avoidance, illuminated open-field, two-compartment exploratory test, second and third order conditioning tasks), and tests of higher level executive function (e.g., serial reaction time tests, delayed match and non-match to sample, and stimulus-reward associations including choices involving delayed reinforcement).

In addition, the level of cognitive function may be measured in mammals, including humans, using neuroimaging techniques, e.g., Positron Emission Tomography (PET), magnetic resonance imaging (MRI), functional magnetic resonance imaging (fMRI), Single Photon Emission Computed Tomography (SPECT), or any other imaging technique that allows one to measure brain function. The level of cognitive function in aging can be tested by any of the above methods using aged mammals.

"Microarray" or "macroarray" refers to an array (i.e. a matrix) that consist of a surface to which probes (e.g., cDNAs, mRNAs, oligonucleotides, peptides, polypeptides, proteins, antibodies or their equivalents) that correspond or are complementary to in sequence or bind to expressed gene products are bound at known or addressable positions. Each position represents a discrete binding site, e.g., a nucleic acid or nucleic acid analogue, to which a particular expressed gene product can specifically bind. Each array typically, but not necessarily, possesses binding sites for products of most or almost all of the expressed gene products in the mammal's genome. It may also be a representative of such genome. A microarray has a higher density of individual probe species or binding sites per area than a macroarray. A nucleic acid or analogue of a binding site can be, e.g., a synthetic oligomer, a full-length cDNA, a less-than full-length cDNA, an expressed sequence tag (EST) as part of a cDNA molecule, or a gene fragment, e.g., an oligonucleotide corresponding to an overlapping portion of a gene. Preferably, oligonucleotide probes are used. Most preferably, the Affymetrix RAT genome 230-2 array (or an equivalent) is used. The RAT genome 230-2 array contains more than 31,000 probe sets to transcripts from approximately 28,000 genes.

"Mild Cognitive Impairment" or "MCI" refers to a condition characterized by isolated memory impairment accompanied by no other cognitive abnormality and relatively normal functional abilities. One set of criteria for a clinical characterization of MCI specifies the following characteristics: (1) memory complaint (as reported by patient, informant, or physician), (2) normal activities of daily living (ADLs), (3) normal global cognitive function, (4) abnormal memory for age (defined as scoring more than 1.5 standard deviations below the mean for a given age), and (5) absence of indicators of dementia (as defined by DSM-IV guidelines). Petersen et al., Srch. Neurol. 56: 303-308 (1999); Petersen, "Mild cognitive impairment: Aging to Alzheimer's Disease." Oxford University Press, N.Y. (2003).

Diagnosis of MCI usually entails an objective assessment of cognitive impairment, which can be garnered through the use of well-established neuropsychological tests, including the Mini Mental State Examination (MMSE), the Cambridge Neuropsychological Test Automated Battery (CANTAB) and individual tests such as Rey Auditory Verbal Learning Test (AVLT), Logical Memory Subtest of the revised Wechsler Memory Scale (WMS-R) and the New York University (NYU) Paragraph Recall Test. See Folstein et al., J Psychiatric Res 12: 189-98 (1975); Robbins et al., Dementia 5: 266-81 (1994); Kluger et al., J Geriatr Psychiatry Neurol 12:168-79 (1999).

"Non-human animal model" refers to a non-human mammal or other animal or organism useful for research.

"Non-parametric test" refers to a statistical test that does not rely on parametric assumptions such as normality. A non-parametric test is used in place of a parametric test when some assumptions about the underlying population are uncertain. In the present invention, a non-parametric test is used when the test or control population is sampled from a non-Gaussian distribution or when a gene's expressed gene product is too high or too low in abundance to be accurately determined.

One of skill in the art would appreciate that non-parametric tests rank the outcome variable from low to high and then analyze the ranks. Preferably, the non-parametric test used for comparing populations in accordance with this invention is selected from the group consisting of a Mann-Whitney U test, a Wilcoxon rank-sum test, a Wilcoxon matched pairs signed-rank test, a Mann-Whitney-Wilcoxon test, a Kruskal-Wallis analysis of variance by ranks, a Friedman two way analysis of variance and a Kolmogorov-Smirnov test. Preferably, the non-parametric test used for assessing the linear association (i.e. correlation) between variables is the Spearman rank correlation coefficient.

"Overexpressed", "overexpression" or "increase" in expression refers to an abundance of an expressed gene product that is higher than the abundance of that same product under other conditions or in other cells or tissues. Overexpression or increased expression may be effected, for example, by one or more structural changes to the gene's encoding nucleic acid or encoded polypeptide sequence (e.g., primary nucleotide or amino acid changes or post-transcriptional modifications such as phosphorylation), altered gene regulation (e.g., in the promoters, regulators, repressors or chromatin structure of the gene), a chemical modification, an altered association with itself or another cellular component, an altered subcellular localization, a modification which causes higher levels of activity through association with other molecules in the cell (e.g., attachment of a targeting domain) and the like.

"Parametric test" refers to a statistical test that requires a parametric assumption, such as normality, i.e., the assumption that the data are sampled from a Gaussian distribution. Preferably, the parametric test, used when comparing levels of expression across populations, in accordance with this invention, is an analysis of variance (ANOVA). Preferably, the parametric test used for assessing the linear association (i.e. correlation) between variables is the Pearson correlation coefficient.

"P value" is the probability associated with an obtained statistic, such as an F-ratio or a t statistic (see "ANOVA", infra). If the p value is less than the significance level, preferably 0.05, then the result constitutes evidence against the null hypothesis, which states that there is no difference among a set of true population means.

"Plurality" refers to two or more.

"Significant" refers to a confidence level for a measurement being real as opposed to being due to chance, for example, as the result of a random sampling error. In accordance with this invention, significant means a confidence or significance level of at least 95%, wherein p<0.05. More preferably, a significance level of 99%, wherein p<0.01.

"Treatment" refers to the use of a compound to beneficially alter cognitive function in the treated mammal. A treatment, as used herein, is administered by providing a mammal with a compound by any appropriate means and in any appropriate formulation and dosing regime.

"Underexpressed", "underexpression" or "decrease" in expression refers to an abundance of an expressed gene product that is lower than the abundance of the same product under other conditions or in other cells or tissues. Such underexpression or decreased expression may be effected, for example, by one or more structural changes to the gene's encoding nucleic acid or polypeptide sequence (e.g., primary nucleotide or amino acid changes or post-transcriptional modifications such as phosphorylation), altered gene regulation (e.g., in the promoters, regulators, repressors or chromatin structure of the gene), an altered structure (which causes reduced levels of activity), an altered association with itself or another cellular component, an altered subcellular localization, a modification which causes reduced levels of activity through association with other molecules in the cell (e.g., binding proteins which inhibit activity or sequestration) and the like.

"Young" or "Y" refers to one or more adolescent or adult mammals in or near the first third of their average lifespan, at or after the age of sexual maturity and when the hippocampus is fully mature. For example, a young human would be twenty-five years of age or less. A young rat would be ten months of age or less, and preferably at least four months of age.

### Description of Various Claims of the Invention

This invention provides methods of identifying a gene or a plurality of genes associated with cognitive impairment in a mammal.

The method comprises providing three populations of mammals - AI, AU and Y - as defined above. Preferably, the mammals used in identifying the genes of this invention are rats and most preferably outbred rat strains.

CA1, CA3 and DG hippocampal tissue is prepared from each member of the three populations using conventional techniques. The abundance of the expressed gene products in each member of these populations is then determined using standard techniques. These techniques include microarray analysis, macroarray analysis, in situ hybridization histochemistry, fluorescent in situ hybridization (FISH), immunocytochemistry (ICC), enzyme linked immunosorbent assay (ELISA), immunoprecipitation, quantitative polymerase chain reaction (PCR), serial analysis of gene expression (SAGE) analysis, radioimmunoassay (RIA) and blot analysis. More preferably, microarray analysis is used.

Genes or a plurality of genes are then selected based on a significant change -- decrease or increase -- in the gene's expressed gene product (e.g., RNAs, proteins, polypeptides and peptides) in the AI population relative to the combined AU and Y populations or in the AU population relative to the combined AI and Y populations.

The significance of the change in the abundance of the expressed gene product is assessed using conventional statistical tests, including parametric and non-parametric tests. Preferably, the parametric test, ANOVA is used. Among the preferred non-parametric tests useful in this invention are a Mann-Whitney U test, a Wilcoxon rank-sum test, a Wilcoxon matched pairs signed-rank test, a Mann-Whitney-Wilcoxon test, a Kruskal-Wallis analysis of variance by ranks, a Friedman two way analysis of variance, and a Kolmogorov-Smirnov test.

The preferred selected genes or plurality of genes whose expressed gene products have increased abundances in CA1, CA3 or DG tissue are displayed in Tables 1, 3, 6, 7, 25, 27, 30, and 31; 9, 11, 14, 15, 33, 35, 38 and 39; or 17, 19, 22, 23, 41, 43, 46 and 47, respectively. Tables 1, 3, 9, 11, 17, 19, 25, 27, 33, 35, 41 and 43 depict the genes whose expressed gene products are increased in the AI populations relative to the combined AU and Y populations. Tables 6, 7, 14, 15, 22, 23, 30, 31, 38, 39, 46 and 47 depict the genes whose expressed gene products are increased in the AU population relative to the combined AI and Y populations.

The preferred selected genes or plurality of genes whose expressed gene products have decreased abundances in CA1, CA3 or DG tissue are displayed in Tables 2, 4, 5, 8, 26, 28, 29 and 32; 10, 12, 13, 16, 34, 36, 37 and 40; or 18, 20, 21, 24, 42, 44, 45 and 48, respectively. Tables 5, 8, 13, 16, 21, 24, 29, 32, 37, 40, 45 and 48 depict the genes whose expressed gene products are decreased in the AI population relative to the combined AU and Y populations. Tables __ depict the genes whose expressed gene products are decreased in the AU population relative to the combined AI and Y populations.

In some elements of this invention, the change in the abundance -- increase or decrease -- of the expressed gene product(s) of the selected gene or plurality of genes is also correlated with the level of cognitive impairment in each member of the AU and AI populations. The increased or decreased abundances of the expressed gene products of the selected genes or plurality of genes positively or negatively, respectively, correlate with the learning index or cognitive impairment of the animals when poorer learners have higher or lower abundances, respectively. Thus, some of the genes in the above Tables, which depict the genes whose expressed gene products are increased in the AI population relative to the combined AU and Y populations or in the AU population relative to the combined AI and Y populations, will positively and negatively correlate, respectively, with cognitive impairment. *See*, *e.g.,* Tables 1, 6, 9, 14, 17, 22, 25, 30, 33, 38, 41 and 46. Other genes whose expressed gene products are decreased in the AI population relative to the combined AU and Y populations or in the AU population relative to the combined AI and Y populations, will negatively or positively, correlate, respectively, with cognitive impairment. *See, e.g.,* Tables 2, 5, 10, 13, 18, 21, 26, 29, 34, 37, 42 and 45.

A gene or a plurality of genes whose abundances of expressed gene product(s) have a significant positive or negative correlation with the learning index or level of cognitive impairment is then selected. The significance of the correlation is assessed using standard methods, including but not limited to parametric and non-parametric statistical tests. Preferably, the parametric Pearson correlation coefficient or the non-parametric Spearman ranked coefficient is used.

The preferred selected genes or plurality of genes, whose expressed gene products have increased abundances in CA1, CA3 or DG tissue and whose abundances positively or negatively, respectively correlate with the learning scores or cognitive impairment of the AI and AU outbred rat populations, are displayed in Tables 1, 6, 25 and 30; 9, 14, 33, 38; and 17, 22, 41 and 46, respectively.

The preferred selected genes or plurality of genes whose expressed gene products have decreased abundances in CA1, CA3 and DG tissue and whose abundances negatively or positively, respectively correlate with the learning scores or cognitive impairment of the AI and AU outbred rat populations are displayed in Tables 2, 5, 26 and 29; 10, 13, 34 and 37; and 18, 21, 42 and 45, respectively.

The Tables referred to above list the rat genes identified in the preferred claims of this invention by GENBANK® or "UniGene" accession numbers. GENBANK® is the National Institute of Health's genetic sequence database and provides an annotated collection of all publicly available DNA sequences (Nucleic Acids Research 33(Database Issue):D34-D36 (2005)). GENBANK® is part of the International Nucleotide Sequence Database Collaboration, which comprises the DNA DataBank of Japan (DDBJ), the European Molecular Biology Laboratory (EMBL), and GENBANK® at the National Center for Biotechnology Information (NCBI). The actual rat nucleotide sequence of the accession numbers listed herein may be found by searching the GENBANK® database on the "Entrez Nucleotide" portal of the NCBI website. Each UniGene entry comprises a set of transcript sequences that appear to come from the same transcription locus (gene or expressed pseudogene), together with information on protein similarities, gene expression, cDNA clone reagents, and genomic location, and is also accessible on the NCBI website.

Non-rat mammalian homologues, including human homologues, of the selected genes may be determined in a variety of ways. For example, one method is to access the "HomoloGene" portal of the NCBI website. and enter the GENBANK® or UniGene accession number to obtain a listing of all HomoloGene listings showing the homologues in every identified species in which the gene is conserved, including humans. The link on the desired species will produce the Entrez gene listing, which will have a reference ID for the human sequence of the gene. Alternatively, the human homologue may be identified by directly accessing the above-listed Entrez Gene website and entering the rat GENBANK® accession number. A map viewer of the homologous chromosomes is then accessible via the gene link. The map viewer identifies the human homologues and a click on the gene symbol will access several databases, including the Entrez Gene Sequence viewer (sv), which provides genomic, protein and mRNA sequences; HomoloGene (hm), see above; and Consensus CDS (CCDS), another gene identification database that contains several links to protein and sequences.

The genes, identified and selected, by the above methods, and those specific individual genes listed in the Tables above, and their homologues are useful in identifying compounds useful in the treatment of cognitive impairment, and preferably cognitive impairment in aging in accordance with this invention.

One method to identify a compound useful in treating cognitive impairment in mammals, particularly humans, in accordance with this invention, comprises the steps of determining the abundance of an expressed gene product in a mammalian cell of the mammalian cell's homologue(s) of a gene or a plurality of genes identified as described above or listed in Tables 1-48 in the presence or absence of a candidate compound.

The mammalian cells can be any species of cell, e.g., human cells, mouse cells, rat cells, non-human primate cells. The cells can be from any known or established mammalian cell line, such as those available from the American Type Culture Collection (ATCC; Mannassas, VA.), for example, CHO cells, HEK293 cells or COS7 cells. The cells can be in primary cell culture or from a transformed cell line. The cells may naturally express the gene product (i.e., the gene may be wholly endogenous to the cell or multicellular organism) or the cell may be a recombinant cell or transgenic organism comprising one or more recombinantly expressed gene products.

Preferably, the cells are of human origin and more preferably are selected from the group consisting of neuronal cells and glial cells, including but not limited to, oligodendrocytes, astrocytes, microglia, pyramidal cells, granule cells, motoneurons, and Purkinje cells. Methods for culturing neuronal cells are well known see, e.g., Brewer et al., J. Neuroscience Res. 35:567 (1993) [hippocampal neurons]; Walsh et al., Neuroscience 69:915-29 (1995) [suprachiasmatic neurons]; Zoran et al., Dev Biol. 179:212-22 (1996) [motoneuronal cultures]. Preferably, the neuronal cell is a hippocampal cell or line derived from the hippocampus or a hippocampal region. More preferably, the cell is selected from a CA1, CA3 or DG hippocampal cell.

A number of different screening protocols can be utilized to identify compounds that change the abundance of an expressed gene product of a mammalian cell's homologue(s) of a gene or a plurality of genes identified as described above or individually listed in the above Tables in the mammalian cells. In general terms, the methods include culturing the cells in the presence of individual members of a plurality of candidate compounds to identify a compound that significantly changes -- increases or decreases - in the appropriate direction, as defined herein, the abundance of an expressed gene product of a mammalian cell's homologue(s) of a gene or a plurality of genes identified as described above or individually listed in the Tables 1-48.

For example, a compound that decreases the abundance or attenuates the function of an expressed gene product of a gene identified in the methods of this invention or listed in Tables 1, 3, 9, 11, 17, 19, 25, 27, 33, 35, 41 and 43 that was increased in the AI population as compared to the combined AU and Y populations would be useful in treating cognitive impairment. Similarly, a compound that increases the abundance or enhances the function of an expressed gene product of a gene identified in the methods of this invention or listed in Tables 2, 4, 10, 12, 18, 20, 26, 28, 34, 36, 42 and 44 that was decreased in the AI population as compared to the combined AU and Y population would be useful in treating cognitive impairment.

In contrast, a compound that decreases the abundance or attenuates the function of expressed gene products of at least one gene identified in the methods of this invention or listed in Tables 5, 8, 13, 16, 21, 24, 29, 32, 37, 40, 45 and 48 that was decreased in the AU populations as compared to the combined AI and Y populations would be useful in treating cognitive impairment. Similarly, a compound that increases the abundance or enhances the function of one or more expressed gene products of at least one gene identified in the methods of this invention or listed in Tables 6, 7, 14, 15, 22, 23, 30, 31, 38, 39, 46 and 47 that was increased in the AU population as compared to the combined AI and Y populations would be useful in treating cognitive impairment.

One or more compounds from among the candidate compounds are then selected based on the compound's ability to significantly change, in the appropriate direction, as defined herein, the abundance or function of the expressed gene product of a selected gene or plurality of genes in the cell treated with the compound. Preferably, one gene whose decreased abundance of expressed gene product significantly correlates (negatively) with the level of cognitive impairment is the GABA-A α5 receptor gene (corresponding to GENBANK® accession number NM_017295), as shown in Example 10 and Table 10 (CA3 AI ANOVA negative correlation) and its homologues. Preferably, compounds that can significantly increase the abundance or enhance the function of GABA-A α5 protein produced by the gene would be useful for treating cognitive impairment are (1) the GABA-A α5 receptor agonist QH-ii-066 (1-methyl-7-acetyleno-5-phenyl-1,3-dihydro-benzolel-1,4-diazepin-2-one), see, Platt et al., Contribution of α1GABAA and α5GABAA Receptor Subtypes to the Discriminative Stimulus Effects of Ethanol in Squirrel Monkeys, J. Pharmacol. Exp. Ther. 313: 658-667 (2005); (2) S(CH₂)₃OH, corresponding to compound number 44 in Chambers et al., Identification of a Novel, Selective GABAA α5 Receptor Inverse Agonist Which Enhances Cognition, J. Med. Chem. 46:2227-2240 (2003); and (3) 8-ethylthio-3-methyl-5-(1-oxidopyridin-2-yl)-3,4-dihydronaphthalen-1(2H)-one, corresponding to compound number 19 in Szekeres et al., 3,4-Dihydronaphthalen-1(2H)-ones: novel ligands for the benzodiazepine site of alpha5-containing GABAA receptors. Bioorg. Med. Chem. Lett. 14:2871-2875 (2004).

Another method of identifying compounds useful in treating cognitive impairment assays the candidate compounds in mammals, preferably aged mammals, and more preferably aged mammals with cognitive impairment. In this element, the abundance or function of the expressed gene product of the mammal's homologue of a gene or plurality of genes selected in accordance with this invention or individually listed in the above Tables, is measured in hippocampal tissue, preferably CA1, CA3 or DG tissue of the mammal, to whom the candidate compound has been administered. Those compounds that cause a significant change -- increase or decrease - in the appropriate direction, as defined herein, in that abundance or function in mammals treated with the compound are selected.

Compounds are selected that significantly change -- increase or decrease - in the appropriate direction, as defined herein, the abundance or function of the expressed gene product of the mammal's homologue of a gene or plurality of genes selected in the method described above or individually listed in the above Tables in an aged mammal with cognitive impairment who has been treated with the compound, relative to a member of the group consisting of an aged mammal without cognitive impairment, a young mammal, an aged cognitively impaired mammal in the absence of the compound, and two or more of them. Preferably, the selected compound does not alter that abundance or function in a young mammal and/or an aged cognitively unimpaired mammal to whom the candidate compound is administered. Also preferably, the selected compound does not significantly alter the cognitive status of a young mammal and/or an aged cognitively unimpaired mammal to whom the candidate compound is administered.

The methods of this invention for identifying compounds useful in the treatment of cognitive impairment, and preferably that due to aging, also include assays based on the cognitive status of a mammal in the presence and absence of the candidate compound.

The cognitive status of a mammal, preferably an aged mammal and most preferably an aged cognitively impaired mammal, and more preferably a rat, is determined in the presence and absence of a candidate compound. The cognitive status of the mammal may be assessed using various functions. Preferably, the functions are hippocampal-dependent functions. More preferably, the functions are spatial memory acquisition, long-term spatial memory, and spatial memory retrieval.

It is preferable that the candidate compound be one that is believed to increase or decrease, in the appropriate direction, as defined herein, the abundance or function of an expressed gene product of the mammal's homologue of a gene or plurality of genes selected in the methods described above or listed in the Tables.

This belief may be based on actual experimental data using other elements of this invention or may be based on information in the scientific literature. A compound that beneficially alters the cognitive status of the treated animal is then selected as being useful in the treatment of cognitive impairment. Preferably, the selected compound beneficially alters the cognitive status in the treated mammal relative to a member of the group consisting of an aged mammal without cognitive impairment, a young mammal, an aged cognitively impaired mammal to whom the compound is not administered and two or more of them. Preferably, the compound does not significantly alter the cognitive status of young mammals or aged unimpaired mammals of the same species who are treated with the compound.

Candidate compounds for use in the methods of the invention include a wide variety of general types of well-known and available compounds including, but not limited to, small organic molecules (e.g., MW < 1000, preferably < 500); polypeptides; carbohydrates such as oligosaccharides and polysaccharides; polynucleotides; antibodies, lipids or phospholipids; fatty acids; steroids; amino acid analogs, and peptidomimetics. Candidate compounds can be obtained from libraries, such as natural product libraries and combinatorial libraries. A number of different libraries and collections containing large numbers of natural and synthetic compounds are commercially available and one of ordinary skill in the art would be familiar with methods for preparing and obtaining such libraries. Methods of automating assays are also known that permit screening of several thousands of compounds in a short period.

Compounds identified in accordance with the methods of this invention are useful in the treatment of cognitive impairment in mammals, preferably human, and preferably in methods of treating cognitive impairment in aged mammals, preferably humans. The particular compound from among the compounds selected by the methods of this invention to be used can be determined by those skilled in the art, and will depend, for example, on factors such as the severity of the cognitive impairment; the time period over which treatment of the cognitive impairment is desired; whether the compound is administered in a clinical setting or by the individual; or whether an individual suffers from age-related cognitive impairment.

Compounds, selected in accordance with this invention for use in treating cognitive impairment, and particularly that due to aging, or for use in the screening methods of this invention, can be formulated in pharmaceutical compositions in such a manner to ensure proper distribution in vivo. For example, the blood-brain barrier excludes many highly hydrophilic compounds. To ensure that the selected compounds the blood-brain barrier, they can be formulated, for example, in liposomes, or chemically derivatized. A wide variety of carriers can be used to facilitate targeted drug delivery across the blood-brain barrier to brain tissues, preferably the hippocampus, including but not limited to the use of liposomes, nanoparticles, microparticles, microspheres, encapsulated microbubbles or similar structures which envelope biologically or pharmaceutically active agents, carrier molecules including polymers, and protein including hydrophile proteins.

Administration of a selected compound, or candidate compound(s) to be screened can be in a single dose, or in multiple doses. An effective amount of a compound can be determined by those skilled in the art, and can depend on the chemical and biological properties of the compound and the method of contacting the subject. Typically between 0.1 and 1000 mg/kg is administered daily, for one or more days.

Administration of the compound can be carried out using one of a variety of methods known to those of skill in the art. For example, a compound can be administered, intravenously, arterially, intradermally, intramuscularly, intraperitonealy, intravenously, subcutaneously, ocularly, sublingually, orally (by ingestion), intranasally (by inhalation), intraspinally, intracerebrally, and transdermally (by absorbtion, e.g., through a skin duct). A compound can also appropriately be introduced by rechargeable or biodegradable polymeric devices, which provide for the slow release or controlled delivery of drugs.

Appropriate methods of administering a compound to a mammal will also depend, for example, on the age of the mammal, whether the mammal is active or inactive at the time of administering, whether the mammal is cognitively impaired at the time of administering, and the chemical and biological properties of the compound (e.g. solubility, digestibility, bioavailability, stability and toxicity). Preferably, a compound is administered orally, e.g., to a mammal by ingestion, where the compound is dissolved in food and provided to the mammal at mealtime.

Pharmaceutical compositions for oral administration can be formulated using pharmaceutically acceptable carriers well known in the art in dosages suitable for oral administration that enable the compositions to be formulated as, e.g., tablets, pills, dragees, capsules, liquids, gels, syrups, slurries, suspensions, and the like. Other appropriate routes of administration and doses of the compound can be determined by those skilled in the art.

It will be understood that the efficacy and safety of a compound in laboratory mammals can be evaluated before administering the compound to humans. For example, the compound can be tested for its maximal efficacy and any potential side-effects using several different non-human mammals, across a range of doses, in a range of formulations, and at various times of day, such as before or after sleeping, before or after eating, and the like. Preferably, the compound selected by the methods of the invention will cause few or no deleterious or unwanted side effects in any of the test populations. More preferably, the compound will cause few or no deleterious side effects in the mammal whose cognitive status is beneficially altered by the administration of the compound. Most preferably, the compound will cause few or no deleterious side effects in an aged cognitively impaired mammal to which the compound is administered.

Illustrative methods of this invention are described in the following Examples. Methods and materials similar or equivalent to those described can also be used in the practice of the present invention and will be apparent to those of skill in the art. The materials, methods, and examples are thus illustrative only and not intended to be limiting.

### EXAMPLES

We performed the following examples using rats of an outbred strain behaviorally characterized as described in more detail below into one of three groups: Y, AU and AI.

### Behavioral Characterization of Rats

We performed behavioral tests on 9 young (4-6 months) and 18 aged (25-27 months) pathogen-free male outbred Long-Evans rats with the Morris Water Maze (MWM) protocol. An additional 10 aged rats were tested in the MWM, followed by training and testing in the Radial Arm Maze to assess test-retest reliability for individual differences in cognitive function across the two tasks.

### Morris Water Maze Apparatus

In the MWM assay, rats learn and remember the location of an escape platform guided by a configuration of spatial cues surrounding the maze. See, Morris, Learning and Motivation 12:239-260 (1981). The cognitive basis of performance is tested in probe trials using measures of the animal's spatial bias in searching for the location of the escape platform. Aged rats in the study population have no difficulty swimming to a visible platform, but an age-dependent cognitive impairment can be detected when the platform is camouflaged, requiring the use of spatial information.

When reassessed using the MWM in a new spatial environment several weeks after the original characterization, the AI animals are consistently impaired, whereas the AU animals again perform proficiently. Colombo et al., Proc. Natl. Acad. Sci. USA, 94:14195-99 (1997). The difference in cognitive ability in the MWM assessment for AI and AU rats is reliable even over an interval of 3 months. See, Gallagher and Burwell, Neurobiol. Aging 10:691-708 (1989). Further, AI and AU characterization in the MWM differentiates the performance of the same aged subjects in other behavioral tasks that require the same cognitive function, such as the Barnes circular maze, and the radial arm maze (RAM). This naturally occurring impairment in an aged population of rodents indicates that cognitive aging is not inevitable or strictly linked to chronological age, and, importantly, it affords the opportunity to compare the trajectory of changes in the brain that lead to decline or preserved memory.

The MWM apparatus consists of a large, circular pool (diameter 1.83 m; height, 0.58 m) filled with water (27°C) that has been made opaque through the addition of non-toxic pigment or some other substance. In the typical "hidden platform" version of the task, rats are trained to find a camouflaged white escape platform (height, 34.5 cm) that is positioned in the center of one quadrant of the maze just 1.0 cm below the water surface. This platform can be retracted to the bottom of the tank or raised to its normal position from outside the maze during behavioral testing. The location of this platform remained constant from trial to trial. Because there were no local cues that marked the position of the platform, the rat's ability to locate it efficiently from any starting position at the perimeter of the pool depended on using information surrounding the maze. The maze was surrounded by black curtains with white patterns affixed to provide a configuration of spatial cues.

A second platform (height 37.5 cm) with its surface painted black was elevated 2 cm above the water surface during cue training, the version of the task used to control for factors unrelated to cognition. The behavior of a rat in the pool was recorded by a camera suspended 2.5 m above the center of the pool, connected to a video tracking system (HVS Image Advanced Tracker VP200) and a PC computer running HVS software developed by Richard Baker of HVS Image, Hampton, UK.

### Morris Water Maze Procedure

We optimized the MWM protocol for sensitivity to the effects of aging on cognition and for measures of reliable individual differences within the aged population of outbred Long-Evans rats (Gallagher et al., Behav. Neurosci. 107:618-626 (1993)).

Rats received three trials per day for 8 consecutive days, using a 60 second inter-trial interval. On each training trial, the rat was released in the maze from one of four equally spaced starting positions around the perimeter of the pool. The starting position varied from trial to trial, thus preventing the use of a response strategy (e.g. always turning left from the start location to locate the escape platform). If a rat did not locate the escape platform within 90 sec on any trial, the experimenter guided the rat to the platform, where it remained for 30 sec. Every sixth trial consisted of a probe trial to assess the development of spatial bias in the maze. During these trials, the rat swam with the platform retracted to the bottom of the pool for 30 sec, at which time the platform was raised to its normal position for completion of an escape trial. At the completion of the protocol using the hidden platform, rats were assessed for cue learning using the visible platform. The location of this platform varied from trial to trial in a single session of 6 training trials.

We used the proximity of the animal's position with respect to the goal for analysis of training trial and probe trial performance. The proximity measure was obtained by sampling the position of the animal in the maze (10X/sec) to provide a record of distance from the escape platform in 1 sec averages. For both probe trials and training trials, a correction procedure was implemented so that trial performance was relatively unbiased by differences in distance to the goal from the various start locations at the perimeter of the pool. In making this correction the average swimming speed was calculated for each trial (path length/latency). Then, the amount of time required to swim to the goal at that speed from the start location used in the trial was removed from the record prior to computing trial performance, i.e. cumulative distance on training trials and average distance from the goal on probe trials. Thus, scores obtained using the proximity measure are designed to reflect search error, representing deviations from an optimal search, i.e. direct path to the goal and search in the immediate vicinity of that location during probe trials.

### Morris Water Maze Analysis

Computer records of video-tracking were compiled to provide data on each rat's performance in the maze. Measures on training trials and probe trials were analyzed by ANOVA.

### Morris Water Maze Data Results

The performance during training with the hidden, camouflaged platform differed between the groups of young and aged rats [F(1,23)= 12.69, p<.002]. No difference between the groups occurred for the cue training trials with a visible platform. Latencies to escape during cue training averaged 9.36 seconds for young and 10.60 seconds for the aged rats.

The average proximity measure on interpolated probe trials was used to calculate a spatial learning index for each individual subject as described in detail in Gallagher et al., Behav. Neurosci. 107:618-626 (1993). When a rat rapidly learned to search for the platform close to its position, its spatial learning index is low. Overall, aged rats differed from young [F(1,23) = 15.18, p<.001]. Aged rats were classified as either AU or AI relative to the learning index profile of the young study population. Aged rats that fall within the normative range of young rats (index scores <241) were designated AU. The remaining aged subjects that have index scores outside the range of young performance were designated AI.

### Radial Arm Maze Apparatus

Each arm (7 x 75 cm) of the elevated eight arm radial arm maze (RAM) projected from each facet of an octagonal center platform (30 cm diameter, 51.5 cm height). Clear side walls on the arms were 10 cm high and were angled at 65° to form a trough. A food well (4 cm diameter, 2 cm deep) was located at the distal end of each arm. Blocks constructed of Plexiglas (30 cm H x 12 cm W) could be positioned to block entry to any arm. Numerous extra maze cues were provided in the room surrounding the apparatus and overhead fixtures provided lighting.

### Radial Arm Maze Procedures

Rats were first habituated to the maze for an 8 min session on four consecutive days. In each of these sessions food rewards were scattered on the RAM, initially on the center platform and arms and then progressively confined to the arms. After this habituation phase, a standard training protocol was used in which a food pellet was located at the end of each arm. Rats received one trial each day for 18 days; each daily trial terminated when all eight food pellets had been obtained or when either 16 choices were made or 15 min had elapsed. An error consisted of returning to an arm (all four paws on the arm) from which food had already been obtained.

After completion of this phase, the memory demand of the task was increased by imposing a delay during the trial. At the beginning of each trial three arms were blocked. The identity and configuration of the blocked arms was varied across trials. Rats were allowed to obtain food on the five arms to which access was permitted at the beginning of the trial. The rat was then removed from the maze for 60 seconds, during which time the barriers on the maze were removed, thus allowing access to all eight arms. Rats were then placed back onto the center platform and allowed to obtain the remaining food rewards.

### Radial Arm Maze Analysis

A memory error occurred during test trials using a 60 second delay when a rat returned to one of the five arms that were already visited prior to the delay. Each rat's performance was averaged across four consecutive test trials. Parametric statistics (unpaired t-tests) were used to compare performance between young and aged groups. Correlational analysis (Pearson's r) was used to examine the relationship between performance of aged rats (N=10) in the MWM (learning index scores) and RAM (memory errors).

### Radial Arm Maze Results

The performance of young adult rats in the delay version of the RAM varies as a function of the delay interval, ranging from 60 seconds to eight hours (Chappell et al. Neuropharmacology 37: 481-488, (1998)). Aged rats previously characterized in the MWM, committed more memory errors after a 60 second delay relative to young rats (p < .025). On average young rats committed 0.17 errors, whereas aged rats committed an average of 1.52 errors. The ten aged rats, however, exhibited a wide range of performance on the RAM. A significant relationship was found between the initial MWM characterization and memory performance in the RAM (r value = .82).

### Gene Expression Analysis

We then analyzed the gene expression profiles in the CA1, CA3 or DG hippocampal regions. We determined the abundance of a plurality of expressed gene products in CA1, CA3 or DG hippocampal tissue from each mammal of the three populations -- AI, AU and Y. We selected those genes, the abundance of whose expressed gene products was significantly increased or decreased in the AI population as compared to the combined Y and AU populations or significantly increased or decreased in the AU population as compared to the combined Y and AI populations. These genes may relate specifically to age-related cognitive impairment.

We selected from the above genes those whose increased or decreased abundance of expressed gene product(s) showed a significant correlation with the level of cognitive impairment in the AU and AI populations.

These analyses are described below.

### Preparation of RNA from Behaviorally Characterized Rats

Twenty-four outbred Long-Evans rats, behaviorally characterized as described above, were killed by live decapitation to obtain fresh brain tissue. The brain was removed, the hippocampus dissected and the CA1, CA3 or DG hippocampal region was microdissected from 500 micron sections taken through the transverse axis of the entire hippocampal formation (both left and right hippocampi) of 24 characterized rats. There were 8 animals in each group (AI, AU and Y) and the CA1, CA3 or DG region of each animal was processed independently.

Total RNA was isolated using Trizol reagent (Invitrogen, Carlsbad, CA) according to the standard protocol (homogenization in Trizol reagent followed by chloroform extraction and isopropanol precipitation). Total RNA was further purified using the RNeasy mini kit (Qiagen, Valencia, CA). cRNA probes were then generated from the RNA samples at The Johns Hopkins Microarray Core Facility, generally according to Affymetrix specifications as detailed herein.

Briefly, 5 micrograms of total RNA were used to synthesize first strand cDNA using oligonucleotide probes with 24 oligo-dT plus T7 promoter as primer (Proligo LLC, Boulder, CA), and the SuperScript Choice System (Invitrogen). Following the double stranded cDNA synthesis, the product was purified by phenolchloroform extraction, and biotinilated anti-sense cRNA was generated through in vitro transcription using the BioArray RNA High Yield Transcript Labeling kit (ENZO Life Sciences Inc., Farmingdale, NY). 15 ug of the biotinilated cRNA was fragmented at 94°C for 35 min (100mM Trix-acetate, pH 8.2, 500mM KOAc, 150mM MgOAC). 10ug of total fragmented cRNA was hybridized to the RAT genome 230-2 Affymetrix GeneChip array for 16 hours at 45°C with constant rotation (60 rpm). The cRNA prepared from the CA1, CA3 or DG regions for each animal were hybridized to an individual microarray.

Affymetrix Fluidics Station 450 was then used to wash and stain the chips, removing the non-hybridized target and incubating with a streptavidin-phycoerythrin conjugate to stain the biotinilated cRNA. The staining was then amplified using goat immunoglobulin-G (IgG) as blocking reagent and biotinilated anti-streptavidin antibody (goat), followed by a second staining step with a streptavidin-phycoerythrin conjugate.

For quality control of the total RNA from the samples, the Agilent Bioanalyzer, Lab on a Chip technology, was used to confirm that all the samples had optimal rRNA ratios (1:2, for 18S and 28S, respectively) and clean run patterns.

For quality control of the hybridization, chip image, and comparison between chips, the following parameters were considered: Scaling factor: related to the overall intensity of the chip, to confirm the similar signal intensity and staining through out the samples; Background: estimation of unspecific or cross-hybridization; Percentage of present calls (for "MAS5" analysis only, *see infra,* Data Analysis of Microarray): percentage of transcripts that are considered significantly hybridized to the chip (present) by the algorithm; Glyseraldehyde-3-phosphate dehydrogenase (GAPDH) (3'/5'): representation of the RNA integrity by measuring the ratio of 3' to 5' regions for the housekeeping gene GAPDH, its presence in the chip and a ratio close to 1 advocates for a good integrity of the target (sample); Spikes (BioB/BioC) to confirm the detection level and sensitivity after hybridization.

### Data Analysis of Microarray

Fluorescence was detected using the Affymetrix G3000 GeneArray Scanner and image analysis of each GeneChip was done through the GeneChip Operating System 1.1.1 (GCOS) software from Affymetrix, using the standard default settings. All of the GeneChip arrays use short oligonucleotides to probe for genes in an RNA sample.

For comparison between different chips, global scaling was used, scaling all probe sets to a user defined target intensity (TGT) of 150. Two different methods were used to estimate the relative expression of genes in different RNA samples. Examples 1-24 processed and summarized the probe set data by "MAS5" analysis. Examples 25-48 processed and summarized the probe set data by "gcRMA" analysis.

The first method, the MAS5 statistical method, produces absolute and comparison analysis results for GeneChip expression arrays. Mas5 employs a statistical algorithm that performs a background adjustment by making regional adjustments or "calls". This process makes expression level calls as to whether a particular probe set is present or absent. (See, Affymetric statistical algorithm description document, available on the Affymetrix website.)

In Examples 1-24, the total number of present calls and scaling factors were similar across all chips (normalization was scaled by a constant). Further analysis for presence/absence and statistical difference was performed on a region by region basis in the following manner. Probe sets were determined to be present in a region if it had a present call in four of eight animals in a single group, as per the standard MAS5 default settings using Affymetrix software.

The second method used to process and summarize the probe set data was "gcRMA" analysis. This statistical method employs background adjustment by estimating a global signal over the entire probe set and making a whole array adjustment, using quantile normalization. GcRMA is an open-source method that is based on robust averaging techniques and sequence-dependent affinity corrections. The robust averaging employed in gcRMA confers a strong immunity to outliers. See, Wu et al. A Model Based Background Adjustement for Oligonucleotide Expression Arrays. Journal of American Statistical Association. 99:909-917 (2004).

In Examples 25-48, gcRMA analysis was used to perform background and normalization processing, which coupled all the genes together, as did the MAS5 analysis in Examples 1-24. Further analysis for statistical difference included performing a background subtraction. Mismatched sequences (11 for each probe set) were ignored and only the perfect match sequences were considered. There was no elimination of genes based on present/absent calls. See, Quin et al., Evaluation of methods for oligonucleotide array data via quantitative real-time PCR. BMC Bioinformatics, 7:23 (2006).

For Examples 25-48, probe sets were annotated using the most recent Affymetrix annotation of April 2006 and all probe sets representing a specific gene were identified. For Examples 1-24, probe sets were annotated using the Affymetrix annotations of June 20, 2005.

The probe set signal values were then analyzed in the following Examples by various statistical methods to identify those genes or plurality of genes expressed gene products that significantly changed in abundance -- increase or decrease - and to identify those genes whose increased or decreased expression product(s) abundance correlated to cognitive impairment.

### Example 1

An ANOVA was conducted on the probe set signal values for all present probe sets by combining two groups of animals and comparing them to the third group. An "AI ANOVA" was performed, where Aged Unimpaired were combined with Young and compared to Aged Impaired. Any probe sets having a p-value less than 0.05 were considered significantly changed and the genes or plurality of genes selected.

Pearson's correlations comparing probe set signal values to learning indices were then calculated for the aged animals (excluding young) across all present probe sets. Again, correlations representing a p-value of less than 0.05 were considered significantly changed.

### Microarray Results

Table 1 shows the genes that were identified as being associated with cognitive impairment in this experiment. The reference sequence transcript ID (GENBANK® number for the exemplar sequence) was used to identify genes considered significantly changed. When this exemplar ID was not available, a UniGene identifier was used in conjunction with the GENBANK® accession number.

**TABLE 1**

| **CA1 - AI ANOVA POSITIVE CORRELATION** | | |
|---|---|---|
| | | |

| **GENBANK® ID** | **GENBANK® ID** | **GENBANK® ID** |
|---|---|---|
| BI295776 | NM_022617 | NM_207602 |
| AA963364 | NM_023981 | NM_212528 |
| AY190520 | NM_024138 | XM_214769 |
| NM_001002016 | NM_030863 | XM_214968 |
| NM_001005534 | NM_031037 | XM_215095 |
| NM_001008374 | NM_031147 | XM_216004 |
| NM_001008862 | NM_031818 | XM_221231 |
| NM_001012177 | NM_053418 | XM_224538 |
| NM_001013112 | NM_053485 | XM_230616 |
| NM_012543 | NM_053525 | XM_235566 |
| NM_012577 | NM_053536 | XM_237000 |
| NM_012650 | NM_053669 | XM_243637 |
| NM_012918 | NM_053774 | XM_340967 |
| NM_013001 | NM_053896 | XM_343773 |
| NM_013157 | NM_053910 | XM_344524 |
| NM_017068 | NM_057103 | XM_345140 |
| NM_017223 | NM_080887 | XM_575962 |
| NM_017347 | NM_130413 | |
| NM_019212 | NM_133621 | |
| NM_019289 | NM_134390 | **UniGene ID** |
| NM_019305 | NM_145674 | Rn.110441 |
| NM_019359 | NM_172038 | Rn.11453 |
| NM_019362 | NM_173325 | |
| NM_021657 | NM_175582 | |
| NM_022244 | NM_198738 | |
| NM_022261 | NM_198759 | |

The genes in this set show abundance of expressed gene product(s) in the Aged Impaired population that are significantly increased, as determined by ANOVA, compared to those abundances in Young and Aged Unimpaired populations combined. In addition, the abundance of expressed gene product(s) in the aged animals positively correlate with learning index of the animals, such that poorer learners have higher abundances of expressed gene products of the selected genes. Therefore, compounds useful in treating cognitive impairment selected using these genes should decrease the abundance or attenuate the function of the expressed gene products of these genes.

### Example 2

An ANOVA was conducted on the probe set signal values for all present probe sets by combining two groups of animals and comparing them to the third group. An "AI ANOVA" was performed, where Aged Unimpaired were combined with Young and compared to Aged Impaired. Any probe sets having a p-value less than 0.05 were considered significantly changed and the genes or plurality of genes selected.

Pearson's correlations comparing probe set signal values to learning indices were then calculated for the aged animals (excluding young) across all present probe sets. Again, correlations representing a p-value of less than 0.05 were considered significantly changed.

### Microarray Results

Table 2 shows the genes that were identified as being associated with cognitive impairment in this experiment. The reference sequence transcript ID (GENBANK® number for the exemplar sequence) was used to identify genes considered significantly changed. When this exemplar ID was not available, a UniGene identifier was used in conjunction with the GENBANK® accession number.

**TABLE 2**

| **CA1 - AI ANOVA NEGATIVE CORRELATION** | | |
|---|---|---|
| | | |

| **GENBANK® ID** | **GENBANK® ID** | **GENBANK® ID** |
|---|---|---|
| BG381750 | NM_053434 | XM_218506 |
| NM_001004210 | NM_053686 | XM_220281 |
| NM_001004279 | NM_053748 | XM_220629 |
| NM_001004442 | NM_053883 | XM_221635 |
| NM_001011955 | NM_133563 | XM_222773 |
| NM_001012035 | NM_134383 | XM_223693 |
| NM_001013178 | NM_134408 | XM_226779 |
| NM_001024765 | NM_138838 | XM_232995 |
| NM_012919 | NM_139091 | XM_235878 |
| NM_013083 | NM_153472 | XM_236914 |
| NM_013086 | NM_172332 | XM_341663 |
| NM_017322 | NM_173133 | XM_342044 |
| NM_020078 | NM_199091 | XM_342920 |
| NM_022395 | XM_214043 | XM_343059 |
| NM_022867 | XM_214245 | XM_343154 |
| NM_022934 | XM_214428 | XM_343581 |
| NM_024000 | XM_214836 | |
| NM_030861 | XM_215812 | |
| NM_031699 | XM_215883 | |
| NM_031707 | XM_216102 | **UniGene ID** |
| NM_031730 | XM_216884 | Rn.101929 |
| NM_031821 | XM_217464 | |
| NM_053328 | XM_217893 | |
| NM_053402 | XM_218502 | |

The genes in this set show abundance of expressed gene product(s) in the Aged Impaired population that are significantly decreased, as determined by ANOVA, compared to those abundances in Young and Aged Unimpaired populations combined. In addition, the abundance of expressed gene product(s) in the aged animals negatively correlate with learning index of the animals, such that poorer learners have lower abundances of expressed gene products of the selected genes. Therefore, compounds useful in treating cognitive impairment selected using these genes should increase the abundance or enhance the function of the expressed gene products of these genes.

### Example 3

An ANOVA was conducted on the probe set signal values for all present probe sets by combining two groups of animals and comparing them to the third group. An "AI ANOVA" was performed, where Aged Unimpaired were combined with Young and compared to Aged Impaired. Any probe sets having a p-value less than 0.05 were considered significantly changed and the genes or plurality of genes selected.

### Microarray Results

Table 3 shows the genes that were identified as being associated with cognitive impairment in this experiment. The reference sequence transcript ID (GENBANK® number for the exemplar sequence) was used to identify genes considered significantly changed. When this exemplar ID was not available, a UniGene identifier was used in conjunction with the GENBANK® accession number.

**TABLE 3**

| **CA1** - **AI ANOVA INCREASE** | | |
|---|---|---|
| | | |

| **GENBANK® ID** | **GENBANK® ID** | **GENBANK® ID** |
|---|---|---|
| AJ249701 | NM_013055 | NM_053455 |
| NM_017154 | NM_013091 | NM_053492 |
| AW528959 | NM_013096 | NM_053502 |
| AF452647 | NK_013107 | NM_053516 |
| NM_001002835 | NM_013198 | NM_053538 |
| NM_001004209 | NM_017009 | NM_053612 |
| NM_001005539 | NM_017109 | NM_053639 |
| NM_001005565 | NM_017169 | NM_053818 |
| NM_001007654 | NM_017181 | NM_054001 |
| NM_001007682 | NM_017209 | NM_057107 |
| NM_001007749 | NM_017320 | NM_057120 |
| NM_001009651 | NM_017351 | NM_057123 |
| NM_001011890 | NM_017356 | NM_057185 |
| NM_001011934 | NM_019238 | NM_057197 |
| NM_001011946 | NM_019278 | NM_080890 |
| NM_001011974 | NM_019335 | NM_130409 |
| NM_001011993 | NM_019363 | NM_130428 |
| NM_001012069 | NM_019620 | NM_133298 |
| NM_001012097 | NM_021576 | NM_133392 |
| NM_001012141 | NM_021690 | NM_133393 |
| NM_001012154 | NM_022390 | NM_133548 |
| NM_001012351 | NM_022500 | NM_133605 |
| NM_001013086 | NM_022502 | NM_134334 |
| NM_001013118 | NM_022512 | NM_134349 |
| NM_001013121 | NM_022526 | NM_134407 |
| NM_001013179 | NM_022531 | NM_134410 |
| NM_001013238 | NM_022864 | NM_138508 |
| NM_001015004 | NM_022948 | NM_138521 |
| NM_012488 | NM_024155 | NM_138539 |
| NM_012512 | NM_024387 | NM_138826 |
| NM_012523 | NM_030992 | NM_139060 |
| NM_012562 | NM_031153 | NM_139110 |
| NM_012654 | NM_031357 | NM_139185 |
| NM_012671 | NM_031509 | NM_139192 |
| NM_012703 | NM_031552 | NM_139256 |
| NM_012749 | NM_031640 | NM_145775 |
| NM_012771 | NM_031714 | NM_153315 |
| NM_012823 | NM_031797 | NM_153621 |
| NM_012838 | NM_031798 | NM_173095 |
| NM_012884 | NM_053021 | NM_173118 |
| NM_012899 | NM_053314 | NM_173123 |
| NM_012984 | NM_053323 | NM_173141 |
| NM_013015 | NM_053424 | |
| NM_175578 | XM_217470 | XM_341509 |
| NM_175756 | XM_219447 | XM_342291 |
| NM_176077 | XM_220264 | XM_342331 |
| NM_178095 | XM_221369 | XM_342794 |
| NM_182821 | XM_221387 | XM_343057 |
| NM_199404 | XM_223080 | XM_343126 |
| NM_207591 | XM_223087 | XM_343159 |
| NM_212466 | XM_223190 | XM_343259 |
| XM_213329 | XM_223781 | XM_343310 |
| XM_213574 | XM_223785 | XM_343640 |
| XM_213610 | XM_224337 | XM_343650 |
| XM_214250 | XM_225885 | XM_573983 |
| XM_214298 | XM_227701 | XM_576401 |
| XM_214403 | XM_228073 | XM_579460 |
| XM_214518 | XM_229225 | XM_579675 |
| XM_215037 | XM_230296 | |
| XM_215578 | XM_231120 | **UniGene ID** |
| XM_215935 | XM_231287 | Rn.116787 |
| XM_216367 | XM_232531 | Rn.129174 |
| XM_216565 | XM_232671 | Rn.24948 |
| XM_216665 | XM_234483 | Rn.24948 |
| XM_216882 | XM_237049 | |
| XM_217252 | XM_341081 | |

The genes in this set show abundance of expressed gene product(s) in the Aged Impaired population that are significantly increased, as determined by ANOVA, compared to those abundances in Young and Aged Unimpaired populations combined. Therefore, compounds useful in treating cognitive impairment selected using these genes should decrease the abundance or attenuate the function of the expressed gene products of these genes.

### Example 4

An ANOVA was conducted on the probe set signal values for all present probe sets by combining two groups of animals and comparing them to the third group. An "AI ANOVA" was performed, where Aged Unimpaired were combined with Young and compared to Aged Impaired. Any probe sets having a p-value less than 0.05 were considered significantly changed and the genes or plurality of genes selected.

### Microarray Results

Table 4 shows the genes that were identified as being associated with cognitive impairment in this experiment. The reference sequence transcript ID (GENBANK® number for the exemplar sequence) was used to identify genes considered significantly changed. When this exemplar ID was not available, a UniGene identifier was used in conjunction with the GENBANK® accession number.

**TABLE 4**

| **CA1 - AI ANOVA DECREASE** | | |
|---|---|---|
| **GENBANK® ID** | **GENBANK® ID** | **GENBANK® ID** |
| AI231320 | NM_022254 | XM_213679 |
| BF284573 | NK_022289 | XM_213906 |
| BF392810 | NM_022548 | XM_213954 |
| NM_001002289 | NM_031036 | XM_214454 |
| NM_001004133 | NM_031318 | XM_214668 |
| NM_001004284 | NM_031325 | XM_215566 |
| NM_001009605 | NM_031514 | XM_216158 |
| NM_001009953 | NM_031560 | XM_216656 |
| NM_001011901 | NM_031740 | XM_217115 |
| NM_001011964 | NM_031745 | XM_218828 |
| NM_001011998 | NM_031753 | XM_219128 |
| NM_001012011 | NM_031777 | XM_221496 |
| NM_001012078 | NM_032085 | XM_221888 |
| NM_001012144 | NM_053375 | XM_221962 |
| NM_001012179 | NM_053401 | XM_222251 |
| NM_001012214 | NM_053410 | XM_222661 |
| NM_001012473 | NM_053441 | XM_222726 |
| NM_001013036 | NM_053483 | XM_224707 |
| NM_001013096 | NM_053772 | XM_224929 |
| NM_001013244 | NM_053849 | XM_225220 |
| NM_001013910 | NM_053868 | XM_226888 |
| NM_001014793 | NM_053933 | XM_230449 |
| NM_012527 | NM_057196 | XM_232220 |
| NM_012550 | NM_057200 | XM_236932 |
| NM_012561 | NM_080411 | XM_239171 |
| NM_012573 | NM_080582 | XM_239260 |
| NM_012576 | NM_080902 | XM_341157 |
| NM_012769 | NM_133406 | XM_341239 |
| NM_012911 | NM_133425 | XM_341391 |
| NM_012985 | NM_133429 | XM_341712 |
| NM_013102 | NM_138837 | XM_341745 |
| NM_017041 | NM_138849 | XM_342107 |
| NM_017074 | NM_138866 | XM_342344 |
| NM_017204 | NM_138887 | XM_342600 |
| NM_017242 | NM_144758 | XM_342808 |
| NM_017318 | NM_172034 | XM_343046 |
| NM_017336 | NM_173145 | XM_343175 |
| NM_019169 | NM_181380 | XM_343761 |
| NM_019211 | NM_184051 | XM_344594 |
| NM_020306 | NM_198726 | XM_345861 |
| NM_021597 | NM_212520 | XM_345981 |
| NM_021697 | U57097 | |
| NM_021850 | X53232 | |
| NM_022252 | XM_213426 | |

| **UniGene ID** | | |
|---|---|---|
| Rn.122667 | | |
| Rn.15446 | | |
| Rn.48866 | | |

The genes in this set show abundance of expressed gene product(s) in the Aged Impaired population that are significantly decreased, as determined by ANOVA, compared to those abundances in Young and Aged Unimpaired populations combined. Therefore, compounds useful in treating cognitive impairment selected using these genes should increase the abundance or enhance the function of the expressed gene products of these genes.

### Example 5

An ANOVA was conducted on the probe set signal values for all present probe sets by combining two groups of animals and comparing them to the third group. An "AU ANOVA" was performed, where Aged Impaired were combined with Young and compared to Aged Unimpaired. Any probe sets having a p-value less than 0.05 were considered significantly changed and the genes or plurality of genes selected.

Pearson's correlations comparing probe set signal values to learning indices were then calculated for the aged animals (excluding young) across all present probe sets. Again, correlations representing a p-value of less than 0.05 were considered significantly changed.

### Microarray Results

Table 5 shows the genes that were identified as being associated with cognitive impairment in this experiment. The reference sequence transcript ID (GENBANK® number for the exemplar sequence) was used to identify genes considered significantly changed. When this exemplar ID was not available, a UniGene identifier was used in conjunction with the GENBANK® accession number.

**TABLE 5**

| **CA1** - **AU ANOVA POSITIVE CORRELATION** | | |
|---|---|---|
| **GENBANK® ID** | **GENBANK® ID** | **GENBANK® ID** |
| AI145040 | NM_139260 | XM_346061 |
| NM_001007629 | NM_172041 | |
| NM_001012028 | XM_213564 | |
| NM_012546 | XM_214003 | |
| NM_019152 | XM_222245 | **UniGene ID** |
| NM_022177 | XM_233462 | Rn.36521 |
| NM_022286 | XM_341100 | |
| NM_022858 | XM_341558 | |
| NM_022946 | XM_342548 | |
| NM_080692 | XM_343468 | |
| NM_138515 | XM_343764 | |

The genes in this set show abundance of expressed gene product(s) in the Aged Unimpaired population that are significantly decreased, as determined by ANOVA, compared to those abundances in Young and Aged Impaired populations combined. In addition, the abundance of expressed gene product(s) in the aged animals positively correlate with learning index of the animals, such that poorer learners have higher abundances of expressed gene products of the selected genes. Therefore, compounds useful in treating cognitive impairment selected using these genes should decrease the abundance or attenuate the function of the expressed gene products of these genes.

### Example 6

An ANOVA was conducted on the probe set signal values for all present probe sets by combining two groups of animals and comparing them to the third group. An "AU ANOVA" was performed, where Aged Impaired were combined with Young and compared to Aged Unimpaired. Any probe sets having a p-value less than 0.05 were considered significantly changed and the genes or plurality of genes selected.

Pearson's correlations comparing probe set signal values to learning indices were then calculated for the aged animals (excluding young) across all present probe sets. Again, correlations representing a p-value of less than 0.05 were considered significantly changed.

### Microarray Results

Table 6 shows the genes that were identified as being associated with cognitive impairment in this experiment. The reference sequence transcript ID (GENBANK® number for the exemplar sequence) was used to identify genes considered significantly changed. When this exemplar ID was not available, a UniGene identifier was used in conjunction with the GENBANK® accession number.

**TABLE 6**

| **CA1 - AU ANOVA NEGATIVE CORRELATION** | | |
|---|---|---|
| **GENBANK® ID** | | |
| NM_001002830 | | |
| NM_001012034 | | |
| NM_001012036 | | |
| NM_012806 | | |
| NM_017126 | | |
| NM_019249 | | |
| NM_019361 | | |
| NM_021754 | | |
| NM_031058 | | |
| NM_031315 | | |
| NM_032083 | | |
| NM_053578 | | |
| NM_080584 | | |
| NM_175604 | | |
| NM_182844 | | |
| NM_199397 | | |
| NM_199463 | | |
| XM_214033 | | |
| XM_215528 | | |
| XM_218041 | | |
| XM_218963 | | |
| XM_221213 | | |
| XM_225253 | | |
| XM_226334 | | |
| XM_228197 | | |
| XM_237115 | | |
| XM_341694 | | |
| XM_342580 | | |
| XM_343228 | | |

The genes in this set show abundance of expressed gene product(s) in the Aged Unimpaired population that are significantly increased, as determined by ANOVA, compared to those abundances in Young and Aged Impaired populations combined. In addition, the abundance of expressed gene product(s) in the aged animals negatively correlate with learning index of the animals, such that poorer learners have lower abundances of expressed gene products of the selected genes. Therefore, compounds useful in treating cognitive impairment selected using these genes should increase the abundance or enhance the function of the expressed gene products of these genes.

### Example 7

An ANOVA was conducted on the probe set signal values for all present probe sets by combining two groups of animals and comparing them to the third group. An "AU ANOVA" was performed, where Aged Impaired were combined with Young and compared to Aged Unimpaired. Any probe sets having a p-value less than 0.05 were considered significantly changed and the genes or plurality of genes selected.

### Microarray Results

Table 7 shows the genes that were identified as being associated with cognitive impairment in this experiment. The reference sequence transcript ID (GENBANK® number for the exemplar sequence) was used to identify genes considered significantly changed. When this exemplar ID was not available, a UniGene identifier was used in conjunction with the GENBANK® accession number.

**TABLE 7**

| **CA1** - **AU ANOVA INCREASE** | | |
|---|---|---|
| | | |

| **GENBANK® ID** | **GENBANK® ID** | **GENBANK® ID** |
|---|---|---|
| NM_001004256 | NM_020092 | U77829 |
| NM_001007797 | NM_021266 | XM_213883 |
| NM_001008317 | NM_022215 | XM_214163 |
| NM_001008509 | NM_022270 | XM_215283 |
| NM_001008515 | NM_022545 | XM_215733 |
| NM_001008556 | NM_022921 | XM_215947 |
| NM_001008876 | NM_024125 | XM_216225 |
| NM_001009619 | NM_031087 | XM_216968 |
| NM_001011953 | NM_031623 | XM_217388 |
| NM_001011979 | NM_031694 | XM_218648 |
| NM_001012105 | NM_032065 | XM_218816 |
| NM_001012131 | NM_053662 | XM_219885 |
| NM_001013110 | NM_053842 | XM_221307 |
| NM_001013151 | NM_053876 | XM_221656 |
| NM_001013201 | NM_053901 | XM_221787 |
| NM_001013210 | NM_057101 | XM_227674 |
| NM_001013213 | NM_130740 | XM_232413 |
| NM_001015003 | NM_131911 | XM_236263 |
| NM_001015015 | NM_133411 | XM_236911 |
| NM_012935 | NM_134326 | XM_237828 |
| NM_012992 | NM_134395 | XM_341742 |
| NM_013088 | NM_138536 | XM_341803 |
| NM_013150 | NM_139253 | XM_342154 |
| NM_016994 | NM_145084 | XM_342459 |
| NM_017000 | NM_145092 | XM_342579 |
| NM_017031 | NM_153303 | XM_342591 |
| NM_017137 | NM_153475 | XM_343619 |
| NM_017271 | NM_175762 | XM_344606 |
| NM_017303 | NM_175764 | XM_345421 |
| NM_017306 | NM_199117 | XM_573903 |
| NM_017313 | NM_199118 | XM_575860 |
| NM_019251 | NM_212496 | XM_576437 |
| NM_019257 | NM_213565 | |

The genes in this set show abundance of expressed gene product(s) in the Aged Unimpaired population that are significantly increased, as determined by ANOVA, compared to those abundances in Young and Aged Impaired populations combined. Therefore, compounds useful in treating cognitive impairment selected using these genes should increase the abundance or enhance the function of the expressed gene products of these genes.

### Example 8

An ANOVA was conducted on the probe set signal values for all present probe sets by combining two groups of animals and comparing them to the third group. An "AU ANOVA" was performed, where Aged Impaired were combined with Young and compared to Aged Unimpaired. Any probe sets having a p-value less than 0.05 were considered significantly changed and the genes or plurality of genes selected.

### Microarray Results

Table 8 shows the genes that were identified as being associated with cognitive impairment in this experiment. The reference sequence transcript ID (GENBANK® number for the exemplar sequence) was used to identify genes considered significantly changed. When this exemplar ID was not available, a UniGene identifier was used in conjunction with the GENBANK® accession number.

**TABLE 8**

| **CA1** - **AU ANOVA DECREASE** | | |
|---|---|---|
| | | |

| **GENBANK® ID** | **GENBANK® ID** | **GENBANK® ID** |
|---|---|---|
| AI236198 | NM_023972 | XM_214963 |
| NM_001004446 | NM_030988 | XM_220884 |
| NM_001007150 | NM_031018 | XM_220982 |
| NM_001008279 | NM_031654 | XM_221273 |
| NM_001013071 | NM_052983 | XM_223768 |
| NM_001013122 | NM_053428 | XM_232640 |
| NM_012545 | NM_053788 | XM_234901 |
| NM_012648 | NM_053797 | XM_340775 |
| NM_012762 | NM_053811 | XM_340999 |
| NM_012784 | NM_053859 | XM_342134 |
| NM_012808 | NM_053893 | XM_342863 |
| NM_013197 | NM_053996 | XM_343559 |
| NM_017061 | NM_080394 | XM_574284 |
| NM_019159 | NM_080580 | |
| NM_021671 | NM_130430 | **UniGene ID** |
| NM_021703 | NM_131906 | Rn.95299 |
| NM_022250 | NM_133313 | |
| NM_022609 | NM_133400 | |
| NM_022676 | NM_138854 | |
| NM_022853 | NM_207592 | |

The genes in this set show abundance of expressed gene product(s) in the Aged Unimpaired population that are significantly decreased, as determined by ANOVA, compared to those abundances in Young and Aged Impaired populations combined. Therefore, compounds useful in treating cognitive impairment selected using these genes should decrease the abundance or attenuate the function of the expressed gene products of these genes.

### Example 9

An ANOVA was conducted on the probe set signal values for all present probe sets by combining two groups of animals and comparing them to the third group. An "AI ANOVA" was performed, where Aged Unimpaired were combined with Young and compared to Aged Impaired. Any probe sets having a p-value less than 0.05 were considered significantly changed and the genes or plurality of genes selected.

Pearson's correlations comparing probe set signal values to learning indices were then calculated for the aged animals (excluding young) across all present probe sets. Again, correlations representing a p-value of less than 0.05 were considered significantly changed.

### Microarray Results

Table 9 shows the genes that were identified as being associated with cognitive impairment in this experiment. The reference sequence transcript ID (GENBANK® number for the exemplar sequence) was used to identify genes considered significantly changed. When this exemplar ID was not available, a UniGene identifier was used in conjunction with the GENBANK® accession number.

**TABLE 9**

| **CA3 - AI ANOVA POSITIVE CORRELATION** | | |
|---|---|---|
| | | |

| **GENBANK® ID** | **GENBANK® ID** | **GENBANK® ID** |
|---|---|---|
| BF420467 | NM_012825 | NM_021769 |
| AF452731 | NM_012925 | NM_021868 |
| AY190520 | NM_012999 | NM_022178 |
| NM_001001511 | NM_013005 | NM_022207 |
| NM_001007146 | NM_013045 | NM_022285 |
| NM_001007617 | NM_013060 | NM_022861 |
| NM_001007797 | NM_013096 | NM_024161 |
| NM_001008289 | NM_013137 | NM_024364 |
| NM_001008331 | NM_013199 | NM_030832 |
| NM_001008880 | NM_017012 | NM_031006 |
| NM_001008893 | NM_017015 | NM_031022 |
| NM_001009474 | NM_017026 | NM_031034 |
| NM_001011890 | NM_017031 | NM_031049 |
| NM_001011893 | NM_017041 | NM_031092 |
| NM_001011915 | NM_017068 | NM_031093 |
| NM_001011946 | NM_017073 | NH_031140 |
| NM_001011991 | NM_017175 | NM_031357 |
| NM_001012025 | NM_017181 | NM_031521 |
| NM_001012072 | NM_017197 | NM_031576 |
| NM_001012075 | NM_017206 | NM_031587 |
| NM_001012097 | NM_017214 | NM_031613 |
| NM_001012106 | NM_017223 | NM_031755 |
| NM_001012123 | NM_017239 | NM_031798 |
| NM_01012162 | NM_017248 | NM_031841 |
| NM_001012203 | NM_017261 | NM_032062 |
| NM_001012215 | NM_017274 | NM_032066 |
| NM_001013112 | NM_017307 | NM_032517 |
| NM_001013121 | NM_017329 | NM_052807 |
| NM_001013130 | NM_017333 | NM_053314 |
| NM_001013192 | NM_017354 | NM_053328 |
| NM_001013218 | NM_017357 | NM_053416 |
| NM_001017386 | NM_019161 | NM_053467 |
| NM_012502 | NM_019185 | NM_053492 |
| NM_012519 | NM_019249 | NM_053536 |
| NM_012577 | NM_019256 | NM_053615 |
| NM_012618 | NM_019288 | NM_053639 |
| NM_012671 | NM_019312 | NM_053643 |
| NM_012720 | NM_019341 | NM_053681 |
| NM_012778 | NM_019358 | NM_053741 |
| NM_012781 | NM_020098 | NM_053766 |
| NM_012804 | NM_021595 | NM_053799 |
| NM_012809 | NM_021682 | NM_053814 |
| NM_053824 | XM_215095 | XM_237381 |
| NM_053936 | XM_215184 | XM_238057 |
| NM_057137 | XM_215264 | XM_238103 |
| NM_057138 | XM_215733 | XM_238213 |
| NM_057148 | XM_215739 | XM_240330 |
| NM_057197 | XM_216340 | XM_241375 |
| NM_057200 | XM_216688 | XM_242032 |
| NM_130403 | XM_216755 | XM_242057 |
| NM_133560 | XM_217062 | XM_340739 |
| NM_133602 | XM_217279 | XM_340825 |
| NM_133605 | XM_217409 | XM_340879 |
| NM_134351 | XM_217570 | XM_340911 |
| NM_138832 | XM_218162 | XM_341052 |
| NM_138905 | XM_218226 | XM_341172 |
| NM_138914 | XM_218292 | XM_341352 |
| NM_139038 | XM_218816 | XM_341538 |
| NM_139103 | XM_219262 | XM_341940 |
| NM_145081 | XM_219998 | XM_342141 |
| NM_145094 | XM_220420 | XM_342405 |
| NM_147136 | XM_220805 | XM_342591 |
| NM_147210 | XM_220986 | XM_342626 |
| NM_152847 | XM_222745 | XM_342648 |
| NM_153470 | XM_222780 | XM_342662 |
| NM_172030 | XM_223583 | XM_342928 |
| NM_172033 | XM_225008 | XM_343131 |
| NM_173120 | XM_225014 | XM_343174 |
| NM_175578 | XM_225404 | XM_343358 |
| NM_175582 | XM_225628 | XM_343380 |
| NM_175595 | XM_226237 | XM_343570 |
| NM_175838 | XM_226779 | XM_343919 |
| NM_177928 | XM_227203 | XM_343971 |
| NM_178095 | XM_228073 | XM_344403 |
| NM_199119 | XM_229225 | XM_345446 |
| NM_199253 | XM_229988 | XM_573298 |
| NM_212529 | XM_230288 | XM_574593 |
| NM_213629 | XM_231121 | XM_576343 |
| XM_213779 | XM_231193 | XM_579200 |
| XM_213824 | XM_231287 | XM_579675 |
| XM_213849 | XM_231714 | |
| XM_213920 | XM_232172 | |
| XM_214172 | XM_232671 | |
| XM_214187 | XM_232809 | **UniGene ID** |
| XM_214539 | XM_235326 | Rn.22102 |
| XM_214740 | XM_237042 | |
| XM_214968 | XM_237232 | |

The genes in this set show abundance of expressed gene product(s) in the Aged Impaired population that are significantly increased, as determined by ANOVA, compared to those abundances in Young and Aged Unimpaired populations combined. In addition, the abundance of expressed gene product(s) in the aged animals positively correlate with learning index of the animals, such that poorer learners have higher abundances of expressed gene products of the selected genes. Therefore, compounds useful in treating cognitive impairment selected using these genes should decrease the abundance or attenuate the function of the expressed gene products of these genes.

### Example 10

An ANOVA was conducted on the probe set signal values for all present probe sets by combining two groups of animals and comparing them to the third group. An "AI ANOVA" was performed, where Aged Unimpaired were combined with Young and compared to Aged Impaired. Any probe sets having a p-value less than 0.05 were considered significantly changed and the genes or plurality of genes selected.

Pearson's correlations comparing probe set signal values to learning indices were then calculated for the aged animals (excluding young) across all present probe sets. Again, correlations representing a p-value of less than 0.05 were considered significantly changed.

### Microarray Results

Table 10 shows the genes that were identified as being associated with cognitive impairment in this experiment. The reference sequence transcript ID (GENBANK® number for the exemplar sequence) was used to identify genes considered significantly changed. When this exemplar ID was not available, a UniGene identifier was used in conjunction with the GENBANK® accession number.

**TABLE 10**

| **CA3 - AI ANOVA NEGATIVE CORRELATION** | | |
|---|---|---|
| | | |

| **GENBANK® ID** | **GENBANK® ID** | **GENBANK® ID** |
|---|---|---|
| BG379941 | NM_017135 | NM_053316 |
| BM389079 | NM_017294 | NM_053404 |
| AI228348 | NM_017295 | NM_053490 |
| AY325138 | NM_019133 | NM_053556 |
| NM_001004133 | NM_019166 | NM_053585 |
| NM_001004210 | NM_019226 | NM_053607 |
| NM_001004235 | NM_019368 | NM_053660 |
| NM_001004442 | NM_021688 | NM-053682 |
| NM_001005564 | NM_021842 | NM_053693 |
| NM_001005884 | NM_021850 | NM_053795 |
| NM_001006972 | NM_022188 | NM_053801 |
| NM_001007150 | NM_022249 | NM_053849 |
| NM_001007656 | NM_022264 | NM_053876 |
| NM_001009605 | NM_022399 | NM_053883 |
| NM_001009679 | NM_022511 | NM_053928 |
| NM_001011901 | NM_022688 | NM_053961 |
| NM_001011923 | NM_022860 | NM_057098 |
| NM_001011955 | NM_022867 | NM_057099 |
| NM_001011978 | NM_022934 | NM_057190 |
| NM_001011996 | NM_023957 | NM_130779 |
| NM_001012012 | NM_023979 | NM_133394 |
| NM_001012035 | NM_024139 | NM_133562 |
| NM_001012103 | NM_024403 | NM_133609 |
| NM_001012144 | NM_030830 | NM_134346 |
| NM_001012170 | NM_030841 | NM_138548 |
| NM_001013087 | NM_030869 | NM_138866 |
| NM_001013170 | NM_031030 | NM_138899 |
| NM_001014785 | NM_031070 | NM_138910 |
| NM_012600 | NM_031104 | NM_138911 |
| NM_012664 | NM_031119 | NM_139325 |
| NM_012670 | NM_031134 | NM_145098 |
| NM_012784 | NM_031569 | NM_145677 |
| NM_012869 | NM_031641 | NM_153730 |
| NM_013067 | NM_031642 | NM_172008 |
| NM_013083 | NM_031662 | NM_172039 |
| NM_013090 | NM_031693 | NM_172062 |
| NM_013174 | NM_031715 | NM_172074 |
| NM_013192 | NM_031763 | NM_173133 |
| NM_013219 | NM_031840 | NM_173154 |
| NM_017010 | NM_033349 | NM_173309 |
| NM_017011 | NM_053291 | NM_181377 |
| NM_017051 | NM_053301 | NM_181379 |
| NM_198726 | XM_218717 | XM_239504 |
| NM_199086 | XM_220178 | XM_340906 |
| NM_199091 | XM_220219 | XM_340916 |
| NM_199373 | XM_220428 | XM_341090 |
| NM_212490 | XM_220604 | XM_341157 |
| XM_213234 | XM_220698 | XM_341314 |
| XM_213469 | XM_221023 | XM_341669 |
| XM_213906 | XM_221034 | XM_341763 |
| XM_214072 | XM_221043 | XM_342107 |
| XM_214153 | XM_221202 | XM_342295 |
| XM_214307 | XM_221962 | XM_342588 |
| XM_214554 | XM_222107 | XM_342823 |
| XM_214859 | XM_223580 | XM_342924 |
| XM_215069 | XM_223693 | XM_342930 |
| XM_215124 | XM_223921 | XM_343910 |
| XM_215751 | XM_223981 | XM_344048 |
| XM_215826 | XM_224733 | XM_347168 |
| XM_215883 | XM_226888 | XM_574001 |
| XM_215931 | XM_227499 | XM_576494 |
| XM_216013 | XM_230523 | XM_577170 |
| XM_216180 | XM_230531 | |
| XM_216212 | XM_231176 | |
| XM_216766 | XM_231925 | **UniGene ID** |
| XM_216910 | XM_232735 | Rn.102180 |
| XM_217147 | XM_233544 | Rn.21816 |
| XM_217464 | XM_235500 | Rn.24317 |
| XM_217893 | XM_238177 | |
| XM_218084 | XM_238346 | |
| XM_218186 | XM_239171 | |

The genes in this set show abundance of expressed gene product(s) in the Aged Impaired population that are significantly decreased, as determined by ANOVA, compared to those abundances in Young and Aged Unimpaired populations combined. In addition, the abundance of expressed gene product(s) in the aged animals negatively correlate with learning index of the animals, such that poorer learners have lower abundances of expressed gene products of the selected genes. Therefore, compounds useful in treating cognitive impairment selected using these genes should increase the abundance or enhance the function of the expressed gene products of these genes.

### Example 11

An ANOVA was conducted on the probe set signal values for all present probe sets by combining two groups of animals and comparing them to the third group. An "AI ANOVA" was performed, where Aged Unimpaired were combined with Young and compared to Aged Impaired. Any probe sets having a p-value less than 0.05 were considered significantly changed and the genes or plurality of genes selected.

### Microarray Results

Table 11 shows the genes that were identified as being associated with cognitive impairment in this experiment. The reference sequence transcript ID (GENBANK® number for the exemplar sequence) was used to identify genes considered significantly changed. When this exemplar ID was not available, a UniGene identifier was used in conjunction with the GENBANK® accession number.

**TABLE 11**

| **CA3 - AI ANOVA INCREASE** | | |
|---|---|---|
| | | |

| **GENBANK® ID** | **GENBANK® ID** | **GENBANK® ID** |
|---|---|---|
| BI295776 | NM_012507 | NM_019269 |
| AI178069 | NM_012532 | NM_019311 |
| AF022087 | NM_012543 | NM_019318 |
| AF155825 | NM_012654 | NM_019359 |
| AF187100 | NM_012655 | NM_020073 |
| M17422 | NM_012714 | NM_020084 |
| NM_001005539 | NM_012747 | NM_021663 |
| NM_001005761 | NM_012749 | NM_021672 |
| NM_001007002 | NM_012776 | NM_021681 |
| NM_001007005 | NM_012788 | NM_021694 |
| NM_001007145 | NM_012844 | NM_021746 |
| NM_001007607 | NM_012908 | NM_021853 |
| NM_001007628 | NM_012913 | NM_022184 |
| NM_001007636 | NM_012948 | NM_022198 |
| NM_001007641 | NM_012984 | NM_022226 |
| NM_001007744 | NM_013015 | NM_022236 |
| NM_001008306 | NM_013016 | NM_022250 |
| NM_001008316 | NM_013044 | NM_022270 |
| NM_001008356 | NM_013088 | NM_022272 |
| NM_001008509 | NM_013107 | NM_022390 |
| NM_001008767 | NM_013132 | NM_022500 |
| NM_001009623 | NM_013176 | NM_022592 |
| NM_001011910 | NM_013198 | NM_022629 |
| NM_001011954 | NM_017009 | NM_022799 |
| NM_001011959 | NM_017024 | NM_024148 |
| NM_001011981 | NM_017030 | NM_024154 |
| NM_001012040 | NM_017060 | NM_024155 |
| NM_001012163 | NM_017062 | NM_024373 |
| NM_001012181 | NM_017075 | NM_024404 |
| NM_001012193 | NM_017109 | NM_030872 |
| NM_001012217 | NM_017125 | NM_031035 |
| NM_001012459 | NM_017148 | NM_031052 |
| NM_001012464 | NM_017192 | NM_031091 |
| NM_001013058 | NM_017200 | NM_031144 |
| NM_001013086 | NM_017347 | NM_031235 |
| NM_001013148 | NM_017365 | NM_031509 |
| NM_001013173 | NM_019140 | NM_031556 |
| NM_001013179 | NM_019146 | NM_031599 |
| NM_001013195 | NM_019152 | NM_031620 |
| NM_001013200 | NM_019156 | NM_031648 |
| NM_001014843 | NM_019168 | NM_031771 |
| NM_001024278 | NM_019252 | NM_031772 |
| NM_031797 | NM_145092 | XM_216565 |
| NM_031818 | NM_145678 | XM_217197 |
| NM_031837 | NM_145777 | XM_217239 |
| NM_031970 | NM_147206 | XM_217651 |
| NM_032083 | NM_147207 | XM_218195 |
| NM_053021 | NM_152790 | XM_218337 |
| NM_053323 | NM_153298 | XM_218523 |
| NM_053349 | NM_172029 | XM_218617 |
| NM_053418 | NM_172334 | XM_218939 |
| NM_053534 | NM_173118 | XM_218977 |
| NM_053538 | NM_173153 | XM_219785 |
| NM_053576 | NM_173328 | XM_219885 |
| NM_053749 | NM_175764 | XM_219948 |
| NM_053763 | NM_181363 | XM_220357 |
| NM_053794 | NM_181388 | XM_221497 |
| NM_053901 | NM_181639 | XM_223190 |
| NM_053917 | NM_182737 | XM_223535 |
| NM_053926 | NM_199093 | XM_223781 |
| NM_053985 | NM_199270 | XM_223786 |
| NM_053986 | NM_199390 | XM_224337 |
| NM_053994 | NM_199401 | XM_224944 |
| NM_054001 | NM_203335 | XM_225147 |
| NM_057139 | NM_207609 | XM_225160 |
| NM_057187 | NM_212508 | XM_227217 |
| NM_058210 | NM_212523 | XM_227444 |
| NM_058211 | NM_213628 | XM_228164 |
| NM_130420 | XM_213270 | XM_230284 |
| NM_131911 | XM_213329 | XM_231620 |
| NM_133298 | XM_213421 | XM_231749 |
| NM_133300 | XM_213484 | XM_232194 |
| NM_133303 | XM_213684 | XM_232343 |
| NM_133307 | XM_213688 | XM_232354 |
| NM_133318 | XM_214035 | XM_232364 |
| NM_233383 | XM_214403 | XM_232732 |
| NM_133398 | XM_214480 | XM_233141 |
| NM_133418 | XM_214967 | XM_233403 |
| NM_133534 | XM_214979 | XM_235057 |
| NM_133571 | XM_215080 | XM_235558 |
| NM_133615 | XM_215361 | XM_235566 |
| NM_134334 | XM_215576 | XM_236020 |
| NM_134349 | XM_215607 | XM_236227 |
| NM_134372 | XM_215889 | XM_237371 |
| NM_134411 | XM_215924 | XM_237415 |
| NM_138506 | XM_216004 | XM_237754 |
| NM_138521 | XM_216188 | XM_237907 |
| NM_139115 | XM_216386 | XM_240311 |
| XM_242062 | XM_343117 | XM_346854 |
| XM_242644 | XM_343205 | XM_573810 |
| XM_341494 | XM_343259 | XM_574618 |
| XM_341495 | XM_343274 | XM_575585 |
| XM_341548 | XM_343332 | XM_576312 |
| XM_341578 | XM_343395 | XM_576519 |
| XM_341584 | XM_343468 | |
| XM_341605 | XM_343552 | **UniGene ID** |
| XM_341957 | XM_343773 | Rn.110441 |
| XM_342007 | XM_343975 | Rn.129720 |
| XM_342291 | XM_343986 | |
| XM_342317 | XM_344112 | |
| XM_342545 | XM_344409 | |
| XM_342653 | XM_344785 | |
| XM_343055 | XM_345825 | |

The genes in this set show abundance of expressed gene product(s) in the Aged Impaired population that are significantly increased, as determined by ANOVA, compared to those abundances in Young and Aged Unimpaired populations combined. Therefore, compounds useful in treating cognitive impairment selected using these genes should decrease the abundance or attenuate the function of the expressed gene products of these genes.

### Example 12

An ANOVA was conducted on the probe set signal values for all present probe sets by combining two groups of animals and comparing them to the third group. An "AI ANOVA" was performed, where Aged Unimpaired were combined with Young and compared to Aged Impaired. Any probe sets having a p-value less than 0.05 were considered significantly changed and the genes or plurality of genes selected.

### Microarray Results

Table 12 shows the genes that were identified as being associated with cognitive impairment in this experiment. The reference sequence transcript ID (GENBANK® number for the exemplar sequence) was used to identify genes considered significantly changed. When this exemplar ID was not available, a UniGene identifier was used in conjunction with the GENBANK® accession number.

**TABLE 12**

| **CA3** - **AI ANOVA DECREASE** | | |
|---|---|---|
| | | |

| **GENBANK® ID** | **GENBANK® ID** | **GENBANK® ID** |
|---|---|---|
| BI294968 | NM_001011998 | NM_013002 |
| BF284573 | NM_001012060 | NM_013011 |
| AW142828 | NM_001012066 | NM_013019 |
| L13193 | NM_001012078 | NM_013038 |
| BG663483 | NM_001012119 | NM_013048 |
| AF452727 | NM_001012187 | NM_013081 |
| AY316590 | NM_001012191 | NM_013102 |
| NM_001000716 | NM_001012468 | NM_013111 |
| NM_001004072 | NM_001012473 | NM_013127 |
| NM_001004078 | NM_001013034 | NM_013134 |
| NM_001004132 | NM_001013059 | NM_013177 |
| NM_001004233 | NM_001013153 | NM_013214 |
| NM_001005547 | NM_001013156 | NM_013223 |
| NM_001005554 | NM_001013178 | NM_017025 |
| NM_001005905 | NM_001013189 | NM_017029 |
| NM_001005908 | NM_001013207 | NM_017039 |
| NM_001006970 | NM_001013235 | NM_017040 |
| NM_001006997 | NM_001014792 | NM_017042 |
| NM_001007020 | NM_001014793 | NM_017102 |
| NM_001007608 | NM_001015003 | NM_017107 |
| NM_001007626 | NM_001017374 | NM_017136 |
| NM_001007714 | NM_001024765 | NM_017195 |
| NM_001007728 | NM_012500 | NM_017232 |
| NM_001007742 | NM_012517 | NM_017243 |
| NM_001008301 | NM_012526 | NM_017246 |
| NM_001008323 | NM_012583 | NM_017268 |
| NM_001008338 | NM_012598 | NM_017270 |
| NM_001008339 | NM_012647 | NM_017282 |
| NM_001008520 | NM_012663 | NM_017318 |
| NM_001008558 | NM_012734 | NM_017340 |
| NM_001008562 | NM_012736 | NM_017343 |
| NM_001008766 | NM_012757 | NM_017359 |
| NM_001008888 | NM_012839 | NM_017364 |
| NM_001009180 | NM_012892 | NM_019124 |
| NM_001009258 | NM_012923 | NM_019131 |
| NM_001009424 | NM_012932 | NM_019169 |
| NM_001009666 | NM_012952 | NM_019196 |
| NM_001009677 | NM_012953 | NM_019264 |
| NM_001009688 | NM_012956 | NM_019277 |
| NM_001009967 | NM_012960 | NM_019302 |
| NM_001010965 | NM_012963 | NM_019304 |
| NM_001011969 | NM_012985 | NM_019356 |
| NM_021655 | NM_031802 | NM_057107 |
| NM_021697 | NM_031811 | NM_057141 |
| NM_021758 | NM_031824 | NM_057196 |
| NM_021852 | NM_031987 | NM_057210 |
| NM_022008 | NM_032057 | NM_080777 |
| NM_022209 | NM_032614 | NM_080780 |
| NM_022262 | NM_033021 | NM_080886 |
| NM_022387 | NM_053346 | NM_080887 |
| NM_022498 | NM_053357 | NM_080902 |
| NM_022542 | NM_053409 | NM_130423 |
| NM_022585 | NM_053414 | NM_130746 |
| NM_022609 | NM_053420 | NM_130749 |
| NM_022674 | NM_053428 | NM_130894 |
| NM_022700 | NM_053439 | NM_131904 |
| NM_022865 | NM_053441 | NM_133313 |
| NM_022869 | NM_053484 | NM_133320 |
| NM_022939 | NM_053502 | NM_133402 |
| NM_023093 | NM_053518 | NM_133415 |
| NM_023950 | NM_053527 | NM_133427 |
| NM_023971 | NM_053581 | NM_133566 |
| NM_023975 | NM_053590 | NM_133589 |
| NM_024125 | NM_053605 | NM_134331 |
| NM_024152 | NM_053613 | NM_134383 |
| NM_024156 | NM_053622 | NM_134404 |
| NM_024378 | NM_053623 | NM_138519 |
| NM_024484 | NM_053638 | NM_138833 |
| NM_024486 | NM_053726 | NM_138839 |
| NM_030835 | NM_053747 | NM_138856 |
| NM_030856 | NM_053748 | NM_138883 |
| NM_031064 | NM_053752 | NM_139097 |
| NM_031138 | NM_053758 | NM_139098 |
| NM_031146 | NM_053764 | NM_139106 |
| NM_031151 | NM_053772 | NM_139254 |
| NM_031152 | NM_053812 | NM_145184 |
| NM_031237 | NM_053842 | NM_147211 |
| NM_031606 | NM_053856 | NM_152935 |
| NM_031639 | NM_053868 | NM_153297 |
| NM_031643 | NM_053888 | NM_153630 |
| NM_031655 | NM_053893 | NM_153735 |
| NM_031718 | NM_053912 | NM_171990 |
| NM_031720 | NM_053927 | NM_171994 |
| NM_031735 | NM_053948 | NM_172072 |
| NM_031742 | NM_053974 | NM_173102 |
| NM_031745 | NM_053979 | NM_173146 |
| NM_031757 | NM_053996 | NM_173290 |
| NM_031783 | NM_054002 | NM_177425 |
| NM_031785 | NM_054003 | NM_177929 |
| NM_178091 | XM_215286 | XM_236698 |
| NM_181090 | XM_215416 | XM_237808 |
| NM_181626 | XM_215549 | XM_238280 |
| NM_182668 | XM_215570 | XM_238770 |
| NM_182814 | XM_215612 | XM_239761 |
| NM_182819 | XM_216158 | XM_340775 |
| NM_183052 | XM_216265 | XM_340872 |
| NM_183332 | XM_216378 | XM_340889 |
| NM_184050 | XM_216641 | XM_340921 |
| NM_184051 | XM_216893 | XM_340999 |
| NM_198132 | XM_217105 | XM_341288 |
| NM_198732 | XM_217209 | XM_341374 |
| NM_198758 | XM_217560 | XM_341448 |
| NM_198760 | XM_217592 | XM_341700 |
| NM_198765 | XM_217868 | XM_341822 |
| NM_198787 | XM_218620 | XM_341930 |
| NM_198788 | XM_218660 | XM_342149 |
| NM_199385 | XM_219377 | XM_342174 |
| NM_199395 | XM_219525 | XM_342217 |
| NM_199405 | XM_219939 | XM_342300 |
| NM_199410 | XM_220230 | XM_342306 |
| NM_199500 | XM_220281 | XM_342312 |
| NM_207617 | XM_220717 | XM_342493 |
| NM_212494 | XM_220754 | XM_342534 |
| NM_212519 | XM_221212 | XM_342553 |
| NM_212520 | XM_221888 | XM_342582 |
| XM_213362 | XM_221910 | XM_342600 |
| XM_213426 | XM_222669 | XM_342612 |
| XM_213487 | XM_222717 | XM_342679 |
| XM_213564 | XM_223729 | XM_342812 |
| XM_213571 | XM_224271 | XM_342894 |
| XM_213679 | XM_225078 | XM_342920 |
| XM_213765 | XM_226561 | XM_343154 |
| XM_213782 | XM_227301 | XM_343389 |
| XM_213840 | XM_228345 | XM_343459 |
| XM_214003 | XM_230495 | XM_343557 |
| XM_214108 | XM_231271 | XM_343761 |
| XM_214147 | XM_231803 | XM_343776 |
| XM_214241 | XM_232220 | XM_344231 |
| XM_214475 | XM_232739 | XM_344434 |
| XM_214485 | XM_232901 | XM_344594 |
| XM_214491 | XM_233485 | XM_573052 |
| XM_214583 | XM_233529 | XM_573063 |
| XM_214646 | XM_234483 | XM_573256 |
| XM_214701 | XM_234555 | XM_574916 |
| XM_214969 | XM_235185 | XM_578542 |
| XM_215251 | XM_235878 | XM_579413 |

| **UniGene ID** | | |
|---|---|---|
| Rn.128732 | | |
| Rn.15446 | | |
| Rn.23342 | | |
| Rn.25029 | | |
| Rn.92383 | | |

The genes in this set show abundance of expressed gene product(s) in the Aged Impaired population that are significantly decreased, as determined by ANOVA, compared to those abundances in Young and Aged Unimpaired populations combined. Therefore, compounds useful in treating cognitive impairment selected using these genes should increase the abundance or enhance the function of the expressed gene products of these genes.

### Example 13

An ANOVA was conducted on the probe set signal values for all present probe sets by combining two groups of animals and comparing them to the third group. An "AU ANOVA" was performed, where Aged Impaired were combined with Young and compared to Aged Unimpaired. Any probe sets having a p-value less than 0.05 were considered significantly changed and the genes or plurality of genes selected.

Pearson's correlations comparing probe set signal values to learning indices were then calculated for the aged animals (excluding young) across all present probe sets. Again, correlations representing a p-value of less than 0.05 were considered significantly changed.

### Microarray Results

Table 13 shows the genes that were identified as being associated with cognitive impairment in this experiment. The reference sequence transcript ID (GENBANK® number for the exemplar sequence) was used to identify genes considered significantly changed. When this exemplar ID was not available, a UniGene identifier was used in conjunction with the GENBANK® accession number.

**TABLE 13**

| **CA3 - AU ANOVA POSITIVE CORRELATION** | | |
|---|---|---|
| | | |

| **GENBANK® ID** | **GENBANK® ID** | **GENBANK® ID** |
|---|---|---|
| BF419095 | NM_021678 | NM_133579 |
| BF420440 | NM_021767 | NM_134413 |
| NM_001005889 | NM_021835 | NM_138502 |
| NM_001009665 | NM_021847 | NM_138509 |
| NM_001009825 | NM_022254 | NM_138896 |
| NM_001011974 | NM_022380 | NM_139060 |
| NM_001012038 | NM_022690 | NM_139189 |
| NM_001012061 | NM_022703 | NM_139217 |
| NM_001012150 | NM_022850 | NM_144756 |
| NM_001012183 | NM_022946 | NM_148891 |
| NM_001012504 | NM_023020 | NM_153309 |
| NM_001013128 | NM_023983 | NM_153317 |
| NM_012504 | NM_024366 | NM_173152 |
| NM_012518 | NM_024397 | NM_181550 |
| NM_012527 | NM_030871 | XM_213626 |
| NM_012574 | NM_031028 | XM_213746 |
| NM_012633 | NM_031036 | XM_213969 |
| NM_012836 | NM_031081 | XM_214253 |
| NM_012920 | NM_031515 | XM_215403 |
| NM_013029 | NM_031535 | XM_215467 |
| NM_013065 | NM_031608 | XM_215578 |
| NM_013066 | NM_031665 | XM_215919 |
| NM_013126 | NM_031743 | XM_215990 |
| NM_013135 | NM_032613 | XM_217021 |
| NM_017065 | NM_053311 | XM_218828 |
| NM_017066 | NM_053369 | XM_220232 |
| NM_017211 | NM_053424 | XM_221426 |
| NM_017213 | NM_053457 | XM_223205 |
| NM_017242 | NM_053475 | XM_223837 |
| NM_017262 | NM_053508 | XM_224474 |
| NM_017290 | NM_053589 | XM_227623 |
| NM_017304 | NM_053859 | XM_228644 |
| NM_017327 | NM_053878 | XM_233798 |
| NM_019128 | NM_053891 | XM_234901 |
| NM_019204 | NM_053910 | XM_235179 |
| NM_019275 | NM_057116 | XM_236268 |
| NM_019351 | NM_080482 | XM_236362 |
| NM_019375 | NM_080583 | XM_237241 |
| NM_020075 | NM_080904 | XM_238072 |
| NM_020088 | NM_131907 | XM_238336 |
| NM_021597 | NM_133395 | XM_241981 |
| XM_340747 | XM_342808 | XM_573205 |
| XM_341081 | XM_343059 | XM_573259 |
| XM_341104 | XM_343109 | XM_573634 |
| XM_341201 | XM_343157 | XM_576311 |
| XM_341341 | XM_343427 | XM_579869 |
| XM_341857 | XM_343483 | |
| XM_341961 | XM_343513 | |
| XM_342521 | XM_343582 | **UniGene ID** |
| XM_342535 | XM_343630 | Rn.122667 |
| XM_342682 | XM_343736 | Rn.78244 |
| XM_342684 | XM_343764 | |

The genes in this set show abundance of expressed gene product(s) in the Aged Unimpaired population that are significantly decreased, as determined by ANOVA, compared to those abundances in Young and Aged Impaired populations combined. In addition, the abundance of expressed gene product(s) in the aged animals positively correlate with learning index of the animals, such that poorer learners have higher abundances of expressed gene products of the selected genes. Therefore, compounds useful in treating cognitive impairment selected using these genes should decrease the abundance or attenuate the function of the expressed gene products of these genes.

### Example 14

An ANOVA was conducted on the probe set signal values for all present probe sets by combining two groups of animals and comparing them to the third group. An "AU ANOVA" was performed, where Aged Impaired were combined with Young and compared to Aged Unimpaired. Any probe sets having a p-value less than 0.05 were considered significantly changed and the genes or plurality of genes selected.

Pearson's correlations comparing probe set signal values to learning indices were then calculated for the aged animals (excluding young) across all present probe sets. Again, correlations representing a p-value of less than 0.05 were considered significantly changed.

### Microarray Results

Table 14 shows the genes that were identified as being associated with cognitive impairment in this experiment. The reference sequence transcript ID (GENBANK® number for the exemplar sequence) was used to identify genes considered significantly changed. When this exemplar ID was not available, a UniGene identifier was used in conjunction with the GENBANK® accession number.

**TABLE 14**

| **CA3 - AU ANOVA NEGATIVE CORRELATION** | | |
|---|---|---|
| | | |

| **GENBANK® ID** | **GENBANK® ID** | **GENBANK® ID** |
|---|---|---|
| NK_001003978 | NM_032079 | XM_216380 |
| NM_001004075 | NM_053295 | XM_216661 |
| NM_001006991 | NM_053442 | XM_217050 |
| NM_001011905 | NM_053539 | XM_219297 |
| NM_001011992 | NM_053642 | XM_221050 |
| NM_001012160 | NM_053713 | XM_224588 |
| NM_001013206 | NM_053714 | XM_231052 |
| NM_001013224 | NM_053787 | XM_232168 |
| NK_001013234 | NM_130413 | XM_232860 |
| NM_001013873 | NM_134326 | XM_233767 |
| NM_012645 | NM_134456 | XM_235051 |
| NM_013154 | NM_145778 | XM_236210 |
| NM_019364 | NM_145785 | XM_237828 |
| NM_020308 | NM_171995 | XM_243652 |
| NM_022210 | NM_173117 | XM_340911 |
| NM_022394 | NM_175579 | XM_341750 |
| MM_022541 | NM_182816 | XM_342032 |
| NM_024374 | XM_213658 | XM_342044 |
| NM_024385 | XM_213993 | XM_342396 |
| NM_031062 | XM_214730 | XM_342578 |
| NM_031345 | XM_214890 | XM_345160 |
| NM_031596 | XM_215637 | XM_578548 |
| MM_031978 | XM_215908 | |

The genes in this set show abundance of expressed gene product(s) in the Aged Unimpaired population that are significantly increased, as determined by ANOVA, compared to those abundances in Young and Aged Impaired populations combined. In addition, the abundance of expressed gene product(s) in the aged animals negatively correlate with learning index of the animals, such that poorer learners have lower abundances of expressed gene products of the selected genes. Therefore, compounds useful in treating cognitive impairment selected using these genes should increase the abundance or enhance the function of the expressed gene products of these genes.

### Example 15

An ANOVA was conducted on the probe set signal values for all present probe sets by combining two groups of animals and comparing them to the third group. An "AU ANOVA" was performed, where Aged Impaired were combined with Young and compared to Aged Unimpaired. Any probe sets having a p-value less than 0.05 were considered significantly changed and the genes or plurality of genes selected.

### Microarray Results

Table 15 shows the genes that were identified as being associated with cognitive impairment in this experiment. The reference sequence transcript ID (GENBANK® number for the exemplar sequence) was used to identify genes considered significantly changed. When this exemplar ID was not available, a UniGene identifier was used in conjunction with the GENBANK® accession number.

**TABLE 15**

| **CA3** - **AU ANOVA INCREASE** | | |
|---|---|---|
| | | |

| **GENBANK® ID** | **GENBANK® ID** | **GENBANK® ID** |
|---|---|---|
| BF287008 | NM_012988 | NM_053832 |
| AF442812 | NM_013001 | NM_053884 |
| AY597251 | NM_013022 | NM_053946 |
| NM_001002854 | NM_013055 | NM_053998 |
| NM_001004199 | NM_017116 | NM_130409 |
| NM_001004443 | NM_017264 | NM_133314 |
| NM_001005557 | NM_019238 | NM_133387 |
| NM_001006602 | NM_021763 | NM_133548 |
| NM_001006976 | NM_021846 | NM_133552 |
| NM_001007686 | NM_021863 | NM_134389 |
| NM_001007734 | NM_021997 | NM_138508 |
| NM_001008309 | NM_022197 | NM_138846 |
| NM_001008515 | NM_022219 | NM_138847 |
| NM_001008521 | NM_022282 | MM_138907 |
| NM_001009349 | NM_022515 | NM_139083 |
| NM_001009369 | NM_022599 | NM_139094 |
| NM_001009632 | NM_022626 | NM_145783 |
| NM_001009641 | NM_022941 | NM_148890 |
| NM_001009645 | NM_022949 | NM_172324 |
| NM_001009973 | NM_024144 | NM_173323 |
| NM_001011896 | NM_024150 | NM_175756 |
| NM_001011948 | NM_024377 | NM_175762 |
| NM_001012021 | NM_024388 | NM_177933 |
| NM_001012133 | NM_031078 | NM_181432 |
| NM_001013033 | NM_031083 | NM_181628 |
| NM_001013082 | NM_031094 | NM_182844 |
| NM_001013213 | NM_031122 | NM_183331 |
| NM_001013223 | NM_031622 | NM_183402 |
| NM_001013233 | NM_031640 | NM_199111 |
| NM_012512 | NM_031721 | NM_201988 |
| NM_012550 | NM_031728 | U31866 |
| NM_012562 | NM_031751 | XM_213324 |
| NM_012656 | NM_032612 | XM_213346 |
| NM_012731 | NM_033096 | XM_213418 |
| NM_012857 | NM_052809 | XM_213823 |
| NM_012870 | NM_053455 | XM_214958 |
| NM_012887 | NM_053541 | XM_215037 |
| NM_012895 | NM_053580 | XM_215469 |
| NM_012904 | NM_053584 | XM_215528 |
| NM_012935 | NM_053698 | XM_215812 |
| NM_012939 | NM_053774 | XM_215858 |
| NM_012940 | NM_053826 | XM_216316 |
| XM_216757 | XM_232608 | XM_341688 |
| XM_218041 | XM_233138 | XM_342002 |
| XM_218759 | XM_233341 | XM_342154 |
| XM_220606 | XM_233611 | XM_342470 |
| XM_220736 | XM_233820 | XM_343126 |
| XM_221077 | XM_233944 | XM_343318 |
| XM_222152 | XM_234328 | XM_345114 |
| XM_223508 | XM_234441 | XM_574371 |
| XM_223684 | XM_238163 | XM_579460 |
| XM_224971 | XM_242005 | XM_579522 |
| XM_225039 | XM_242556 | |
| XM_225644 | XM_340943 | **UniGene ID** |
| XM_226769 | XM_341066 | Rn.14733 |
| XM_228197 | XM_341251 | |
| XM_228305 | XM_341346 | |
| XM_232202 | XM_341444 | |
| XM_232466 | XM_341644 | |

The genes in this set show abundance of expressed gene product(s) in the Aged Unimpaired population that are significantly increased, as determined by ANOVA, compared to those abundances in Young and Aged Impaired populations combined. Therefore, compounds useful in treating cognitive impairment selected using these genes should increase the abundance or enhance the function of the expressed gene products of these genes.

### Example 16

An ANOVA was conducted on the probe set signal values for all present probe sets by combining two groups of animals and comparing them to the third group. An "AU ANOVA" was performed, where Aged Impaired were combined with Young and compared to Aged Unimpaired. Any probe sets having a p-value less than 0.05 were considered significantly changed and the genes or plurality of genes selected.

### Microarray Results

Table 16 shows the genes that were identified as being associated with cognitive impairment in this experiment. The reference sequence transcript ID (GENBANK® number for the exemplar sequence) was used to identify genes considered significantly changed. When this exemplar ID was not available, a UniGene identifier was used in conjunction with the GENBANK® accession number.

**TABLE 16**

| **CA3** - **ID ANOVA DECREASE** | | |
|---|---|---|
| | | |

| **GENBANK® ID** | **GENBANK® ID** | **GENBANK® ID** |
|---|---|---|
| AI171809 | NM_017259 | NM_053464 |
| BI395698 | NM_017298 | NM_053503 |
| NM_001004083 | NM_017312 | NM_053553 |
| NM_001006971 | NM_019159 | NM_053675 |
| NM_001007616 | NM_019208 | NM_053721 |
| NM_001008519 | NM_019295 | NM_053724 |
| NM_001008694 | NM_019306 | NM_053777 |
| NM_001009681 | NM_019343 | NM_053788 |
| NM_001011930 | NM_020094 | NM_053811 |
| NM_001011938 | NM_021748 | NM_053864 |
| NM_001011939 | NM_021775 | NM_053894 |
| NM_001012004 | NM_021859 | NM_053895 |
| NM_001012175 | NM_022193 | NM_053903 |
| NM_001012189 | NM_022206 | NM_053924 |
| NM_001012345 | NM_022217 | NM_053931 |
| NM_001013045 | NM_022252 | NM_053997 |
| NM_001013094 | NM_022267 | NM_057118 |
| NM_001013096 | NM_022382 | NM_057125 |
| NM_001013138 | NM_022384 | NM_057140 |
| NM_001013186 | NM_022589 | NM_057152 |
| NM_012506 | NM_022606 | NM_057201 |
| NM_012508 | NM_022666 | NM_080885 |
| NM_012545 | NM_022668 | NM_133406 |
| NM_012569 | NM_022675 | NM_133419 |
| NM_012581 | NM_022855 | NM_133535 |
| NM_012673 | NM_022864 | NM_133567 |
| NM_012686 | NM_022945 | NM_133582 |
| NM_012756 | NM_023988 | NM_134366 |
| NM_012798 | NM_024000 | NM_134376 |
| NM_012820 | NM_024146 | NM_134398 |
| NM_012830 | NM_024394 | NM_134455 |
| NM_012841 | NM_030858 | NM_134458 |
| NM_012918 | NM_030862 | NM_134461 |
| NM_013116 | NM_031008 | NM_139329 |
| NM_013181 | NM_031315 | NM_139330 |
| NM_013189 | NM_031353 | NM_139333 |
| NM_016990 | NM_031568 | NM_145089 |
| NM_017093 | NM_031657 | NM_145090 |
| NM_017094 | NM_031667 | NM_145680 |
| NM_017131 | NM_031821 | NM_147209 |
| NM_017204 | NM_031831 | NM_148889 |
| NM_017253 | NM_053335 | NM_173105 |
| NM_017254 | NM_053440 | NM_175708 |
| NM_175754 | XM_216398 | XM_235496 |
| NM_175760 | XM_216725 | XM_235662 |
| NM_175761 | XM_216965 | XM_236009 |
| NM_175869 | XM_217078 | XM_236735 |
| NM_177481 | XM_217283 | XM_238398 |
| NM_178106 | XM_217381 | XM_340886 |
| NM_181478 | XM_217388 | XM_341091 |
| NM_181822 | XM_218615 | XM_341248 |
| NM_182842 | XM_220055 | XM_341500 |
| NM_199372 | XM_220062 | XM_341807 |
| NM_199397 | XM_220224 | XM_343114 |
| NM_212458 | XM_220315 | XM_343175 |
| XM_213574 | XM_222103 | XM_343339 |
| XM_213777 | XM_222177 | XM_343839 |
| XM_213842 | XM_222245 | XM_345861 |
| XM_213925 | XM_222773 | XM_573915 |
| XM_213954 | XM_224713 | XM_575018 |
| XM_213963 | XM_225468 | XM_575080 |
| XM_214751 | XM_227282 | XM_579523 |
| XM_214780 | XM_227409 | XM_579696 |
| XM_214888 | XM_227428 | |
| XM_215182 | XM_228753 | **UniGene ID** |
| XM_215371 | XM_230814 | Rn.129749 |
| XM_215655 | XM_230899 | Rn.116507 |
| XM_215963 | XM_232351 | |
| XM_215984 | XM_233737 | |
| XM_216152 | XM_233839 | |
| XM_216228 | XM_235064 | |
| XM_216349 | XM_235156 | |

The genes in this set show abundance of expressed gene product(s) in the Aged Unimpaired population that are significantly decreased, as determined by ANOVA, compared to those abundances in Young and Aged Impaired populations combined. Therefore, compounds useful in treating cognitive impairment selected using these genes should decrease the abundance or attenuate the function of the expressed gene products of these genes.

### Example 17

An ANOVA was conducted on the probe set signal values for all present probe sets by combining two groups of animals and comparing them to the third group. An "AI ANOVA" was performed, where Aged Unimpaired were combined with Young and compared to Aged Impaired. Any probe sets having a p-value less than 0.05 were considered significantly changed and the genes or plurality of genes selected.

Pearson's correlations comparing probe set signal values to learning indices were then calculated for the aged animals (excluding young) across all present probe sets. Again, correlations representing a p-value of less than 0.05 were considered significantly changed.

### Microarray Results

Table 17 shows the genes that were identified as being associated with cognitive impairment in this experiment. The reference sequence transcript ID (GENBANK® number for the exemplar sequence) was used to identify genes considered significantly changed. When this exemplar ID was not available, a UniGene identifier was used in conjunction with the GENBANK® accession number.

**TABLE 17**

| **DG - AI ANOVA POSITIVE CORRELATION** | | |
|---|---|---|
| **GENBANK® ID** | **GENBANK® ID** | **GENBANK® ID** |
| NM_001001516 | NM_012820 | NM_031105 |
| NM_001004080 | NM_012893 | NM_031120 |
| NM_001004081 | NM_013000 | NM_031357 |
| NM_001004199 | NM_013015 | NM_031514 |
| NM_001004209 | NM_013107 | NM_031576 |
| NM_001004225 | NM_013135 | NM_031587 |
| NM_001004247 | NM_017051 | NM_031599 |
| NM_001004273 | NM_017052 | NM_031632 |
| NM_001004275 | NM_017068 | NM_031654 |
| NM_001005539 | NM_017075 | NM_031725 |
| NM_001006987 | NM_017109 | NM_031798 |
| NM_001006998 | NM_017142 | NM_032612 |
| NM_001007684 | NM_017181 | NM_053295 |
| NM_001007691 | NM_017232 | NM_053418 |
| NM_001008312 | NM_017288 | NM_053588 |
| NM_001008324 | NM_019124 | NM_053598 |
| NM_001008374 | NM_019131 | NM_053612 |
| NM_001009618 | NM_019159 | NM_053670 |
| NM_001009677 | NM_019204 | NM_053698 |
| NM_001009708 | NM_019257 | NM_053777 |
| NM_001011890 | NM_019359 | NM_053870 |
| NM_001011910 | NM_020308 | NM_053945 |
| NM_001011946 | NM_021766 | NM_057114 |
| NM_001011999 | NM_021869 | NM_080888 |
| NM_001012137 | NM_022400 | NM_130416 |
| NM_001012149 | NM_022499 | NM_130420 |
| NM_001012235 | NM_022500 | NM_130894 |
| NM_001013105 | NM_022502 | NM_133551 |
| NM_001013167 | NM_022523 | NM_133557 |
| NM_001013175 | NM_022539 | NM_134367 |
| NM_001013206 | NM_022592 | NM_134370 |
| NM_001013213 | NM_022595 | NM_138508 |
| NM_001013874 | NM_022596 | NM_138900 |
| NM_001017385 | NM_022617 | NM_138917 |
| NM_012543 | NM_022637 | NM_139107 |
| NM_012577 | NM_022853 | NM_139186 |
| NM_012628 | NM_024359 | NM_147210 |
| NM_012686 | NM_024400 | NM_153469 |
| NM_012701 | NM_030859 | NM_172063 |
| NM_012749 | NM_031035 | NM_172068 |
| NM_012777 | NM_031049 | NM_173111 |
| NM_012781 | NM_031057 | NM_175578 |
| NM_012788 | NM_031101 | NM_175582 |

| **GENBANK® ID** | **GENBANK® ID** | **GENBANK® ID** |
|---|---|---|
| NM_199119 | XM_219374 | XM_342318 |
| NM_207591 | XM_220264 | XM_342757 |
| NM_207602 | XM_220699 | XM_342887 |
| XM_214172 | XM_221387 | XM_343119 |
| XM_214383 | XM_223190 | XM_343318 |
| XM_214518 | XM_224944 | XM_343773 |
| XM_214588 | XM_225726 | XM_345674 |
| XM_214968 | XM_227066 | XM_346244 |
| XM_215069 | XM_228114 | XM_346854 |
| XM_215371 | XM_230637 | XM_573772 |
| XM_215754 | XM_232323 | XM_574670 |
| XM_215939 | XM_240330 | XM_579675 |
| XM_216641 | XM_341249 | |
| XM_216740 | XM_341785 | |
| XM_216872 | XM_341957 | |
| XM_218425 | XM_342092 | |

The genes in this set show abundance of expressed gene product(s) in the Aged Impaired population that are significantly increased, as determined by ANOVA, compared to those abundances in Young and Aged Unimpaired populations combined. In addition, the abundance of expressed gene product(s) in the aged animals positively correlate with learning index of the animals, such that poorer learners have higher abundances of expressed gene products of the selected genes. Therefore, compounds useful in treating cognitive impairment selected using these genes should decrease the abundance or attenuate the function of the expressed gene products of these genes.

### Example 18

An ANOVA was conducted on the probe set signal values for all present probe sets by combining two groups of animals and comparing them to the third group. An "AI ANOVA" was performed, where Aged Unimpaired were combined with Young and compared to Aged Impaired. Any probe sets having a p-value less than 0.05 were considered significantly changed and the genes or plurality of genes selected.

Pearson's correlations comparing probe set signal values to learning indices were then calculated for the aged animals (excluding young) across all present probe sets. Again, correlations representing a p-value of less than 0.05 were considered significantly changed.

### Microarray Results

Table 18 shows the genes that were identified as being associated with cognitive impairment in this experiment. The reference sequence transcript ID (GENBANK® number for the exemplar sequence) was used to identify genes considered significantly changed. When this exemplar ID was not available, a UniGene identifier was used in conjunction with the GENBANK® accession number.

**TABLE 18**

| **DG** - **ID ANOVA NEGATIVE CORRELATION** | | |
|---|---|---|
| **GENBANK® ID** | **GENBANK® ID** | **GENBANK® ID** |
| AI043800 | NM_031016 | XM_213969 |
| NM_001001512 | NM_031123 | XM_215883 |
| NM_001004208 | NM_031510 | XM_215890 |
| NM_001004446 | NM_031613 | XM_218226 |
| NM_001008353 | NM_031685 | XM_218515 |
| NM_001008520 | NM_031694 | XM_219879 |
| NM_001011923 | NM_031730 | XM_220708 |
| NM_001012025 | NM_053321 | XM_220992 |
| NM_001012072 | NM_053328 | XM_222103 |
| NM_001013873 | NM_053349 | XM_222717 |
| NM_001015021 | NM_053358 | XM_222773 |
| NM_012590 | NM_053360 | XM_224733 |
| NM_012609 | NM_053625 | XM_225895 |
| NM_012727 | NM_053669 | XM_231148 |
| NM_012769 | NM_053787 | XM_232413 |
| NM_012874 | NM_053883 | XM_234011 |
| NM_012959 | NM_080899 | XM_234470 |
| NM_012994 | NM_080902 | XM_236210 |
| NM_013083 | NM_131907 | XM_236614 |
| NM_013131 | NM_138506 | XM_241375 |
| NM_017290 | NM_138850 | XM_341089 |
| NM_019128 | NM_138911 | XM_341091 |
| NM_019347 | NM_138914 | XM_341106 |
| NM_020089 | NM_139192 | XM_341310 |
| NM_021758 | NM_139259 | XM_342044 |
| NM_021842 | NM_144758 | XM_342325 |
| NM_022281 | NM_145184 | XM_342612 |
| NM_022289 | NM_153317 | XM_342643 |
| NM_022297 | NM_172034 | XM_343263 |
| NM_022387 | NM_172062 | XM_343442 |
| NM_022506 | NM_173117 | XM_344785 |
| NM_022541 | NM_173119 | XM_345981 |
| NM_022546 | NM_173137 | XM_574916 |
| NM_022599 | NM_176857 | XM_576437 |
| NM_022934 | NM_181626 | |
| NM_022938 | NM_199106 | |
| NM_023977 | NM_201560 | |
| NM_024373 | XM_213762 | **UniGene ID** |
| NM_024375 | XM_213963 | Rn.18726 |

The genes in this set show abundance of expressed gene product(s) in the Aged Impaired population that are significantly decreased, as determined by ANOVA, compared to those abundances in Young and Aged Unimpaired populations combined. In addition, the abundance of expressed gene product(s) in the aged animals negatively correlate with learning index of the animals, such that poorer learners have lower abundances of expressed gene products of the selected genes. Therefore, compounds useful in treating cognitive impairment selected using these genes should increase the abundance or enhance the function of the expressed gene products of these genes.

### Example 19

An ANOVA was conducted on the probe set signal values for all present probe sets by combining two groups of animals and comparing them to the third group. An "AI ANOVA" was performed, where Aged Unimpaired were combined with Young and compared to Aged Impaired. Any probe sets having a p-value less than 0.05 were considered significantly changed and the genes or plurality of genes selected.

### Microarray Results

Table 19 shows the genes that were identified as being associated with cognitive impairment in this experiment. The reference sequence transcript ID (GENBANK® number for the exemplar sequence) was used to identify genes considered significantly changed. When this exemplar ID was not available, a UniGene identifier was used in conjunction with the GENBANK® accession number.

**TABLE 19**

| **DG - AI ANOVA INCREASE** | | |
|---|---|---|
| | | |

| **GENBANK® ID** | **GENBANK® ID** | **GENBANK® ID** |
|---|---|---|
| BI303253 | NM_012546 | NM_020073 |
| BF567886 | NM_012562 | NM_020074 |
| BE112341 | NM_012581 | NM_020075 |
| AW142720 | NM_012637 | NM_020088 |
| AI231999 | NM_012663 | NM_020099 |
| BF398752 | NM_012762 | NM_021690 |
| AF030087 | NM_012844 | NM_021769 |
| AY190520 | NM_012862 | NM_021909 |
| NM_001002016 | NM_012913 | NM_022210 |
| NM_001004076 | NM_012925 | NM_022226 |
| NM_001004099 | NM_012939 | NM_022270 |
| NM_001004218 | NM_012971 | NM_022382 |
| NM_001005907 | NM_012974 | NM_022390 |
| NM_001006989 | NM_012985 | NM_022526 |
| NM_001007557 | NM_012992 | NM_022530 |
| NM_001007625 | NM_012993 | NM_022531 |
| NM_001007654 | NM_013044 | NM_022601 |
| NM_001007712 | NM_013070 | NM_022638 |
| NM_001007729 | NM_013122 | NM_022692 |
| NM_001007749 | NM_013137 | NM_023967 |
| NM_001008287 | NM_013146 | NM_024155 |
| NM_001008725 | NM_013156 | NM_024353 |
| NM_001008767 | NM_013194 | NM_024358 |
| NM_001008768 | NM_017060 | NM_024369 |
| NM_001009474 | NM_017116 | NM_030826 |
| NM_001009502 | NM_017125 | NM_030831 |
| NM_001009662 | NM_017132 | NM_030863 |
| NM_001009669 | NM_017175 | NM_031013 |
| NM_001009719 | NM_017192 | NM_031099 |
| NM_001011985 | NM_017200 | NM_031114 |
| NM_001012004 | NM_017280 | NM_031140 |
| NM_001012088 | NM_017307 | NM_031328 |
| NM_001012147 | NM_017320 | NM_031509 |
| NM_001012162 | NM_017351 | NM_031511 |
| NM_001013044 | NM_019156 | NM_031525 |
| NM_001013112 | NM_019232 | NM_031544 |
| NM_001013121 | NM_019249 | NM_031593 |
| NM_001013179 | NM_019253 | NM_031677 |
| NM_001013194 | NM_019289 | NM_031683 |
| NM_001013210 | NM_019312 | NM_031778 |
| NM_001013233 | NM_019363 | NM_031789 |
| NM_001013249 | NM_019904 | NM_031797 |
| NM_012512 | NM_019906 | NM_031818 |
| NM_032067 | NM_177426 | XM_224969 |
| NM_032416 | NM_181365 | XM_225014 |
| NM_052983 | NM_183328 | XM_225259 |
| NM_053314 | NM_198134 | XM_226016 |
| NM_053356 | NM_198786 | XM_226211 |
| NM_053404 | NM_199093 | XM_228073 |
| NM_053411 | NM_199115 | XM_231620 |
| NM_053455 | NM_199118 | XM_233138 |
| NM_053485 | NM_199378 | XM_233345 |
| NM_053516 | NM_199388 | XM_235518 |
| NM_053560 | NM_207598 | XM_236380 |
| NM_053583 | NM_212463 | XM_236675 |
| NM_053584 | NM_212501 | XM_237371 |
| NM_053592 | XM_213972 | XM_237825 |
| NM_053618 | XM_213995 | XM_242644 |
| NM_053714 | XM_214441 | XM_243637 |
| NM_053783 | XM_214656 | XM_341107 |
| NM_053886 | XM_214927 | XM_341227 |
| NM_053896 | XM_215095 | XM_341352 |
| NM_053927 | XM_215222 | XM_341574 |
| NM_053936 | XM_215285 | XM_341578 |
| NM_053999 | XM_215576 | XM_341653 |
| NM_05710 | XM_215733 | XM_341657 |
| NM_057197 | XM_215840 | XM_341664 |
| NM_080480 | XM_215935 | XM_341790 |
| NM_080584 | XM_215942 | XM_341948 |
| NM_080691 | XM_215994 | XM_342007 |
| NM_080698 | XM_216410 | XM_342048 |
| NM_130409 | XM_217061 | XM_342409 |
| NM_133296 | XM_217152 | XM_342542 |
| NM_133298 | XM_217154 | XM_342591 |
| NM_133305 | XM_217271 | XM_342759 |
| NM_133605 | XM_217283 | XM_343006 |
| NM_134449 | XM_217297 | XM_343065 |
| NM_138542 | XM_217367 | XM_343126 |
| NM_138855 | XM_217690 | XM_343166 |
| NM_139110 | XM_218292 | XM_343174 |
| NM_139216 | XM_218816 | XM_343198 |
| NM_139327 | XM_220567 | XM_343332 |
| NM_153297 | XM_220736 | XM_343564 |
| NM_153628 | XM_220810 | XM_343570 |
| NM_172029 | XM_220982 | XM_343845 |
| NM_172033 | XM_222476 | XM_346594 |
| NM_172325 | XM_223524 | XM_574170 |
| NM_173118 | XM_223583 | XM_574618 |
| NM_173123 | XM_223690 | XM_575585 |
| NM_175756 | XM_223781 | |

| **UniGene ID** | | |
|---|---|---|
| Rn.105382 | | |
| Rn.108166 | | |
| Rn.114169 | | |
| Rn.128732 | | |
| Rn.24825 | | |
| Rn.48866 | | |

The genes in this set show abundance of expressed gene product(s) in the Aged Impaired population that are significantly increased, as determined by ANOVA, compared to those abundances in Young and Aged Unimpaired populations combined. Therefore, compounds useful in treating cognitive impairment selected using these genes should decrease the abundance or attenuate the function of the expressed gene products of these genes.

### Example 20

An ANOVA was conducted on the probe set signal values for all present probe sets by combining two groups of animals and comparing them to the third group. An "AI ANOVA" was performed, where Aged Unimpaired were combined with Young and compared to Aged Impaired. Any probe sets having a p-value less than 0.05 were considered significantly changed and the genes or plurality of genes selected.

### Microarray Results

Table 20 shows the genes that were identified as being associated with cognitive impairment in this experiment. The reference sequence transcript ID (GENBANK® number for the exemplar sequence) was used to identify genes considered significantly changed. When this exemplar ID was not available, a UniGene identifier was used in conjunction with the GENBANK® accession number.

**TABLE 20**

| **DG - AI ANOVA DECREASE** | | |
|---|---|---|
| | | |

| **GENBANK® ID** | **GENBANK® ID** | **GENBANK® ID** |
|---|---|---|
| BG381750 | NM_017311 | NM_057208 |
| BM391206 | NM_017318 | NM_080482 |
| BG374818 | NM_017328 | NM_080776 |
| AI406842 | NM_017357 | NM_080781 |
| BM388719 | NM_017359 | NM_130749 |
| BE103926 | NM_019356 | NM_133425 |
| NM_001001508 | NM_021597 | NM_133562 |
| NM_001002819 | NM_021849 | NM_133568 |
| NM_001003978 | NM_022188 | NM_133596 |
| NM_001004082 | NM_022254 | NM_134376 |
| NM_001004133 | NM_022262 | NM_134395 |
| NM_001005765 | NM_022264 | NM_134398 |
| NM_001006970 | NM_022532 | NM_138849 |
| NM_001007680 | NM_022548 | NM_138922 |
| NM_001007742 | NM_022615 | NM_139091 |
| NM_001008299 | NM_022668 | NM_145676 |
| NM_001008317 | NM_022678 | NM_145783 |
| NM_001009679 | NM_022688 | NM_153318 |
| NM_001009704 | NM_022962 | NM_172008 |
| NM_001012017 | NM_024484 | NM_173133 |
| NM_001012131 | NM_030842 | NM_173337 |
| NM_001012150 | NM_030846 | NM_183331 |
| NM_001012197 | NM_030871 | NM_198787 |
| NM_001012214 | NM_031005 | NM_207590 |
| NM_001013035 | NM_031054 | XM_213610 |
| NM_001013092 | NM_031325 | XM_213765 |
| NM_001013128 | NM_031563 | XM_214155 |
| NM_001014187 | NM_031573 | XM_214690 |
| NM_012585 | NM_031596 | XM_214856 |
| NM_012916 | NM_031727 | XM_215134 |
| NM_012934 | NM_031777 | XM_215573 |
| NM_013002 | NM_031977 | XM_215896 |
| NM_013060 | NM_052801 | XM_216047 |
| NM_013066 | NM_053492 | XM_216227 |
| NM_013111 | NM_053589 | XM_217063 |
| NM_013192 | NM_053642 | XM_217115 |
| NM_017011 | NM_053729 | XM_217147 |
| NM_017093 | NM_053779 | XM_217464 |
| NM_017105 | NM_053801 | XM_218660 |
| NM_017155 | NM_053834 | XM_219576 |
| NM_017171 | NM_053851 | XM_220992 |
| NM_017215 | NM_053891 | XM_221888 |
| NM_017303 | NM_053931 | XM_221910 |
| XM_221962 | XM_340739 | XM_343910 |
| XM_222946 | XM_340856 | XM_344063 |
| XM_223729 | XM_340999 | XM_344594 |
| XM_224335 | XM_341112 | XM_344744 |
| XM_224588 | XM_341337 | NM_134383 |
| XM_224707 | XM_341683 | NM_053410 |
| XM_224713 | XM_341688 | XM_579362 |
| XM_225138 | XM_341803 | |
| XM_225468 | XM_342134 | **UniGene ID** |
| XM_226888 | XM_342763 | Rn.101929 |
| XM_232197 | XM_342854 | Rn.11688 |
| XM_232760 | XM_342920 | Rn.39092 |
| XM_235705 | XM_343358 | Rn.6994 |
| XM_237288 | XM_343395 | Rn.8562 |
| XM_239171 | XM_343469 | Rn.98343 |

The genes in this set show abundance of expressed gene product(s) in the Aged Impaired population that are significantly decreased, as determined by ANOVA, compared to those abundances in Young and Aged Unimpaired populations combined. Therefore, compounds useful in treating cognitive impairment selected using these genes should increase the abundance or enhance the function of the expressed gene products of these genes.

### Example 21

An ANOVA was conducted on the probe set signal values for all present probe sets by combining two groups of animals and comparing them to the third group. An "AU ANOVA" was performed, where Aged Impaired were combined with Young and compared to Aged Unimpaired. Any probe sets having a p-value less than 0.05 were considered significantly changed and the genes or plurality of genes selected.

Pearson's correlations comparing probe set signal values to learning indices were then calculated for the aged animals (excluding young) across all present probe sets. Again, correlations representing a p-value of less than 0.05 were considered significantly changed.

### Microarray Results

Table 21 shows the genes that were identified as being associated with cognitive impairment in this experiment. The reference sequence transcript ID (GENBANK® number for the exemplar sequence) was used to identify genes considered significantly changed. When this exemplar ID was not available, a UniGene identifier was used in conjunction with the GENBANK® accession number.

**TABLE 21**

| **DG - AU ANOVA POSITIVE CORRELATION** | | |
|---|---|---|
| | | |

| **GENBANK® ID** | **GENBANK® ID** | **GENBANK® ID** |
|---|---|---|
| AF022087 | NM_053440 | XM_214420 |
| NM_001001514 | NM_053639 | XM_216349 |
| NM_001005534 | NM_053804 | XM_216661 |
| NM_001005547 | NM_053842 | XM_220574 |
| NM_001008301 | NM_053871 | XM_220754 |
| NM_001009825 | NM_053965 | XM_227749 |
| NM_001010953 | NM_057141 | XM_232531 |
| NM_001011895 | NM_080478 | XM_232809 |
| NM_001011920 | NM_130406 | XM_233341 |
| NM_001011941 | NM_133313 | XM_236181 |
| NM_001011990 | NM_152935 | XM_236992 |
| NM_012634 | NM_183402 | XM_340970 |
| NM_017346 | NM_184049 | XM_341550 |
| NM_019379 | NM_198785 | XM_341666 |
| NM_022675 | NM_199380 | XM_341796 |
| NM_022936 | X76489 | XM_342300 |
| NM_031034 | XM_213421 | XM_342592 |
| NM_031575 | XM_213484 | XM_342686 |
| NM_031631 | XM_213782 | XM_343513 |
| NM_031735 | XM_213824 | |
| NM_052981 | XM_214035 | |

The genes in this set show abundance of expressed gene product(s) in the Aged Unimpaired population that are significantly decreased, as determined by ANOVA, compared to those abundances in Young and Aged Impaired populations combined. In addition, the abundance of expressed gene product(s) in the aged animals positively correlate with learning index of the animals, such that poorer learners have higher abundances of expressed gene products of the selected genes. Therefore, compounds useful in treating cognitive impairment selected using these genes should decrease the abundance or attenuate the function of the expressed gene products of these genes.

### Example 22

An ANOVA was conducted on the probe set signal values for all present probe sets by combining two groups of animals and comparing them to the third group. An "AU ANOVA" was performed, where Aged Impaired were combined with Young and compared to Aged Unimpaired. Any probe sets having a p-value less than 0.05 were considered significantly changed and the genes or plurality of genes selected.

Pearson's correlations comparing probe set signal values to learning indices were then calculated for the aged animals (excluding young) across all present probe sets. Again, correlations representing a p-value of less than 0.05 were considered significantly changed.

### Microarray Results

Table 22 shows the genes that were identified as being associated with cognitive impairment in this experiment. The reference sequence transcript ID (GENBANK® number for the exemplar sequence) was used to identify genes considered significantly changed. When this exemplar ID was not available, a UniGene identifier was used in conjunction with the GENBANK® accession number.

**TABLE 22**

| **DG - AU ANOVA NEGATIVE CORRELATION** | | |
|---|---|---|
| | | |

| **GENBANK® ID** | **GENBANK® ID** | **GENBANK® ID** |
|---|---|---|
| NM_001004075 | NM_031036 | XM_224256 |
| NM_001004200 | NM_031131 | XM_224947 |
| NM_001005529 | NM_031354 | XM_225168 |
| NM_001007146 | NM_031569 | XM_225717 |
| NM_001008511 | NM_053499 | XM_227090 |
| NM_001008885 | NM_053733 | XM_230303 |
| NM_001011959 | NM_053961 | XM_230616 |
| NM_001012034 | NM_054006 | XM_232735 |
| NM_001012140 | NM_133411 | XM_233141 |
| NM_001013065 | NM_134326 | XM_235185 |
| NM_001013165 | NM_134373 | XM_235662 |
| NM_001013198 | NM_172039 | XM_239329 |
| NM_001017382 | NM_172317 | XM_242005 |
| NM_012630 | NM_175604 | XM_340742 |
| NM_012775 | NM_175764 | XM_341312 |
| NM_012806 | NM_182737 | XM_341842 |
| NM_012953 | NM_198750 | XM_342392 |
| NM_012955 | NM_199111 | XM_342828 |
| NM_012991 | NM_199400 | XM_343175 |
| NM_013031 | NM_199463 | XM_343278 |
| NM_013094 | NM_212496 | XM_343535 |
| NM_013159 | XM_213749 | XM_343559 |
| NM_013189 | XM_214344 | XM_347168 |
| NM_017158 | XM_215286 | XM_573428 |
| NM_017319 | XM_215813 | XM_576252 |
| NM_019123 | XM_216025 | XM_579545 |
| NM_019149 | XM_216548 | |
| NM_019168 | XM_216784 | |
| NM_019349 | XM_217359 | |
| NM_021688 | XM_218459 | |
| NM_021774 | XM_218523 | |
| NM_022946 | XM_219349 | |
| NM_024394 | XM_222242 | |
| NM_030872 | XM_223938 | |

The genes in this set show abundance of expressed gene product(s) in the Aged Unimpaired population that are significantly increased, as determined by ANOVA, compared to those abundances in Young and Aged Impaired populations combined. In addition, the abundance of expressed gene product(s) in the aged animals negatively correlate with learning index of the animals, such that poorer learners have lower abundances of expressed gene products of the selected genes. Therefore, compounds useful in treating cognitive impairment selected using these genes should increase the abundance or enhance the function of the expressed gene products of these genes.

### Example 23

An ANOVA was conducted on the probe set signal values for all present probe sets by combining two groups of animals and comparing them to the third group. An "AU ANOVA" was performed, where Aged Impaired were combined with Young and compared to Aged Unimpaired. Any probe sets having a p-value less than 0.05 were considered significantly changed and the genes or plurality of genes selected.

### Microarray Results

Table 23 shows the genes that were identified as being associated with cognitive impairment in this experiment. The reference sequence transcript ID (GENBANK® number for the exemplar sequence) was used to identify genes considered significantly changed. When this exemplar ID was not available, a UniGene identifier was used in conjunction with the GENBANK® accession number.

**TABLE 23**

| **DG - AU ANOVA INCREASE** | | |
|---|---|---|
| | | |

| **GENBANK® ID** | **GENBANK® ID** | **GENBANK® ID** |
|---|---|---|
| AF452647 | NM_022515 | XM_214895 |
| NM_001004230 | NM_022694 | XM_215184 |
| NM_001004279 | NM_022709 | XM_215372 |
| NM_001004449 | NM_022961 | XM_215424 |
| NM_001005565 | NM_024125 | XM_215566 |
| NM_001007011 | NM_024139 | XM_215602 |
| NM_001007613 | NM_030844 | XM_215949 |
| NM_001007637 | NM_030858 | XM_216112 |
| NM_001007739 | NM_031028 | XM_216191 |
| NM_001008309 | NM_031138 | XM_216515 |
| NM_001008316 | NM_031518 | XM_217326 |
| NM_001008339 | NM_031597 | XM_217825 |
| NM_001008515 | NM_031822 | XM_217837 |
| NM_001008521 | NM_053402 | XM_219045 |
| NM_001009619 | NM_053421 | XM_220506 |
| NM_001009653 | NM_053502 | XM_220606 |
| NM_001010965 | NM_053613 | XM_221030 |
| NM_001011942 | NM_053621 | XM_221635 |
| NM_001011979 | NM_053826 | XM_222251 |
| NM_001012159 | NM_053876 | XM_222832 |
| NM_001012174 | NM_053901 | XM_225866 |
| NM_001013036 | NM_057152 | XM_226237 |
| NM_001013163 | NM_057196 | XM_226722 |
| NM_012576 | NM_057204 | XM_230846 |
| NM_012583 | NM_130740 | XM_230856 |
| NM_012774 | NM_133405 | XM_231552 |
| NM_012858 | NM_134356 | XM_232855 |
| NM_012935 | NM_134411 | XM_233377 |
| NM_013220 | NM_134432 | XM_233535 |
| NM_016994 | NM_134457 | XM_233679 |
| NM_017138 | NM_138889 | XM_234017 |
| NM_017263 | NM_139253 | XM_234441 |
| NM_017294 | NM_153316 | XM_237291 |
| NM_019170 | NM_173837 | XM_238063 |
| NM_019251 | NM_175598 | XM_240417 |
| NM_019302 | NM_178101 | XM_340918 |
| NM_019374 | NM_198751 | XM_341239 |
| NM_019623 | NM_212525 | XM_341857 |
| NM_021262 | NM_213567 | XM_341950 |
| NM_021264 | XM_213240 | XM_342141 |
| NM_022236 | XM_213336 | XM_342312 |
| NM_022269 | XM_213992 | XM_342398 |
| NM_022398 | XM_214720 | XM_342579 |

| **GENBANK® ID** | | |
|---|---|---|
| XM_342662 | | |
| XM_343446 | | |
| XM_574233 | | |
| XM_577170 | | |

The genes in this set show abundance of expressed gene product(s) in the Aged Unimpaired population that are significantly increased, as determined by ANOVA, compared to those abundances in Young and Aged Impaired populations combined. Therefore, compounds useful in treating cognitive impairment selected using these genes should increase the abundance or enhance the function of the expressed gene products of these genes.

### Example 24

An ANOVA was conducted on the probe set signal values for all present probe sets by combining two groups of animals and comparing them to the third group. An "AU ANOVA" was performed, where Aged Impaired were combined with Young and compared to Aged Unimpaired. Any probe sets having a p-value less than 0.05 were considered significantly changed and the genes or plurality of genes selected.

### Microarray Results

Table 24 shows the genes that were identified as being associated with cognitive impairment in this experiment. The reference sequence transcript ID (GENBANK® number for the exemplar sequence) was used to identify genes considered significantly changed. When this exemplar ID was not available, a UniGene identifier was used in conjunction with the GENBANK® accession number.

**TABLE 24**

| **DG - AU ANOVA DECREASE** | | |
|---|---|---|
| | | |

| **GENBANK® ID** | **GENBANK® ID** | **GENBANK® ID** |
|---|---|---|
| BF564995 | NM_024000 | NM_199097 |
| BM389079 | NM_024146 | XM_213270 |
| AI171599 | NM_024159 | XM_213591 |
| NM_001000510 | NM_030861 | XM_213779 |
| NM_001001515 | NM_030994 | XM_213954 |
| NM_001001799 | NM_031022 | XM_214238 |
| NM_001004132 | NM_031043 | XM_214709 |
| NM_001005381 | NM_031235 | XM_214751 |
| NM_001005876 | NM_031351 | XM_215286 |
| NM_001005879 | NM_031356 | XM_215403 |
| NM_001006968 | NM_031520 | XM_215574 |
| NM_001006994 | NM_031594 | XM_216102 |
| NM_001007667 | NM_031646 | XM_216400 |
| NM_001008331 | NM_032062 | XM_217078 |
| NM_001009652 | NM_032072 | XM_217732 |
| NM_001011891 | NM_032616 | XM_218502 |
| NM_001012053 | NM_053448 | XM_220805 |
| NM_001012083 | NM_053542 | XM_222111 |
| NM_001012175 | NM_053868 | XM_222152 |
| NM_001013090 | NM_057201 | XM_225983 |
| NM_001013153 | NM_080886 | XM_226789 |
| NM_012568 | NM_080887 | XM_227025 |
| NM_012740 | NM_080895 | XM_230967 |
| NM_012798 | NM_131906 | XM_232640 |
| NM_012809 | NM_131914 | XM_234483 |
| NM_012829 | NM_134468 | XM_235156 |
| NM_017063 | NM_138840 | XM_235179 |
| NM_017066 | NM_138858 | XM_235940 |
| NM_017197 | NM_138891 | XM_238151 |
| NM_017201 | NM_139254 | XM_238213 |
| NM_017305 | NM_145089 | XM_340775 |
| NM_017321 | NM_145785 | XM_340802 |
| NM_019185 | NM_152790 | XM_341058 |
| NM_021671 | NM_171994 | XM_341104 |
| NM_021751 | NM_172157 | XM_341497 |
| NM_021770 | NM_173120 | XM_341558 |
| NM_022217 | NM_177481 | XM_341584 |
| NM_022282 | NM_177927 | XM_342477 |
| NM_022589 | NM_178095 | XM_342588 |
| NM_022690 | NM_178847 | XM_342682 |
| NM_022863 | NM_181388 | XM_342863 |
| NM_022928 | NM_182674 | XM_343273 |
| NM_023991 | NM_198764 | XM_343415 |

| **GENBANK® ID** | **GENBANK® ID** | |
|---|---|---|
| XM_347236 | XM_579546 | |
| XM_573983 | NM_022250 | |
| | | |

| **UniGene ID** | | |
|---|---|---|
| Rn.17829 | | |
| Rn.21816 | | |
| Rn.22355 | | |

The genes in this set show abundance of expressed gene product(s) in the Aged Unimpaired population that are significantly decreased, as determined by ANOVA, compared to those abundances in Young and Aged Impaired populations combined. Therefore, compounds useful in treating cognitive impairment selected using these genes should decrease the abundance or attenuate the function of the expressed gene products of these genes.

### Example 25

An ANOVA was conducted on the probe set signal values for all probe sets by combining two groups of animals and comparing them to the third group. An "AI ANOVA" was performed, where Aged Unimpaired were combined with Young and compared to Aged Impaired. All probe sets were considered in the statistical analysis based on the gcRMA method of data extraction from the perfect match sequences for each probe. Any probe sets having a p-value less than 0.05 were considered significantly changed and the genes or plurality of genes selected.

Pearson's correlations comparing probe set signal values to learning indices were then calculated for the aged animals (excluding young) across all present probe sets. Again, correlations representing a p-value of less than 0.05 were considered significantly changed.

### Microarray Results

Table 25 shows the genes that were identified as being associated with cognitive impairment in this experiment. The reference sequence transcript ID (GENBANK® number for the exemplar sequence) was used to identify genes considered significantly changed. When this exemplar ID was not available, a UniGene identifier was used in conjunction with the GENBANK® accession number.

**TABLE 25**

| **CA1 - AI ANOVA POSITIVE CORRELATION** | | |
|---|---|---|
| **GENBANK® ID** | **GENBANK® ID** | **GENBANK® ID** |
| AA800031 | NM_013198 | NM_138914 |
| AI101331 | NM_013219 | NM_145674 |
| AI639001 | NM_017068 | NM_152790 |
| BG377379 | NM_017223 | NM_173101 |
| BG662522 | NM_017267 | NM_175578 |
| NM_001004209 | NM_017356 | NM_175582 |
| NM_001005762 | NM_019318 | NM_181639 |
| NM_001005898 | NM_019362 | NM_207602 |
| NM_001007651 | NM_021775 | NM_207617 |
| NM_001008322 | NM_022198 | NM_212528 |
| NM_001008324 | NM_022226 | XM_213329 |
| NM_001009600 | NM_022502 | XM_214968 |
| NM_001011946 | NM_022617 | XM_215037 |
| NM_001012079 | NM_023978 | XM_215095 |
| NM_001012111 | NM_024163 | XM_216837 |
| NM_001012126 | NM_030863 | XM_219262 |
| NM_001012177 | NM_030992 | XM_222242 |
| NM_001012223 | NM_031014 | XM_224337 |
| NM_001013082 | NM_031034 | XM_225631 |
| NM_001017386 | NM_031147 | XM_227870 |
| NM_001017450 | NM_031521 | XM_228072 |
| NM_001024371 | NM_031552 | XM_232608 |
| NM_001024925 | NM_031812 | XM_238362 |
| NM_001025419 | NM_032066 | XM_341081 |
| NM_001029901 | NM_033443 | XM_341851 |
| NM_001033852 | NM_053021 | XM_341936 |
| NM_001034933 | NM_053407 | XM_341940 |
| NM_012543 | NM_053445 | XM_342291 |
| NM_012562 | NM_053467 | XM_342297 |
| NM_012577 | NM_053485 | XM_342317 |
| NM_012671 | NM_053538 | XM_342521 |
| NM_012686 | NM_053777 | XM_343274 |
| NM_012777 | NM_053783 | XM_343468 |
| NM_012884 | NM_053910 | XM_343773 |
| NM_013015 | NM_054001 | XM_343776 |
| NM_013060 | NM_057204 | XM_344421 |
| NM_013091 | NM_080907 | XM_573100 |
| NM_013107 | NM_133534 | |
| NM_013173 | NM_134390 | |
| NM_013191 | NM_138502 | |

The genes in this set show abundance of expressed gene product(s) in the Aged Impaired population that are significantly increased, as determined by ANOVA, compared to those abundances in Young and Aged Unimpaired populations combined. In addition, the abundance of expressed gene product(s) in the aged animals positively correlate with learning index of the animals, such that poorer learners have higher abundances of expressed gene products of the selected genes. Therefore, compounds useful in treating cognitive impairment selected using these genes should decrease the abundance or attenuate the function of the expressed gene products of these genes.

### Example 26

An ANOVA was conducted on the probe set signal values for all probe sets by combining two groups of animals and comparing them to the third group. An "AI ANOVA" was performed, where Aged Unimpaired were combined with Young and compared to Aged Impaired. All probe sets were considered in the statistical analysis based on the gcRMA method of data extraction from the perfect match sequences for each probe. Any probe sets having a p-value less than 0.05 were considered significantly changed and the genes or plurality of genes selected.

Pearson's correlations comparing probe set signal values to learning indices were then calculated for the aged animals (excluding young) across all present probe sets. Again, correlations representing a p-value of less than 0.05 were considered significantly changed.

### Microarray Results

Table 26 shows the genes that were identified as being associated with cognitive impairment in this experiment. The reference sequence transcript ID (GENBANK® number for the exemplar sequence) was used to identify genes considered significantly changed. When this exemplar ID was not available, a UniGene identifier was used in conjunction with the GENBANK® accession number.

**TABLE 26**

| **CA1 - AI ANOVA NEGATIVE CORRELATION** | | |
|---|---|---|
| **GENBANK® ID** | **GENBANK® ID** | **GENBANK® ID** |
| AI227598 | NM_031821 | XM_221333 |
| AW529408 | NM_053316 | XM_221635 |
| BF397258 | NM_053410 | XM_223693 |
| NM_001005381 | NM_053434 | XM_224389 |
| NM_001008354 | NM_053633 | XM_224929 |
| NM_001008557 | NM_053698 | XM_225039 |
| NM_001010946 | NM_053713 | XM_232936 |
| NM_001012012 | NM_053849 | XM_234385 |
| NM_001012021 | NM_053883 | XM_235179 |
| NM_001012187 | NM_080402 | XM_235552 |
| NM_001012195 | NM_133580 | XM_235878 |
| NM_001013198 | NM_138838 | XM_236953 |
| NM_001017376 | NM_138911 | XM_242556 |
| NM_001025271 | NM_139060 | XM_243390 |
| NM_001025711 | NM_139091 | XM_340809 |
| NM_001025738 | NM_172034 | XM_341663 |
| NM_001033701 | NM_199394 | XM_342107 |
| NM_012736 | NM_199463 | XM_342179 |
| NM_013083 | NM_213627 | XM_342763 |
| NM_013113 | XM_213658 | XM_342930 |
| NM_017030 | XM_214245 | XM_343046 |
| NM_017059 | XM_214369 | XM_343326 |
| NM_019128 | XM_214697 | XM_343574 |
| NM_019142 | XM_215826 | XM_343761 |
| NM_019194 | XM_216102 | XM_344130 |
| NM_021840 | XM_216679 | XM_345861 |
| NM_022229 | XM_216762 | XM_345981 |
| NM_022289 | XM_216884 | XM_573165 |
| NM_022300 | XM_217115 | XM_575396 |
| NM_022615 | XM_217210 | XM_577023 |
| NM_031069 | XM_218506 | |
| NM_031315 | XM_219500 | |
| NM_031707 | XM_219525 | |

The genes in this set show abundance of expressed gene product(s) in the Aged Impaired population that are significantly decreased, as determined by ANOVA, compared to those abundances in Young and Aged Unimpaired populations combined. In addition, the abundance of expressed gene product(s) in the aged animals negatively correlate with learning index of the animals, such that poorer learners have lower abundances of expressed gene products of the selected genes. Therefore, compounds useful in treating cognitive impairment selected using these genes should increase the abundance or enhance the function of the expressed gene products of these genes.

### Example 27

An ANOVA was conducted on the probe set signal values for all probe sets by combining two groups of animals and comparing them to the third group. An "AI ANOVA" was performed, where Aged Unimpaired were combined with Young and compared to Aged Impaired. All probe sets were considered in the statistical analysis based on the gcRMA method of data extraction from the perfect match sequences for each probe. Any probe sets having a p-value less than 0.05 were considered significantly changed and the genes or plurality of genes selected.

### Microarray Results

Table 27 shows the genes that were identified as being associated with cognitive impairment in this experiment. The reference sequence transcript ID (GENBANK® number for the exemplar sequence) was used to identify genes considered significantly changed. When this exemplar ID was not available, a UniGene identifier was used in conjunction with the GENBANK® accession number.

**TABLE 27**

| **CA1 - AI ANOVA INCREASE** | | |
|---|---|---|
| **GENBANK® ID** | **GENBANK® ID** | **GENBANK® ID** |
| AI012566 | NM_001011981 | NM_012771 |
| AI170346 | NM_001011989 | NM_012815 |
| AI179982 | NM_001011991 | NM_012819 |
| AI410079 | NM_001011993 | NM_012823 |
| AI717047 | NM_001012051 | NM_012837 |
| BF396482 | NM_001012057 | NM_012838 |
| BF417285 | NM_001012065 | NM_012925 |
| BG672075 | NM_001012222 | NM_012939 |
| BG673602 | NM_001012744 | NM_013001 |
| BM392140 | NM_001013081 | NM_013044 |
| L26525 | NM_001013086 | NM_013055 |
| NM_001001511 | NM_001013087 | NM_013069 |
| NM_001001513 | NM_001013121 | NM_013137 |
| NM_001001800 | NM_001013137 | NM_013157 |
| NM_001004072 | NM_001013174 | NM_016986 |
| NM_001004081 | NM_001013179 | NM_017008 |
| NM-001004226 | NM-001013190 | NM-017009 |
| NM_001004250 | NM_001013240 | NM_017014 |
| NM_001004273 | NM_001017383 | NM_017113 |
| NM_001005534 | NM_001024247 | NM_017125 |
| NM_001005539 | NM_001024261 | NM_017132 |
| NM_001005565 | NM_001025056 | NM_017154 |
| NM_001006989 | NM_001025282 | NM_017160 |
| NM_001007000 | NM_001025289 | NM_017169 |
| NM_001007617 | NM_001025423 | NM_017177 |
| NM_001007624 | NM_001025648 | NM_017181 |
| NM_001007625 | NM_001025721 | NM_017193 |
| NM_001007629 | NM_001025722 | NM_017196 |
| NM_001007654 | NM_001031641 | NM_017257 |
| NM_001007665 | NM_001031644 | NM_017264 |
| NM_001007677 | NM_001033968 | NM_017274 |
| NM_001007682 | NM_001034004 | NM_017288 |
| NM_001008344 | NM_001034090 | NM_017320 |
| NM_001008365 | NM_001034164 | NM_017333 |
| NM_001008374 | NM_012488 | NM-017348 |
| NM_001008829 | NM_012497 | NM_017351 |
| NM_001008835 | NM_012512 | NM_017356 |
| NM_001009474 | NM_012512 | NM_017359 |
| NM_001009623 | NM_012531 | NM_019238 |
| NM_001011893 | NM_012595 | NM_019289 |
| NM_001011903 | NM_012645 | NM_019290 |
| NM_001011917 | NM_012671 | NM_019346 |
| NM_001011920 | NM_012703 | NM_019358 |
| NM_001011925 | NM_012747 | NM_019359 |
| NM_001011959 | NM_012749 | NM_020082 |
| NM_021576 | NM_053442 | NM_139110 |
| NM_021690 | NM_053455 | NM_139189 |
| NM_021989 | NM_053516 | NM_139216 |
| NM_022266 | NM_053536 | NM_139327 |
| NM_022285 | NM_053553 | NM_145081 |
| NM_022381 | NM_053554 | NM_145775 |
| NM_022390 | NM_053560 | NM_147210 |
| NM_022392 | NM_053597 | NM_153621 |
| NM_022500 | NM_053612 | NM_153628 |
| NM_022510 | NM_053684 | NM_172033 |
| NM_022512 | NM_053773 | NM_172038 |
| NM_022525 | NM_053794 | NM_172222 |
| NM_022526 | NM_053838 | NM_172335 |
| NM_022597 | NM_053959 | NM_173095 |
| NM_022602 | NM_053979 | NM_173118 |
| NM_022668 | NM_053985 | NM_173123 |
| NM_022697 | NM_054006 | NM_173147 |
| NM_022703 | NM_057114 | NM_175756 |
| NM_022856 | NM_057137 | NM_175838 |
| NM_024155 | NM_057197 | NM_176077 |
| NM_024160 | NM_080887 | NM_178095 |
| NM_024366 | NM_080890 | NM_181363 |
| NM_024396 | NM_130403 | NM_181381 |
| NM_030826 | NM_130416 | NM_181628 |
| NM_030859 | NM_130419 | NM_183330 |
| NM_030872 | NM_130428 | NM_184046 |
| NM_030987 | NM_130739 | NM_198738 |
| NM_031057 | NM_133297 | NM_199087 |
| NM_031090 | NM_133298 | NM_199092 |
| NM_031140 | NM_133392 | NM_199118 |
| NM_031357 | NM_133405 | NM_199399 |
| NM_031509 | NM_133418 | NM_199404 |
| NM_031614 | NM_133605 | NM_207591 |
| NM_031624 | NM_133618 | NM_207599 |
| NM_031648 | NM_133621 | NM_207609 |
| NM_031660 | NM_134334 | NM_212495 |
| NM_031668 | NM_134349 | NM_212525 |
| NM_031672 | NM_134389 | NM_212538 |
| NM_031685 | NM_134410 | XM_213335 |
| NM_031698 | NM_134432 | XM_213408 |
| NM_031756 | NM_138508 | XM_213540 |
| NM_031789 | NM_138521 | XM_213650 |
| NM_031818 | NM_138826 | XM_213777 |
| NM_031827 | NM_138828 | XM_213824 |
| NM_031841 | NM_138900 | XM_213879 |
| NM_031973 | NM_138905 | XM_214250 |
| NM_040669 | NM_138917 | XM_214316 |
| NM_053323 | NM_139103 | XM_214478 |
| XM_214480 | XM_224535 | XM_341882 |
| XM_214518 | XM_224538 | XM_341957 |
| XM_214583 | XM_225014 | XM_342002 |
| XM_214769 | XM_225147 | XM_342300 |
| XM_214838 | XM_225160 | XM_342528 |
| XM_215524 | XM_225628 | XM_342600 |
| XM_215607 | XM_225644 | XM_342686 |
| XM_215733 | XM_225711 | XM_343034 |
| XM_215897 | XM_226165 | XM_343259 |
| XM_216565 | XM_228701 | XM_343268 |
| XM_216688 | XM_230036 | XM_343306 |
| XM_216704 | XM_230291 | XM_343396 |
| XM_216740 | XM_231287 | XM_343427 |
| XM_216962 | XM_231620 | XM_344524 |
| XM_217239 | XM_231749 | XM_345535 |
| XM_217335 | XM_235497 | XM_345584 |
| XM_217372 | XM_235558 | XM_345825 |
| XM_218617 | XM_235710 | XM_345849 |
| XM_218816 | XM_237381 | XM_345870 |
| XM_218977 | XM_237790 | XM_347163 |
| XM_219948 | XM_238019 | XM_575585 |
| XM_220095 | XM_238380 | XM_576343 |
| XM_220357 | XM_238770 | XM_576401 |
| XM_220541 | XM_243637 | XM_576459 |
| XM_221100 | XM_341389 | |
| XM_223012 | XM_341509 | |
| XM_223785 | XM_341796 | |

The genes in this set show abundance of expressed gene product(s) in the Aged Impaired population that are significantly increased, as determined by ANOVA, compared to those abundances in Young and Aged Unimpaired populations combined. Therefore, compounds useful in treating cognitive impairment selected using these genes should decrease the abundance or attenuate the function of the expressed gene products of these genes.

### Example 28

An ANOVA was conducted on the probe set signal values for all probe sets by combining two groups of animals and comparing them to the third group. An "AI ANOVA" was performed, where Aged Unimpaired were combined with Young and compared to Aged Impaired. All probe sets were considered in the statistical analysis based on the gcRMA method of data extraction from the perfect match sequences for each probe. Any probe sets having a p-value less than 0.05 were considered significantly changed and the genes or plurality of genes selected.

### Microarray Results

Table 28 shows the genes that were identified as being associated with cognitive impairment in this experiment. The reference sequence transcript ID (GENBANK® number for the exemplar sequence) was used to identify genes considered significantly changed. When this exemplar ID was not available, a UniGene identifier was used in conjunction with the GENBANK® accession number.

**TABLE 28**

| **CA1** - **AI ANOVA DECREASE** | | |
|---|---|---|
| **GENBANK® ID** | **GENBANK® ID** | **GENBANK® ID** |
| AF073379 | NM_001025414 | NM_021266 |
| AI029288 | NM_001025650 | NM_021653 |
| AI229643 | NM_001025667 | NM_021763 |
| AI599699 | NM_001025705 | NM_021843 |
| AW528891 | NM_001033655 | NM_022249 |
| BE118251 | NM_001033699 | NM_022254 |
| BF404393 | NM_001033974 | NM_022688 |
| BF410962 | NM_001033984 | NM_022847 |
| NM_001002289 | NM_001034014 | NM_022850 |
| NM_001002851 | NM_001034068 | NM_022867 |
| NM_001003957 | NM_001034131 | NM_022962 |
| NM_001004198 | NM_012568 | NM_023971 |
| NM_001004210 | NM_012573 | NM_023989 |
| NM_001004277 | NM_012576 | NM_024362 |
| NM_001004279 | NM_012596 | NM_024401 |
| NM_001004442 | NM_012651 | NM_031056 |
| NM_001005536 | NM_012653 | NM_031123 |
| NM_001005872 | NM_012688 | NM_031130 |
| NM_001007728 | NM_012701 | NM_031318 |
| NM_001008320 | NM_012727 | NM_031720 |
| NM_001008353 | NM_012780 | NM_031730 |
| NM_001009268 | NM_012835 | NM_031753 |
| NM_001009605 | NM_012934 | NM_031777 |
| NM_001011901 | NM_012991 | NM_031828 |
| NM_001011966 | NM_013036 | NM_033485 |
| NM_001012043 | NM_013100 | NM_053349 |
| NM_001012143 | NM_013102 | NM_053441 |
| NM_001012144 | NM_013111 | NM_053487 |
| NM_001012211 | NM_013126 | NM_053503 |
| NM_001012475 | NM_013161 | NM_053530 |
| NM_001013066 | NM_013179 | NM_053616 |
| NM_001013096 | NM_013189 | NM_053693 |
| NM_001013099 | NM_013199 | NM_053703 |
| NM_001013128 | NM_017041 | NM_053718 |
| NM_001013133 | NM_017073 | NM_053740 |
| NM_001013178 | NM_017238 | NM_053775 |
| NM_001013244 | NM_017303 | NM_053781 |
| NM_001014792 | NM_017352 | NM_053786 |
| NM_001015011 | NM_019163 | NM_053876 |
| NM_001024318 | NM_019218 | NM_053888 |
| NM_001024766 | NM_019282 | NM_053895 |
| NM_001024790 | NM_019288 | NM_054008 |
| NM_001024794 | NM_019347 | NM_057212 |
| NM_001025136 | NM_019348 | NM_130748 |
| NM_001025402 | NM_020306 | NM_133318 |
| NM_133411 | XM_216318 | XM_232220 |
| NM_133548 | XM_217560 | XM_232599 |
| NM_133591 | XM_217570 | XM_233141 |
| NM_133596 | XM_217601 | XM_233499 |
| NM_134383 | XM_218138 | XM_233792 |
| NM_134388 | XM_218187 | XM_234219 |
| NM_138837 | XM_218200 | XM_234263 |
| NM_138907 | XM_218347 | XM_234299 |
| NM_138922 | XM_218845 | XM_234345 |
| NM_144758 | XM_219372 | XM_234345 |
| NM_147135 | XM_219905 | XM_234470 |
| NM_147142 | XM_221050 | XM_234481 |
| NM_153317 | XM_221212 | XM_234514 |
| NM_173145 | XM_221231 | XM_234540 |
| NM_177419 | XM_221380 | XM_234546 |
| NM_181370 | XM_221426 | XM_235768 |
| NM_181380 | XM_221641 | XM_239074 |
| NM_182668 | XM_221724 | XM_239260 |
| NM_182844 | XM_221888 | XM_341111 |
| NM_198749 | XM_221962 | XM_341391 |
| NM_198760 | XM_222103 | XM_341590 |
| NM_198761 | XM_222184 | XM_341642 |
| NM_198770 | XM_222476 | XM_341653 |
| NM_199373 | XM_222770 | XM_341709 |
| U48828 | XM_223643 | XM_342151 |
| XM_213440 | XM_223729 | XM_342217 |
| XM_213842 | XM_225138 | XM_342481 |
| XM_213954 | XM_225138 | XM_342552 |
| XM_214093 | XM_225168 | XM_342692 |
| XM_214312 | XM_225169 | XM_342734 |
| XM_214621 | XM_225220 | XM_342775 |
| XM_214673 | XM_226349 | XM_342804 |
| XM_214696 | XM_226436 | XM_342808 |
| XM_214823 | XM_226843 | XM_343045 |
| XM_214883 | XM_226874 | XM_343148 |
| XM_215376 | XM_227104 | XM_343548 |
| XM_215451 | XM_227444 | XM_344594 |
| XM_215883 | XM_227811 | XM_345418 |
| XM_215892 | XM_230449 | XM_345789 |
| XM_215984 | XM_230877 | XM_574979 |
| XM_216047 | XM_231798 | XM_575397 |
| XM_216161 | XM_231803 | |
| XM_216225 | XM_232197 | |

The genes in this set show abundance of expressed gene product(s) in the Aged Impaired population that are significantly decreased, as determined by ANOVA, compared to those abundances in Young and Aged Unimpaired populations combined. Therefore, compounds useful in treating cognitive impairment selected using these genes should increase the abundance or enhance the function of the expressed gene products of these genes.

### Example 29

An ANOVA was conducted on the probe set signal values for all probe sets by combining two groups of animals and comparing them to the third group. An "AU ANOVA" was performed, where Aged Impaired were combined with Young and compared to Aged Unimpaired. All probe sets were considered in the statistical analysis based on the gcRMA method of data extraction from the perfect match sequences for each probe. Any probe sets having a p-value less than 0.05 were considered significantly changed and the genes or plurality of genes selected.

Pearson's correlations comparing probe set signal values to learning indices were then calculated for the aged animals (excluding young) across all present probe sets. Again, correlations representing a p-value of less than 0.05 were considered significantly changed.

### Microarray Results

Table 29 shows the genes that were identified as being associated with cognitive impairment in this experiment. The reference sequence transcript ID (GENBANK® number for the exemplar sequence) was used to identify genes considered significantly changed. When this exemplar ID was not available, a UniGene identifier was used in conjunction with the GENBANK® accession number.

**TABLE 29**

| **AU ANOVA POSITIVE CORRELATION** | | |
|---|---|---|
| **GENBANK® ID** | **GENBANK® ID** | **GENBANK® ID** |
| AA955871 | NM_019351 | XM_213564 |
| BF288303 | NM_019354 | XM_214400 |
| BF410275 | NM_031097 | XM_216018 |
| NM_001002016 | NM_033359 | XM_216784 |
| NM_001025403 | NM_053383 | XM_218336 |
| NM_001025744 | NM_053644 | XM_220775 |
| NM_001035233 | NM_053669 | XM_223057 |
| NM_012623 | NM_053721 | XM_223227 |
| NM_012660 | NM_053811 | XM_224618 |
| NM_012869 | NM_053824 | XM_230288 |
| NM_013159 | NM_053930 | XM_230616 |
| NM_013176 | NM_053972 | XM_230765 |
| NM_013197 | NM_057208 | XM_234508 |
| NM_017007 | NM_080577 | XM_239329 |
| NM_017103 | NM_080786 | XM_341972 |
| NM_017294 | NM_130406 | XM_342003 |
| NM_017306 | NM_131906 | XM_342723 |
| NM_017347 | NM_138848 | XM_343303 |
| NM_017354 | NM_153469 | XM_343764 |
| NM_019183 | NM_172041 | XM_573915 |
| NM_019239 | NM_172224 | XM_575671 |
| NM_019309 | NM_173325 | |

The genes in this set show abundance of expressed gene product(s) in the Aged Unimpaired population that are significantly decreased, as determined by ANOVA, compared to those abundances in Young and Aged Impaired populations combined. In addition, the abundance of expressed gene product(s) in the aged animals positively correlate with learning index of the animals, such that poorer learners have higher abundances of expressed gene products of the selected genes. Therefore, compounds useful in treating cognitive impairment selected using these genes should decrease the abundance or attenuate the function of the expressed gene products of these genes.

### Example 30

An ANOVA was conducted on the probe set signal values for all probe sets by combining two groups of animals and comparing them to the third group. An "AU ANOVA" was performed, where Aged Impaired were combined with Young and compared to Aged Unimpaired. All probe sets were considered in the statistical analysis based on the gcRMA method of data extraction from the perfect match sequences for each probe. Any probe sets having a p-value less than 0.05 were considered significantly changed and the genes or plurality of genes selected.

Pearson's correlations comparing probe set signal values to learning indices were then calculated for the aged animals (excluding young) across all present probe sets. Again, correlations representing a p-value of less than 0.05 were considered significantly changed.

### Microarray Results

Table 30 shows the genes that were identified as being associated with cognitive impairment in this experiment. The reference sequence transcript ID (GENBANK® number for the exemplar sequence) was used to identify genes considered significantly changed. When this exemplar ID was not available, a UniGene identifier was used in conjunction with the GENBANK® accession number.

**TABLE 30**

| **CA1 - AU ANOVA NEGATIVE CORRELATION** | | |
|---|---|---|
| **GENBANK® ID** | **GENBANK® ID** | **GENBANK® ID** |
| BG378690 | NM_031122 | XM_215082 |
| NM_001002830 | NM_031133 | XM_215616 |
| NM_001004075 | NM_031139 | XM_216295 |
| NM_001008382 | NM_031642 | XM_216400 |
| NM_001008524 | NM_031645 | XM_216712 |
| NM_001011936 | NM_032058 | XM_216841 |
| NM_001013204 | NM_053389 | XM_223674 |
| NM_001013212 | NM_053499 | XM_225726 |
| NM_001033674 | NM_053500 | XM_228197 |
| NM_001034129 | NM_053578 | XM_233467 |
| NM_017089 | NM_057207 | XM_236745 |
| NM_017271 | NM_134395 | XM_237l79 |
| NM_019161 | NM_134408 | XM_239510 |
| NM_019364 | NM_134417 | XM_340856 |
| NM_021262 | NM_138536 | XM_342131 |
| NM_022921 | NM_138856 | XM_342268 |
| NM_024002 | NM_145677 | XM_343318 |
| NM_024131 | NM_152861 | XM_343413 |
| NM_031041 | NM_172243 | XM_343513 |
| NM_031082 | XM_213725 | |

The genes in this set show abundance of expressed gene product(s) in the Aged Unimpaired population that are significantly increased, as determined by ANOVA, compared to those abundances in Young and Aged Impaired populations combined. In addition, the abundance of expressed gene product(s) in the aged animals negatively correlate with learning index of the animals, such that poorer learners have lower abundances of expressed gene products of the selected genes. Therefore, compounds useful in treating cognitive impairment selected using these genes should increase the abundance or enhance the function of the expressed gene products of these genes.

### Example 31

An ANOVA was conducted on the probe set signal values for all probe sets by combining two groups of animals and comparing them to the third group. An "AU ANOVA" was performed, where Aged Impaired were combined with Young and compared to Aged Unimpaired. All probe sets were considered in the statistical analysis based on the gcRMA method of data extraction from the perfect match sequences for each probe. Any probe sets having a p-value less than 0.05 were considered significantly changed and the genes or plurality of genes selected.

### Microarray Results

Table 31 shows the genes that were identified as being associated with cognitive impairment in this experiment. The reference sequence transcript ID (GENBANK® number for the exemplar sequence) was used to identify genes considered significantly changed. When this exemplar ID was not available, a UniGene identifier was used in conjunction with the GENBANK® accession number.

**TABLE 31**

| **CA1 - AU ANOVA INCREASE** | | |
|---|---|---|
| **GENBANK® ID** | **GENBANK® ID** | **GENBANK® ID** |
| AA859127 | NM_001025725 | NM_057108 |
| AA998007 | NM_001030024 | NM_057143 |
| AI137495 | NM_001033866 | NM_057196 |
| BF287008 | NM_001034110 | NM_080906 |
| BI294974 | NM_012592 | NM_080910 |
| BM384139 | NM_012720 | NM_130740 |
| BM388245 | NM_012805 | NM_131911 |
| BM390128 | NM_012806 | NM_131914 |
| NM_001004090 | NM_012848 | NM_133303 |
| NM_001004238 | NM_012895 | NM_134326 |
| NM_001004255 | NM_012913 | NM_138530 |
| NM_001004443 | NM_012924 | NM_138835 |
| NM_001005528 | NM_012935 | NM_138873 |
| NM_001006969 | NM_012992 | NM_145084 |
| NM_001007557 | NM_013057 | NM_145670 |
| NM_001007609 | NM_013088 | NM_145673 |
| NM_001007654 | NM_013150 | NM_145788 |
| NM_001008339 | NM_013151 | NM_147205 |
| NM_001008364 | NM_016994 | NM_153475 |
| NM_001008515 | NM_017212 | NM_172325 |
| NM_001009604 | NM_017290 | NM_175764 |
| NM_001009973 | NM_017318 | NM_182821 |
| NM_001009974 | NM_019251 | NM_182955 |
| NM_001010961 | NM_019361 | NM_201415 |
| NM_001011922 | NM_021584 | NM_212496 |
| NM_001011950 | NM_021846 | NM_213628 |
| NM_001011953 | NM_022205 | XM_213370 |
| NM_001012003 | NM_022252 | XM_213560 |
| NM_001012030 | NM_022499 | XM_213898 |
| NM_001012039 | NM_022529 | XM_213906 |
| NM_001012096 | NM_022545 | XM_214153 |
| NM_001012105 | NM_024125 | XM_214163 |
| NM_001012131 | NM_024153 | XM_214927 |
| NM_001012181 | NM_031058 | XM_215423 |
| NM_001012459 | NM_031112 | XM_215564 |
| NM_001013151 | NM_031148 | XM_215947 |
| NM_001013152 | NM_031517 | XM_215949 |
| NM_001013154 | NM_031623 | XM_216202 |
| NM_001013165 | NM_031740 | XM_216403 |
| NM_001013165 | NM_031763 | XM_216407 |
| NM_001013184 | NM_053482 | XM_216643 |
| NM_001024964 | NM_053662 | XM_216757 |
| NM_001025035 | NM_053707 | XM_216893 |
| NM_001025137 | NM_053926 | XM_217086 |
| NM_001025709 | NM_057107 | XM_218162 |
| XM_218502 | XM_226329 | XM_341578 |
| XM_218648 | XM_230845 | XM_342154 |
| XM_219879 | XM_233535 | XM_342580 |
| XM_219925 | XM_233883 | XM_342662 |
| XM_220398 | XM_234238 | XM_342810 |
| XM_221120 | XM_234377 | XM_342819 |
| XM_221307 | XM_234394 | XM_343050 |
| XM_223327 | XM_235296 | XM_344976 |
| XM_223580 | XM_236948 | XM_574504 |
| XM_223597 | XM_237093 | XM_575968 |
| XM_224863 | XM_238731 | XM_578542 |
| XM_225439 | XM_340798 | |
| XM_226076 | XM_341508 | |

The genes in this set show abundance of expressed gene product(s) in the Aged Unimpaired population that are significantly increased, as determined by ANOVA, compared to those abundances in Young and Aged Impaired populations combined. Therefore, compounds useful in treating cognitive impairment selected using these genes should increase the abundance or enhance the function of the expressed gene products of these genes.

### Example 32

An ANOVA was conducted on the probe set signal values for all probe sets by combining two groups of animals and comparing them to the third group. An "AU ANOVA" was performed, where Aged Impaired were combined with Young and compared to Aged Unimpaired. All probe sets were considered in the statistical analysis based on the gcRMA method of data extraction from the perfect match sequences for each probe. Any probe sets having a p-value less than 0.05 were considered significantly changed and the genes or plurality of genes selected.

### Microarray Results

Table 32 shows the genes that were identified as being associated with cognitive impairment in this experiment. The reference sequence transcript ID (GENBANK® number for the exemplar sequence) was used to identify genes considered significantly changed. When this exemplar ID was not available, a UniGene identifier was used in conjunction with the GENBANK® accession number.

**TABLE 32**

| **CA1** - **AU ANOVA DECREASE** | | |
|---|---|---|
| **GENBANK® ID** | **GENBANK® ID** | **GENBANK® ID** |
| AA945615 | NM_001034163 | NM_031073 |
| AI764408 | NM_001034849 | NM_031132 |
| BE103926 | NM_012504 | NM_031332 |
| BF403383 | NM_012555 | NM_031353 |
| BF567766 | NM_012572 | NM_031356 |
| BI287800 | NM_012598 | NM_031511 |
| NM_001002815 | NM_012654 | NM_031528 |
| NM_001004132 | NM_012774 | NM_031569 |
| NM_001007607 | NM_012853 | NM_031571 |
| NM_001007616 | NM_012863 | NM_031575 |
| NM_001007629 | NM_012877 | NM_031617 |
| NM_001007725 | NM_012950 | NM_031738 |
| NM_001007758 | NM_012983 | NM_031771 |
| NM_001008369 | NM_013002 | NM_031967 |
| NM_001008823 | NM_013127 | NM_032062 |
| NM_001009258 | NM_013160 | NM_032613 |
| NM_001009405 | NM_017049 | NM_033097 |
| NM_001011894 | NM_017134 | NM_033376 |
| NM_001011995 | NM_017139 | NM_053306 |
| NM_001012101 | NM_017239 | NM_053310 |
| NM_001012191 | NM_017256 | NM_053360 |
| NM_001012737 | NM_017316 | NM_053428 |
| NM_001013044 | NM_019140 | NM_053559 |
| NM_001013108 | NM_019156 | NM_053594 |
| NM_001013161 | NM_020074 | NM_053599 |
| NM_001013224 | NM_021659 | NM_053601 |
| NM_001013432 | NM_021859 | NM_053643 |
| NM_001013910 | NM_022202 | NM_053655 |
| NM_001015004 | NM_022382 | NM_053686 |
| NM_001017381 | NM_022396 | NM_053764 |
| NM_001024756 | NM_022600 | NM_053788 |
| NM_001024781 | NM_022609 | NM_053801 |
| NM_001024795 | NM_022676 | NM_053842 |
| NM_001024823 | NM_022690 | NM_053846 |
| NM_001025032 | NM_022853 | NM_053859 |
| NM_001025032 | NM_022946 | NM_053870 |
| NM_001025416 | NM_023972 | NM_053960 |
| NM_001025693 | NM_024147 | NM_053994 |
| NM_001031656 | NM_024355 | NM_057213 |
| NM_001031845 | NM_024356 | NM_080394 |
| NM_001033757 | NM_024383 | NM_080580 |
| NM_001033882 | NM_030862 | NM_080885 |
| NM_001033951 | NM_031008 | NM_130829 |
| NM_001034020 | NM_031037 | NM_131913 |
| NM_001034133 | NM_031046 | NM_133397 |
| NM_133410 | XM_215469 | XM_234205 |
| NM_133511 | XM_215742 | XM_235426 |
| NM_133566 | XM_215887 | XM_235500 |
| NM_134331 | XM_215939 | XM_235640 |
| NM_134377 | XM_216189 | XM_239504 |
| NM_134461 | XM_216745 | XM_240330 |
| NM_139104 | XM_217021 | XM_241981 |
| NM_139263 | XM_217124 | XM_242982 |
| NM_144740 | XM_217188 | XM_340775 |
| NM_171992 | XM_217279 | XM_340911 |
| NM_172039 | XM_217388 | XM_340967 |
| NM_173115 | XM_218412 | XM_340999 |
| NM_173146 | XM_218851 | XM_341790 |
| NM_173312 | XM_219529 | XM_341998 |
| NM_173837 | XM_219801 | XM_342032 |
| NM_181092 | XM_220224 | XM_342134 |
| NM_181638 | XM_220602 | XM_342389 |
| NM_182673 | XM_220884 | XM_342409 |
| NM_182824 | XM_220982 | XM_342632 |
| NM_182842 | XM_221672 | XM_342905 |
| NM_198758 | XM_221977 | XM_343058 |
| NM_199111 | XM_222223 | XM_343247 |
| NM_203338 | XM_222896 | XM_343394 |
| NM_207592 | XM_224261 | XM_343459 |
| X55812 | XM_224952 | XM_343483 |
| XM_213318 | XM_225259 | XM_343582 |
| XM_213993 | XM_225625 | XM_344434 |
| XM_214630 | XM_226213 | XM_346464 |
| XM_214669 | XM_230296 | XM_576183 |
| XM_214720 | XM_232488 | |
| XM_214775 | XM_232640 | |
| XM_214916 | XM_233231 | |

The genes in this set show abundance of expressed gene product(s) in the Aged Unimpaired population that are significantly decreased, as determined by ANOVA, compared to those abundances in Young and Aged Impaired populations combined. Therefore, compounds useful in treating cognitive impairment selected using these genes should decrease the abundance or attenuate the function of the expressed gene products of these genes.

### Example 33

An ANOVA was conducted on the probe set signal values for all probe sets by combining two groups of animals and comparing them to the third group. An "AI ANOVA" was performed, where Aged Unimpaired were combined with Young and compared to Aged Impaired. All probe sets were considered in the statistical analysis based on the gcRMA method of data extraction from the perfect match sequences for each probe. Any probe sets having a p-value less than 0.05 were considered significantly changed and the genes or plurality of genes selected.

Pearson's correlations comparing probe set signal values to learning indices were then calculated for the aged animals (excluding young) across all present probe sets. Again, correlations representing a p-value of less than 0.05 were considered significantly changed.

### Microarray Results

Table 33 shows the genes that were identified as being associated with cognitive impairment in this experiment. The reference sequence transcript ID (GENBANK® number for the exemplar sequence) was used to identify genes considered significantly changed. When this exemplar ID was not available, a UniGene identifier was used in conjunction with the GENBANK® accession number.

**TABLE 33**

| **CA3 - AI ANOVA POSITIVE CORRELATION** | | |
|---|---|---|
| **GENBANK® ID** | **GENBANK® ID** | **GENBANK® ID** |
| AA800192 | NM_001015009 | NN_017031 |
| AA892549 | NM_001017386 | NM_017037 |
| AA956317 | NM_001017503 | NM_017041 |
| AI103026 | NM_001024238 | NM_017068 |
| AI556426 | NM_001024771 | NM_017073 |
| BF398122 | NM_001025125 | NM-017092 |
| BI282114 | NM_001025289 | NM_017148 |
| BM391371 | NM_001031647 | NM_017166 |
| NM_001001511 | NM_001033696 | NM_017197 |
| NM_001002016 | NM_001033701 | NM_017206 |
| NM_001004080 | NM_001033852 | NM_017223 |
| NM_001004255 | NM_001034090 | NM_017251 |
| NM_001005765 | NM_012507 | NM_017261 |
| NM_001007145 | NM_012514 | NM_017307 |
| NM_001007677 | NM_012543 | NM_017333 |
| NM_001007797 | NM_012569 | NM_017354 |
| NM_001008316 | NM_012577 | NM_017357 |
| NM_001008331 | NM_012618 | NM_019161 |
| NM_001008374 | NM_012655 | NM_019168 |
| NM_001008725 | NM_012671 | NM_019175 |
| NM_001008767 | NM_012720 | NM_019204 |
| NM_001008880 | NM_012720 | NM_019257 |
| NH_001009474 | NM_012747 | NM_019321 |
| NM_001010961 | NM_012749 | NM_019359 |
| NM_001011893 | NM_012777 | NM_021595 |
| NM_001011929 | NM_012778 | NM_021682 |
| NM_001011946 | NM_012804 | NM_021762 |
| NM_001011954 | NM_012816 | NM_021868 |
| NM_001011991 | NM_012825 | NM_022207 |
| NM_001012025 | NM_012894 | NM_022238 |
| NM_001012040 | NM_012920 | NM_022276 |
| NM_001012075 | NM_012925 | NM_022285 |
| NM_001012097 | NM_012963 | NM_022390 |
| NM_001012111 | NM_012983 | NM_022799 |
| NM_001012137 | NM_013005 | NM_022861 |
| NM_001012138 | NM_013044 | NM_023978 |
| NM_001012150 | NM_013045 | NM_024398 |
| NM_001012203 | NM_013058 | NM_030832 |
| NM_001012215 | NM_013060 | NM_030868 |
| NM_001012235 | NM_013065 | NM_030872 |
| NM_001013110 | NM_013096 | NM_031031 |
| NM_001013121 | NM_013137 | NM_031033 |
| NM_001013130 | NM_013176 | NM_031034 |
| NM_001013200 | NM_013198 | NM_031035 |
| NM_001013246 | NM_017006 | NM_031049 |
| NM_031087 | NM_138914 | XM_217372 |
| NM_031091 | NM_139038 | XM_217409 |
| NM_031092 | NM_139103 | XM_218162 |
| NM_031093 | NM_139330 | XM_218816 |
| NM_031140 | NM_145092 | XM_219998 |
| NM_031144 | NM_145670 | XM_220207 |
| NM_031521 | NM_147210 | XM_221100 |
| NM_031552 | NM_152790 | XM_221497 |
| NM_031665 | NM_152847 | XM_221562 |
| NM_031841 | NM_153308 | XM_222024 |
| NM_032990 | NM_153311 | XM_222617 |
| NM_052807 | NM_172033 | XM_222661 |
| NM_053021 | NM_172068 | XM_222745 |
| NM_053323 | NM_173152 | XM_222780 |
| NM_053328 | NM_175578 | XM_223227 |
| NM_053369 | NM_175582 | XM_223583 |
| NM_053424 | NM_175838 | XM_224256 |
| NM_053467 | NM_176857 | XM_224337 |
| NM_053475 | NM_178095 | XM_224732 |
| NM_053502 | NM_181388 | XM_224852 |
| NM_053576 | NM_199119 | XM_225008 |
| NM_053598 | XM_213329 | XM_225014 |
| NM_053612 | XM_213408 | XM_225160 |
| NM_053681 | XM_213421 | XM_225404 |
| NM_053735 | XM_213746 | XM_225512 |
| NM_053757 | XM_213779 | XM_226165 |
| NM_053766 | XM_213849 | XM_227203 |
| NM_053799 | XM_213920 | XM_227409 |
| NM_053824 | XM_213966 | XM_227581 |
| NM_053926 | XM_214029 | XM_230036 |
| NM_053936 | XM_214371 | XM_230592 |
| NM_053985 | XM_214518 | XM_231287 |
| NM_053994 | XM_214583 | XM_231620 |
| NM_054001 | XM_214968 | XM_232354 |
| NM_057114 | XM_215080 | XM_232671 |
| NM_057148 | XM_215095 | XM_232732 |
| NM_057197 | XM_215184 | XM_233761 |
| NM_057200 | XM_215367 | XM_234328 |
| NM_057208 | XM_215469 | XM_234345 |
| NM_080691 | XM_215733 | XM_234377 |
| NM_130403 | XM_215872 | XM_234468 |
| NM_133317 | XM_216386 | XM_235064 |
| NM_133398 | XM_216688 | XM_235657 |
| NM_133548 | XM_216704 | XM_236362 |
| NM_133560 | XM_216759 | XM_237042 |
| NM_133602 | XM_216965 | XM_237241 |
| NM_133605 | XM_217192 | XM_237381 |
| NM_138905 | XM_217367 | XM_238019 |
| XM_238057 | XM_341911 | XM_343630 |
| XM_238103 | XM_342100 | XM_343764 |
| XM_238213 | XM_342102 | XM_343773 |
| XM_238362 | XM_342291 | XM_343856 |
| XM_238649 | XM_342297 | XM_343971 |
| XM_240978 | XM_342317 | XM_344046 |
| XM_241375 | XM_342521 | XM_344785 |
| XM_241671 | XM_342535 | XM_345446 |
| XM_340810 | XM_342626 | XM_345584 |
| XM_340879 | XM_342662 | XM_345625 |
| XM_340997 | XM_342928 | XM_347166 |
| XM_341052 | XM_343055 | XM_347223 |
| XM_341172 | XM_343092 | XM_573100 |
| XM_341215 | XM_343157 | XM_575396 |
| XM_341288 | XM_343268 | XM_576311 |
| XM_341538 | XM_343274 | XM_576343 |
| XM_341605 | XM_343281 | XM_576401 |
| XM_341633 | XM_343427 | |
| XM_341882 | XM_343570 | |

The genes in this set show abundance of expressed gene product(s) in the Aged Impaired population that are significantly increased, as determined by ANOVA, compared to those abundances in Young and Aged Unimpaired populations combined. In addition, the abundance of expressed gene product(s) in the aged animals positively correlate with learning index of the animals, such that poorer learners have higher abundances of expressed gene products of the selected genes. Therefore, compounds useful in treating cognitive impairment selected using these genes should decrease the abundance or attenuate the function of the expressed gene products of these genes.

### Example 34

An ANOVA was conducted on the probe set signal values for all probe sets by combining two groups of animals and comparing them to the third group. An "AI ANOVA" was performed, where Aged Unimpaired were combined with Young and compared to Aged Impaired. All probe sets were considered in the statistical analysis based on the gcRMA method of data extraction from the perfect match sequences for each probe. Any probe sets having a p-value less than 0.05 were considered significantly changed and the genes or plurality of genes selected.

Pearson's correlations comparing probe set signal values to learning indices were then calculated for the aged animals (excluding young) across all present probe sets. Again, correlations representing a p-value of less than 0.05 were considered significantly changed.

### Microarray Results

Table 34 shows the genes that were identified as being associated with cognitive impairment in this experiment. The reference sequence transcript ID (GENBANK® number for the exemplar sequence) was used to identify genes considered significantly changed. When this exemplar ID was not available, a UniGene identifier was used in conjunction with the GENBANK® accession number.

**TABLE 34**

| **CA3 - AI ANOVA NEGATIVE CORRELATION** | | |
|---|---|---|
| **GENBANK® ID** | **GENBANK® ID** | **GENBANK® ID** |
| BF282715 | NM_013090 | NM_053575 |
| BM391206 | NM_013111 | NM_053613 |
| NM_001001718 | NM_013113 | NM_053621 |
| NM_001002818 | NM_013130 | NM_053622 |
| NM_001003653 | NM_013174 | NM_053792 |
| NM_001004075 | NM_017126 | NM_053810 |
| NM_001004217 | NM_017135 | NM_053849 |
| NM_001004222 | NM_017352 | NM_053883 |
| NM_001005543 | NM_021688 | NM_053950 |
| NM_001006602 | NM_021699 | NM_053990 |
| NM_001006984 | NM_022186 | NM_053997 |
| NM_001006994 | NM_022188 | NM_054008 |
| NM_001007656 | NM_022264 | NM_133394 |
| NM_001007707 | NM_022272 | NM_133425 |
| NM_001008304 | NM_022511 | NM_133562 |
| NM_001008511 | NM_022599 | NM_133596 |
| NM_001008553 | NM_022688 | NM_133596 |
| NM_001008557 | NM_022860 | NM_138833 |
| NM_001009422 | NM_022938 | NM_138866 |
| NM_001009666 | NM_022941 | NM_138893 |
| NM_001009677 | NM_022948 | NM_139254 |
| NM_001010946 | NM_022961 | NM_139337 |
| NM_001011923 | NM_023104 | NM_145098 |
| NM_001011934 | NM_024366 | NM_147141 |
| NM_001011957 | NM_024381 | NM_152861 |
| NM_001011996 | NM_024390 | NM_172062 |
| NM_001012012 | NM_030830 | NM_173094 |
| NM_001012098 | NM_030841 | NM_173115 |
| NM_001012101 | NM_031006 | NM_178091 |
| NM_001012125 | NM_031030 | NM_182844 |
| NM_001012192 | NM_031062 | NM_184051 |
| NM_001013133 | NM_031070 | NM_198726 |
| NM_001013201 | NM_031344 | NM_198756 |
| NM_001013906 | NM_031520 | NM_198783 |
| NM_001014785 | NM_031569 | NM_199098 |
| NM_001024250 | NM_031583 | NM_199500 |
| NM_001024756 | NM_031742 | NM_206881 |
| NM_001029898 | NM_031749 | NM_212496 |
| NM_001031649 | NM_031851 | NM_212544 |
| NM_001033069 | NM_032079 | XM_213426 |
| NM_012500 | NM_033349 | XM_213626 |
| NM_012600 | NM_053334 | XM_213672 |
| NM_012739 | NM_053388 | XM_214331 |
| NM_013083 | NM_053528 | XM_214400 |
| XM_214475 | XM_222717 | XM_341314 |
| XM_214485 | XM_223327 | XM_341326 |
| XM_214491 | XM_223693 | XM_341520 |
| XM_214550 | XM_223921 | XM_341663 |
| XM_214895 | XM_225039 | XM_341955 |
| XM_215069 | XM_225072 | XM_341969 |
| XM_215082 | XM_226843 | XM_342107 |
| XM_215134 | XM_227414 | XM_342295 |
| XM_215826 | XM_227428 | XM_342692 |
| XM_215883 | XM_232735 | XM_343140 |
| XM_215931 | XM_232745 | XM_343413 |
| XM_216112 | XM_232901 | XM_343429 |
| XM_217385 | XM_233544 | XM_343614 |
| XM_217908 | XM_234219 | XM_344009 |
| XM_218851 | XM_235295 | XM_344168 |
| XM_219796 | XM_235878 | XM_344426 |
| XM_220219 | XM_236614 | XM_345668 |
| XM_221270 | XM_240178 | XM_345848 |
| XM_221304 | XM_340884 | XM_346887 |
| XM_221627 | XM_340889 | |
| XM_221833 | XM_341015 | |
| XM_221910 | XM_341249 | |

The genes in this set show abundance of expressed gene product(s) in the Aged Impaired population that are significantly decreased, as determined by ANOVA, compared to those abundances in Young and Aged Unimpaired populations combined. In addition, the abundance of expressed gene product(s) in the aged animals negatively correlate with learning index of the animals, such that poorer learners have lower abundances of expressed gene products of the selected genes. Therefore, compounds useful in treating cognitive impairment selected using these genes should increase the abundance or enhance the function of the expressed gene products of these genes.

### Example 35

An ANOVA was conducted on the probe set signal values for all probe sets by combining two groups of animals and comparing them to the third group. An "AI ANOVA" was performed, where Aged Unimpaired were combined with Young and compared to Aged Impaired. All probe sets were considered in the statistical analysis based on the gcRMA method of data extraction from the perfect match sequences for each probe. Any probe sets having a p-value less than 0.05 were considered significantly changed and the genes or plurality of genes selected.

### Microarray Results

Table 35 shows the genes that were identified as being associated with cognitive impairment in this experiment. The reference sequence transcript ID (GENBANK® number for the exemplar sequence) was used to identify genes considered significantly changed. When this exemplar ID was not available, a UniGene identifier was used in conjunction with the GENBANK® accession number.

**TABLE 35**

| **CA3 - AI ANOVA INCREASE** | | |
|---|---|---|
| **GENBANK® ID** | **GENBANK® ID** | **GENBANK® ID** |
| AI029288 | NM_001012275 | NM_013132 |
| AI235284 | NM_001013087 | NM_013164 |
| AW535280 | NM_001013120 | NM_013191 |
| BE118404 | NM_001013179 | NM_013194 |
| BF288606 | NM_001013210 | NM_016989 |
| BI273920 | NM_001013213 | NM_017009 |
| BI274299 | NM_001013250 | NM_017013 |
| BM390128 | NM_001015026 | NM_017015 |
| NM_001001800 | NM_001024903 | NM_017024 |
| NM_001004068 | NM_001024925 | NM_017052 |
| NM_001004214 | NM_001025418 | NM_017060 |
| NM_001004239 | NM_001025423 | NM_017062 |
| NM_001005539 | NM_001025739 | NM_017109 |
| NM_001006963 | NM_001025744 | NM_017125 |
| NM_001007641 | NM_001031641 | NM_017137 |
| NM_001007712 | NM_001033683 | NM_017181 |
| NM_001007734 | NM_001033964 | NM_017193 |
| NM_001007739 | NM_001034004 | NM_017196 |
| NM_001007803 | NM_001034110 | NM_017200 |
| NM_001008312 | NM_001034157 | NM_017212 |
| NM_001008509 | NM_001034933 | NM_017234 |
| NM_001008861 | NM_001034999 | NM_017259 |
| NM_001008893 | NM_012519 | NM_017274 |
| NM_001009239 | NM_012532 | NM_017289 |
| NM_001009641 | NM_012555 | NM_017320 |
| NM_001009660 | NM_012573 | NM_017347 |
| NM_001009668 | NM_012703 | NM_017351 |
| NM_001009683 | NM_012713 | NM_019160 |
| NM_001011890 | NM_012717 | NM_019242 |
| NM_001011910 | NM_012722 | NM_019249 |
| NM_001011920 | NM_012775 | NM_019269 |
| NM_001011970 | NM_012788 | NM_019358 |
| NM_001011974 | NM_012806 | NM_020082 |
| NM_001011989 | NM_012842 | NM_021264 |
| NM_001012039 | NM_012884 | NM_021695 |
| NM_001012057 | NM_013015 | NM_021989 |
| NM_001012061 | NM_013016 | NM_022178 |
| NM_001012067 | NM_013029 | NM_022281 |
| NM_001012123 | NM_013047 | NM_022401 |
| NM_001012162 | NM_013070 | NM_022500 |
| NM_001012181 | NM_013086 | NM_022502 |
| NM_001012201 | NM_013088 | NM_022516 |
| NM_001012208 | NM_013091 | NM_022592 |
| NM_001012217 | NM_013107 | NM_022617 |
| NM_022677 | NM_053633 | NM_199093 |
| NM_022699 | NM_053639 | NM_199118 |
| NM_022800 | NM_053796 | NM_199388 |
| NM_022853 | NM_053840 | NM_199499 |
| NM_022856 | NM_053893 | NM_203366 |
| NM_023025 | NM_053895 | NM_206950 |
| NM_024001 | NM_058211 | NM_207609 |
| NM_024155 | NM_078622 | NM_212523 |
| NM_024372 | NM_080584 | NM_213563 |
| NM_024396 | NM_080688 | NM_214457 |
| NM_030871 | NM_131911 | XM_213688 |
| NM_030989 | NM_133298 | XM_214035 |
| NM_031005 | NM_133303 | XM_214480 |
| NM_031018 | NM_133307 | XM_214958 |
| NM_031027 | NM_133511 | XM_215076 |
| NM_031099 | NM_133534 | XM_215177 |
| NM_031107 | NM_133545 | XM_215466 |
| NM_031132 | NM_133598 | XM_215576 |
| NM_031321 | NM_133611 | XM_215607 |
| NM_031357 | NM_134334 | XM_215757 |
| NM_031509 | NM_134349 | XM_215935 |
| NM_031576 | NM_134390 | XM_215949 |
| NM_031620 | NM_134408 | XM_216004 |
| NM_031648 | NM_134453 | XM_216407 |
| NM_031672 | NM_138520 | XM_216565 |
| NM_031677 | NM_138828 | XM_216717 |
| NM_031694 | NM_138832 | XM_216872 |
| NM_031797 | NM_139043 | XM_216910 |
| NM_031806 | NM_139105 | XM_216968 |
| NM_031818 | NM_139110 | XM_217062 |
| NM_031827 | NM_139216 | XM_217188 |
| NM_031970 | NM_139255 | XM_217254 |
| NM_032067 | NM_147136 | XM_217263 |
| NM_032462 | NM_147206 | XM_217441 |
| NM_032619 | NM_147207 | XM_217570 |
| NM_033097 | NM_171992 | XM_218293 |
| NM_033230 | NM_172030 | XM_218336 |
| NM_033443 | NM_173116 | XM_218382 |
| NM_052981 | NM_173118 | XM_218617 |
| NM_053434 | NM_175595 | XM_219296 |
| NM_053442 | NM_175843 | XM_219539 |
| NM_053493 | NM_176075 | XM_219785 |
| NM_053506 | NM_177419 | XM_220175 |
| NM_053516 | NM_181475 | XM_220333 |
| NM_053550 | NM_182821 | XM_220805 |
| NM_053553 | NM_182843 | XM_220918 |
| NM_053560 | NM_198743 | XM_220986 |
| NM_053599 | NM_198787 | XM_221307 |
| XM_221387 | XM_233603 | XM_341940 |
| XM_222911 | XM_234281 | XM_341957 |
| XM_223781 | XM_235480 | XM_342002 |
| XM_224474 | XM_235552 | XM_342052 |
| XM_224535 | XM_235558 | XM_342218 |
| XM_224561 | XM_235609 | XM_342286 |
| XM_225020 | XM_235710 | XM_342397 |
| XM_225147 | XM_236009 | XM_342800 |
| XM_225628 | XM_236020 | XM_343259 |
| XM_226510 | XM_236196 | XM_343303 |
| XM_226874 | XM_236560 | XM_343396 |
| XM_226987 | XM_237371 | XM_343412 |
| XM_227066 | XM_237415 | XM_343479 |
| XM_227870 | XM_238205 | XM_344524 |
| XM_228044 | XM_240311 | XM_345535 |
| XM_228073 | XM_242032 | XM_574504 |
| XM_231566 | XM_242644 | XM_575585 |
| XM_231620 | XM_242982 | XM_576238 |
| XM_231650 | XM_243637 | XM_576459 |
| XM_231763 | XM_341111 | |
| XM_232809 | XM_341133 | |
| XM_232865 | XM_341877 | |

The genes in this set show abundance of expressed gene product(s) in the Aged Impaired population that are significantly increased, as determined by ANOVA, compared to those abundances in Young and Aged Unimpaired populations combined. Therefore, compounds useful in treating cognitive impairment selected using these genes should decrease the abundance or attenuate the function of the expressed gene products of these genes.

### Example 36

An ANOVA was conducted on the probe set signal values for all probe sets by combining two groups of animals and comparing them to the third group. An "AI ANOVA" was performed, where Aged Unimpaired were combined with Young and compared to Aged Impaired. All probe sets were considered in the statistical analysis based on the gcRMA method of data extraction from the perfect match sequences for each probe. Any probe sets having a p-value less than 0.05 were considered significantly changed and the genes or plurality of genes selected.

### Microarray Results

Table 36 shows the genes that were identified as being associated with cognitive impairment in this experiment. The reference sequence transcript ID (GENBANK® number for the exemplar sequence) was used to identify genes considered significantly changed. When this exemplar ID was not available, a UniGene identifier was used in conjunction with the GENBANK® accession number.

**TABLE 36**

| **CA3 - AI ANOVA DECREASE** | | |
|---|---|---|
| **GENBANK® ID** | **GENBANK® ID** | **GENBANK® ID** |
| A943681 | NM_001009619 | NM_001017377 |
| AI228348 | NM_001009640 | NM_001024261 |
| AW524426 | NM_001009661 | NM_001024360 |
| AW524733 | NM_001009719 | NM_001024754 |
| BE103926 | NM_001009831 | NM_001025032 |
| BF282660 | NM_001009967 | NM_001025114 |
| BF407666 | NM_001010963 | NM_001025271 |
| BG379941 | NM_001010965 | NM_001025411 |
| BG380494 | NM_001011901 | NM_001025686 |
| BI287800 | NM_001011906 | NM_001025696 |
| NM_001002253 | NM_001011924 | NM_001025705 |
| NM_001002828 | NM_001011932 | NM_001025738 |
| NM_001003978 | NM_001011950 | NM_001029900 |
| NM_001004078 | NM_001011951 | NM_001029910 |
| NM_001004132 | NM_001011955 | NM_001030023 |
| NM_001004133 | NM_001011966 | NM_001030029 |
| NM_001004210 | NM_001011998 | NM_001030030 |
| NM_001004227 | NM_001012017 | NM_001030037 |
| NM_001004235 | NM_001012035 | NM_001031645 |
| NM_001004250 | NM_001012038 | NM_001031659 |
| NM_001004277 | NM_001012060 | NM_001033079 |
| NM_001004279 | NM_001012068 | NM_001033674 |
| NM_001004442 | NM_001012103 | NM_001033675 |
| NM_001005884 | NM_001012119 | NM_001033679 |
| NM_001005885 | NM_001012129 | NM_001033684 |
| NM_001005905 | NM_001012144 | NM_001033702 |
| NM_001005908 | NM_001012152 | NM_001033708 |
| NM_001006970 | NM_001012187 | NM_001033715 |
| NM_001006981 | NM_001012191 | NM_001033899 |
| NM_001006997 | NM_001012195 | NM_001033974 |
| NM_001007014 | NM_001012197 | NM_001034014 |
| NM_001007149 | NM_001012473 | NM_001034083 |
| NM_001007643 | NM_001012738 | NM_001034104 |
| NM_001007720 | NM_001013058 | NM_001034112 |
| NM_001007742 | NM_001013076 | NM_001034925 |
| NM_001008299 | NM_001013170 | NM_001034937 |
| NM_001008317 | NM_001013183 | NM_001034994 |
| NM_001008382 | NM_001013189 | NM_001035001 |
| NM_001008766 | NM_001013198 | NM_001035221 |
| NM_001008888 | NM_001013218 | NM_012508 |
| NM_001009180 | NM_001013235 | NM_012513 |
| NM_001009258 | NM_001013426 | NM_012526 |
| NM_001009424 | NM_001013434 | NM_012598 |
| NM_001009536 | NM_001014793 | NM_012647 |
| NM_012664 | NM_019169 | NM_024362 |
| NM_012670 | NM_019182 | NM_024374 |
| NM_012736 | NM_019194 | NM_024403 |
| NM_012757 | NM_019196 | NM_024486 |
| NM_012828 | NM_019218 | NM_030835 |
| NM_012839 | NM_019226 | NM_031090 |
| NM_012856 | NM_019234 | NM_031138 |
| NM_012887 | NM_019264 | NM_031146 |
| NM_012905 | NM_019277 | NM_031152 |
| NM_012932 | NM_019304 | NM_031153 |
| NM_012934 | NM_019348 | NM_031235 |
| NM_012956 | NM_019376 | NM_031237 |
| NM_012985 | NM_019377 | NM_031325 |
| NM_013002 | NM_021697 | NM_031330 |
| NM_013018 | NM_021739 | NM_031334 |
| NM_013055 | NM_021757 | NM_031358 |
| NM_013067 | NM_021758 | NM_031360 |
| NM_013079 | NM_021765 | NM_031579 |
| NM_013131 | NM_021850 | NM_031603 |
| NM_013134 | NM_022008 | NM_031639 |
| NM_013177 | NM_022209 | NM_031662 |
| NM_013179 | NM_022278 | NM_031675 |
| NM_013189 | NM_022289 | NM_031676 |
| NM_013192 | NM_022300 | NM_031693 |
| NM_013214 | NM_022301 | NM_031718 |
| NM_013219 | NM_022383 | NM_031719 |
| NM_013223 | NM_022387 | NM_031728 |
| NM_017011 | NM_022521 | NM_031745 |
| NM_017025 | NM_022585 | NM_031757 |
| NM_017042 | NM_022609 | NM_031783 |
| NM_017063 | NM_022674 | NM_031785 |
| NM_017107 | NM_022685 | NM_031786 |
| NM_017136 | NM_022850 | NM_031802 |
| NM_017175 | NM_022863 | NM_031813 |
| NM_017243 | NM_022869 | NM_031824 |
| NM_017268 | NM_022934 | NM_031828 |
| NM_017278 | NM_022962 | NM_031969 |
| NM_017295 | NM_023957 | NM_031977 |
| NM_017313 | NM_023960 | NM_031978 |
| NM_017318 | NM_023974 | NM_031987 |
| NM_017322 | NM_023975 | NM_032057 |
| NM_017332 | NM_023977 | NM_032083 |
| NM_017344 | NM_023979 | NM_032614 |
| NM_017346 | NM_024137 | NM_052809 |
| NM_017352 | NM_024139 | NM_053291 |
| NM_017359 | NM_024156 | NM_053301 |
| NM_019124 | NM_024161 | NM_053316 |
| NM_019140 | NM_024351 | NM_053319 |
| NM_053337 | NM_057108 | NM_181083 |
| NM_053339 | NM_057196 | NM_181473 |
| NM_053346 | NM_080402 | NM_181626 |
| NM_053349 | NM_080411 | NM_182814 |
| NM_053351 | NM_080481 | NM_182819 |
| NM_053357 | NM_080582 | NM_183052 |
| NM_053404 | NM_080781 | NM_183332 |
| NM_053410 | NM_080887 | NM_184050 |
| NM_053420 | NM_080902 | NM_198749 |
| NM_053428 | NM_130429 | NM_198757 |
| NM_053441 | NM_130746 | NM_198788 |
| NM_053458 | NM_130779 | NM_199094 |
| NM_053490 | NM_131904 | NM_199104 |
| NM_053522 | NM_133313 | NM_199385 |
| NM_053556 | NM_133427 | NM_199393 |
| NM_053558 | NM_133528 | NM_199410 |
| NM_053588 | NM_133539 | NM_201421 |
| NM_053594 | NM_133566 | NM_207617 |
| NM_053605 | NM_134383 | NM_212494 |
| NM_053607 | NM_134456 | NM_212519 |
| NM_053623 | NM_138519 | NM_212549 |
| NM_053655 | NM_138856 | NM_213559 |
| NM_053682 | NM_138865 | X53232 |
| NM_053690 | NM_138890 | XM_213362 |
| NM_053693 | NM_138910 | XM_213382 |
| NM_053698 | NM_138911 | XM_213782 |
| NM_053707 | NM_139097 | XM_213898 |
| NM_053747 | NM_139325 | XM_214021 |
| NM_053748 | NM_144758 | XM_214053 |
| NM_053750 | NM_145184 | XM_214241 |
| NM_053764 | NM_145772 | XM_214296 |
| NM_053772 | NM_145788 | XM_214420 |
| NM_053775 | NM_147211 | XM_214446 |
| NM_053795 | NM_153297 | XM_214505 |
| NM_053801 | NM_153630 | XM_214625 |
| NM_053825 | NM_171990 | XM_214701 |
| NM_053868 | NM_172039 | XM_214833 |
| NM_053876 | NM_172074 | XM_214954 |
| NM_053888 | NM_172243 | XM_215044 |
| NM_053909 | NM_172332 | XM_215113 |
| NM_053928 | NM_173133 | XM_215118 |
| NM_053933 | NM_173137 | XM_215178 |
| NM_053947 | NM_173154 | XM_215286 |
| NM_053948 | NM_173290 | XM_215416 |
| NM_053979 | NM_173308 | XM_215424 |
| NM_054009 | NM_175595 | XM_215481 |
| NM_057098 | NM_177425 | XM_215549 |
| NM_057099 | NM_177929 | XM_215570 |
| XM_215612 | XM_222214 | XM_340987 |
| XM_215706 | XM_222478 | XM_340999 |
| XM_215717 | XM_222670 | XM_341071 |
| XM_215751 | XM_222946 | XM_341337 |
| XM_215758 | XM_223485 | XM_341354 |
| XM_215769 | XM_223580 | XM_341558 |
| XM_215771 | XM_223981 | XM_341653 |
| XM_216013 | XM_224271 | XM_341661 |
| XM_216212 | XM_224332 | XM_341669 |
| XM_216378 | XM_224417 | XM_341686 |
| XM_216393 | XM_224478 | XM_341700 |
| XM_216398 | XM_225078 | XM_341709 |
| XM_216563 | XM_225138 | XM_341803 |
| XM_216643 | XM_225923 | XM_341947 |
| XM_216661 | XM_226014 | XM_342048 |
| XM_217019 | XM_226238 | XM_342101 |
| XM_217124 | XM_226422 | XM_342149 |
| XM_217464 | XM_227811 | XM_342174 |
| XM_217560 | XM_229106 | XM_342180 |
| XM_217587 | XM_230523 | XM_342217 |
| XM_217592 | XM_230531 | XM_342223 |
| XM_218196 | XM_230861 | XM_342340 |
| XM_218790 | XM_231148 | XM_342442 |
| XM_219525 | XM_231251 | XM_342489 |
| XM_219685 | XM_232252 | XM_342534 |
| XM_219693 | XM_232343 | XM_342551 |
| XM_219801 | XM_233231 | XM_342581 |
| XM_219939 | XM_233467 | XM_342600 |
| XM_220047 | XM_233609 | XM_342632 |
| XM_220155 | XM_233944 | XM_342657 |
| XM_220167 | XM_234011 | XM_342829 |
| XM_220178 | XM_234272 | XM_342851 |
| XM_220269 | XM_234483 | XM_342857 |
| XM_220281 | XM_235185 | XM_342924 |
| XM_220428 | XM_235224 | XM_343018 |
| XM_220506 | XM_235496 | XM_343154 |
| XM_220534 | XM_235497 | XM_343175 |
| XM_220541 | XM_236476 | XM_343175 |
| XM_220753 | XM_236745 | XM_343459 |
| XM_220992 | XM_236941 | XM_343469 |
| XM_221050 | XM_237291 | XM_343557 |
| XM_221212 | XM_237327 | XM_343579 |
| XM_221276 | XM_237787 | XM_344130 |
| XM_221888 | XM_237957 | XM_344450 |
| XM_221941 | XM_238334 | XM_344706 |
| XM_221946 | XM_238346 | XM_344971 |
| XM_221962 | XM_340874 | XM_345150 |
| XM_222177 | XM_340986 | XM_345266 |
| XM_345938 | XM_574916 | XM_578542 |
| XM_573165 | XM_574979 | |
| XM_573256 | XM_574991 | |
| XM_574503 | XM_576252 | |

The genes in this set show abundance of expressed gene product(s) in the Aged Impaired population that are significantly decreased, as determined by ANOVA, compared to those abundances in Young and Aged Unimpaired populations combined. Therefore, compounds useful in treating cognitive impairment selected using these genes should increase the abundance or enhance the function of the expressed gene products of these genes.

### Example 37

An ANOVA was conducted on the probe set signal values for all probe sets by combining two groups of animals and comparing them to the third group. An "AU ANOVA" was performed, where Aged Impaired were combined with Young and compared to Aged Unimpaired. All probe sets were considered in the statistical analysis based on the gcRMA method of data extraction from the perfect match sequences for each probe. Any probe sets having a p-value less than 0.05 were considered significantly changed and the genes or plurality of genes selected.

Pearson's correlations comparing probe set signal values to learning indices were then calculated for the aged animals (excluding young) across all present probe sets. Again, correlations representing a p-value of less than 0.05 were considered significantly changed.

### Microarray Results

Table 37 shows the genes that were identified as being associated with cognitive impairment in this experiment. The reference sequence transcript ID (GENBANK® number for the exemplar sequence) was used to identify genes considered significantly changed. When this exemplar ID was not available, a UniGene identifier was used in conjunction with the GENBANK® accession number.

**TABLE 37**

| **CA3 - AU ANOVA POSITIVE CORRELATION** | | |
|---|---|---|
| **GENBANK® ID** | **GENBANK® ID** | **GENBANK® ID** |
| AW525560 | NM_017211 | NM_053335 |
| BF562800 | NM_017242 | NM_053358 |
| M59859 | NM_017288 | NM_053391 |
| NM_001002277 | NM_017290 | NM_053457 |
| NM_001003401 | NM_017294 | NM_053589 |
| NM_001004082 | NM_017304 | NM_053664 |
| NM_001004220 | NM_017327 | NM_053777 |
| NM_001005761 | NM_019128 | NM_053818 |
| NM_001005889 | NM_019148 | NM_053859 |
| NM_001006957 | NM_019288 | NM_053878 |
| NM_001007616 | NM_019375 | NM_053910 |
| NM_001008335 | NM_020075 | NM_053980 |
| NM_001008368 | NM_020088 | NM_054003 |
| NM_001008879 | NM_021597 | NM_057139 |
| NM_001009692 | NM_021659 | NM_057140 |
| NM_001009825 | NM_021748 | NM_057201 |
| NM_001011915 | NM_021767 | NM_080482 |
| NM_001012064 | NM_021775 | NM_080904 |
| NM_001012157 | NM_022206 | NM_133395 |
| NM_001012183 | NM_022254 | NM_133405 |
| NM_001012504 | NM_022533 | NM_133567 |
| NM_001017960 | NM_022589 | NM_133568 |
| NM_001024371 | NM_022668 | NM_134413 |
| NM_001024790 | NM_022690 | NM_138502 |
| NM_001024906 | NM_022703 | NM_138896 |
| NM_001029897 | NM_022946 | NM_138907 |
| NM_001033961 | NM_023020 | NM_139060 |
| NM_001034020 | NM_024397 | NM_144756 |
| NM_001034131 | NM_030862 | NM_145091 |
| NM_001034924 | NM_030990 | NM_145094 |
| NM_012504 | NM_031036 | NM_148891 |
| NM_012518 | NM_031037 | NM_153317 |
| NM_012673 | NM_031066 | NM_172023 |
| NM_012734 | NM_031117 | NM_173120 |
| NM_012809 | NM_031150 | NM_175708 |
| NM_012836 | NM_031515 | NM_175761 |
| NM_012877 | NM_031608 | NM_181087 |
| NM_012886 | NM_031613 | NM_181634 |
| NM_013038 | NM_031690 | NM_184049 |
| NM_013066 | NM_031707 | NM_199372 |
| NM_013126 | NM_031715 | XM_213824 |
| NM_013135 | NM_031730 | XM_214031 |
| NM_013181 | NM_031743 | XM_214172 |
| NM_017155 | NM_032617 | XM_214253 |
| NM_017190 | NM_053311 | XM_214740 |
| XM_214775 | XM_232946 | XM_341789 |
| XM_215182 | XM_232987 | XM_341857 |
| XM_215919 | XM_233767 | XM_341961 |
| XM_216739 | XM_233798 | XM_342279 |
| XM_217021 | XM_234901 | XM_342405 |
| XM_217059 | XM_234908 | XM_342612 |
| XM_217279 | XM_235179 | XM_342682 |
| XM_217432 | XM_235639 | XM_342684 |
| XM_218226 | XM_236268 | XM_343114 |
| XM_218704 | XM_237790 | XM_343175 |
| XM_220420 | XM_238072 | XM_343358 |
| XM_220423 | XM_238336 | XM_343468 |
| XM_221672 | XM_239329 | XM_343483 |
| XM_222245 | XM_240330 | XM_343513 |
| XM_224538 | XM_241981 | XM_343588 |
| XM_224713 | XM_340912 | XM_344661 |
| XM_226076 | XM_341081 | XM_344862 |
| XM_227510 | XM_341201 | XM_345867 |
| XM_231121 | XM_341341 | |
| XM_232345 | XM_341352 | |
| XM_232351 | XM_341548 | |

The genes in this set show abundance of expressed gene product(s) in the Aged Unimpaired population that are significantly decreased, as determined by ANOVA, compared to those abundances in Young and Aged Impaired populations combined. In addition, the abundance of expressed gene product(s) in the aged animals positively correlate with learning index of the animals, such that poorer learners have higher abundances of expressed gene products of the selected genes. Therefore, compounds useful in treating cognitive impairment selected using these genes should decrease the abundance or attenuate the function of the expressed gene products of these genes.

### Example 38

An ANOVA was conducted on the probe set signal values for all probe sets by combining two groups of animals and comparing them to the third group. An "AU ANOVA" was performed, where Aged Impaired were combined with Young and compared to Aged Unimpaired. All probe sets were considered in the statistical analysis based on the gcRMA method of data extraction from the perfect match sequences for each probe. Any probe sets having a p-value less than 0.05 were considered significantly changed and the genes or plurality of genes selected.

Pearson's correlations comparing probe set signal values to learning indices were then calculated for the aged animals (excluding young) across all present probe sets. Again, correlations representing a p-value of less than 0.05 were considered significantly changed.

### Microarray Results

Table 38 shows the genes that were identified as being associated with cognitive impairment in this experiment. The reference sequence transcript ID (GENBANK® number for the exemplar sequence) was used to identify genes considered significantly changed. When this exemplar ID was not available, a UniGene identifier was used in conjunction with the GENBANK® accession number.

**TABLE 38**

| **CA3 - AU ANOVA NEGATIVE CORRELATION** | | |
|---|---|---|
| **GENBANK® ID** | **GENBANK® ID** | **GENBANK® ID** |
| AA956668 | NM_031686 | XM_215858 |
| AI716912 | NM_031807 | XM_215947 |
| NM_001002807 | NM_031985 | XM_216380 |
| NM_001005561 | NM_033021 | XM_218759 |
| NM_001008773 | NM_053539 | XM_219297 |
| NM_001008826 | NM_053635 | XM_222184 |
| NM_001009651 | NM_053642 | XM_224232 |
| NM_001011983 | NM_053787 | XM_228305 |
| NM_001014044 | NM_053890 | XM_228644 |
| NM_001017376 | NM_080899 | XM_230634 |
| NM_001024274 | NM_133304 | XM_230846 |
| NM_001025407 | NM_134457 | XM_232247 |
| NM_012517 | NM_138710 | XM_232253 |
| NM_012548 | NM_138839 | XM_233970 |
| NM_013001 | NM_138874 | XM_234017 |
| NM_013218 | NM_138887 | XM_234238 |
| NM_017171 | NM_139192 | XM_236380 |
| NM_019318 | NM_181638 | XM_238163 |
| NM_019384 | NM_199084 | XM_342432 |
| NM_020308 | XM_213658 | XM_343910 |
| NM_022282 | XM_214191 | XM_344606 |
| NM_022626 | XM_214509 | XM_345418 |
| NM_022681 | XM_214906 | XM_347168 |
| NM_022686 | XM_215303 | XM_573259 |
| NM_031642 | XM_215540 | |

The genes in this set show abundance of expressed gene product(s) in the Aged Unimpaired population that are significantly increased, as determined by ANOVA, compared to those abundances in Young and Aged Impaired populations combined. In addition, the abundance of expressed gene product(s) in the aged animals negatively correlate with learning index of the animals, such that poorer learners have lower abundances of expressed gene products of the selected genes. Therefore, compounds useful in treating cognitive impairment selected using these genes should increase the abundance or enhance the function of the expressed gene products of these genes.

### Example 39

An ANOVA was conducted on the probe set signal values for all probe sets by combining two groups of animals and comparing them to the third group. An "AU ANOVA" was performed, where Aged Impaired were combined with Young and compared to Aged Unimpaired. All probe sets were considered in the statistical analysis based on the gcRMA method of data extraction from the perfect match sequences for each probe. Any probe sets having a p-value less than 0.05 were considered significantly changed and the genes or plurality of genes selected.

### Microarray Results

Table 39 shows the genes that were identified as being associated with cognitive impairment in this experiment. The reference sequence transcript ID (GENBANK® number for the exemplar sequence) was used to identify genes considered significantly changed. When this exemplar ID was not available, a UniGene identifier was used in conjunction with the GENBANK® accession number.

**TABLE 39**

| **CA3 - AU ANOVA INCREASE** | | |
|---|---|---|
| **GENBANK® ID** | **GENBANK® ID** | **GENBANK® ID** |
| AI071000 | NM_001034111 | NM_031699 |
| BI285065 | NM_001034164 | NM_031721 |
| NM_001004253 | NM_001034199 | NM_031771 |
| NM_001004273 | NM_012636 | NM_031779 |
| NM_001005557 | NM_012645 | NM_031789 |
| NM_001005907 | NM_012731 | NM_031793 |
| NM_001007666 | NM_012801 | NM_031798 |
| NM_001007704 | NM_012935 | NM_053453 |
| NM_001007755 | NM_012945 | NM_053541 |
| NM_001008293 | NM_012988 | NM_053584 |
| NM_001008344 | NM_013006 | NM_053604 |
| NM_001008521 | NM_013040 | NM_053713 |
| NM_001011894 | NM_013057 | NM_053714 |
| NM_001011941 | NM_016993 | NM_053826 |
| NM_001012016 | NM_017067 | NM_053842 |
| NM_001012021 | NM_017103 | NM_053887 |
| NM_001012049 | NM_017172 | NM_053946 |
| NM_001012087 | NM_017303 | NM_053965 |
| NM_001012128 | NM_017305 | NM_053998 |
| NM_001012160 | NM_019163 | NM_057107 |
| NM_001012169 | NM_019340 | NM_057147 |
| NM_001012174 | NM_019363 | NM_130400 |
| NM_001012459 | NM_022197 | NM_130409 |
| NM_001013077 | NM_022506 | NM_130413 |
| NM_001013137 | NM_022715 | NM_133392 |
| NM_001013148 | NM_022921 | NM_133571 |
| NM_001013178 | NM_024002 | NM_133593 |
| NM_001013193 | NM_024373 | NM_133620 |
| NM_001013873 | NM_024388 | NM_134326 |
| NM_001014790 | NM_024405 | NM_134364 |
| NM_001015027 | NM_024489 | NM_134389 |
| NM_001017381 | NM_030987 | NM_138873 |
| NM_001024247 | NM_030992 | NM_138888 |
| NM_001024275 | NM_031021 | NM_139332 |
| NM_001025027 | NM_031023 | NM_139336 |
| NM_001025123 | NM_031082 | NM_145789 |
| NM_001025693 | NM_031083 | NM_172157 |
| NM_001025708 | NM_031098 | NM_173123 |
| NM_001025716 | NM_031114 | NM_181363 |
| NM_001029909 | NM_031322 | NM_181432 |
| NM_001031655 | NM_031597 | NM_182816 |
| NM_001031660 | NM_031599 | NM_182953 |
| NM_001033699 | NM_031640 | NM_183331 |
| NM_001033705 | NM_031656 | NM_199111 |
| NM_199268 | XM_223390 | XM_237808 |
| NM_199403 | XM_223418 | XM_238039 |
| NM_201988 | XM_223643 | XM_238166 |
| NM_207602 | XM_223820 | XM_341173 |
| XM_213338 | XM_224588 | XM_341208 |
| XM_213370 | XM_224627 | XM_341280 |
| XM_213418 | XM_224733 | XM_341295 |
| XM_213679 | XM_225559 | XM_341312 |
| XM_214043 | XM_225644 | XM_341380 |
| XM_214362 | XM_225688 | XM_341508 |
| XM_214621 | XM_225864 | XM_341578 |
| XM_216225 | XM_226237 | XM_341590 |
| XM_216517 | XM_226769 | XM_341639 |
| XM_216725 | XM_226976 | XM_341688 |
| XM_217146 | XM_227475 | XM_342044 |
| XM_217191 | XM_227657 | XM_342271 |
| XM_217209 | XM_231524 | XM_342528 |
| XM_217210 | XM_232330 | XM_342605 |
| XM_217284 | XM_232466 | XM_342641 |
| XM_219531 | XM_232578 | XM_342837 |
| XM_220095 | XM_233485 | XM_343002 |
| XM_220335 | XM_233535 | XM_343088 |
| XM_220612 | XM_233815 | XM_343318 |
| XM_220813 | XM_234205 | XM_343326 |
| XM_221034 | XM_234540 | XM_343548 |
| XM_221231 | XM_234921 | XM_343559 |
| XM_221263 | XM_234942 | XM_344002 |
| XM_221333 | XM_235248 | XM_345652 |
| XM_222155 | XM_236275 | XM_347163 |
| XM_222773 | XM_236292 | XM_575968 |
| XM_223117 | XM_236914 | XM_576312 |
| XM_223161 | XM_237458 | |

The genes in this set show abundance of expressed gene product(s) in the Aged Unimpaired population that are significantly increased, as determined by ANOVA, compared to those abundances in Young and Aged Impaired populations combined. Therefore, compounds useful in treating cognitive impairment selected using these genes should increase the abundance or enhance the function of the expressed gene products of these genes.

### Example 40

An ANOVA was conducted on the probe set signal values for all probe sets by combining two groups of animals and comparing them to the third group. An "AU ANOVA" was performed, where Aged Impaired were combined with Young and compared to Aged Unimpaired. All probe sets were considered in the statistical analysis based on the gcRMA method of data extraction from the perfect match sequences for each probe. Any probe sets having a p-value less than 0.05 were considered significantly changed and the genes or plurality of genes selected.

### Microarray Results

Table 40 shows the genes that were identified as being associated with cognitive impairment in this experiment. The reference sequence transcript ID (GENBANK® number for the exemplar sequence) was used to identify genes considered significantly changed. When this exemplar ID was not available, a UniGene identifier was used in conjunction with the GENBANK® accession number.

**TABLE 40**

| **CA3 - AU ANOVA DECREASE** | | |
|---|---|---|
| **GENBANK® ID** | **GENBANK® ID** | **GENBANK® ID** |
| AW251888 | NM_012700 | NM_022693 |
| BE111604 | NM_012755 | NM_022864 |
| BI303536 | NM_012756 | NM_022953 |
| NM_001004200 | NM_012776 | NM_023101 |
| NM_001004206 | NM_012798 | NM_023972 |
| NM_001004233 | NM_012841 | NM_024000 |
| NM_001004262 | NM_012918 | NM_024146 |
| NM_001005555 | NM_012941 | NM_024163 |
| NM_001005560 | NM_012947 | NM_030846 |
| NM_001005881 | NM_013073 | NM_031008 |
| NM_001007607 | NM_013199 | NM_031056 |
| NM_001008364 | NM_013222 | NM_031081 |
| NM_001008694 | NM_016990 | NM_031097 |
| NM_001009618 | NM_017008 | NM_031147 |
| NM_001011925 | NM_017049 | NM_031331 |
| NM_001011926 | NM_017051 | NM_031342 |
| NM_001012004 | NM_017053 | NM_031518 |
| NM_001012065 | NM_017102 | NM_031522 |
| NM_001012066 | NM_017204 | NM_031595 |
| NM_001012113 | NM_017253 | NM_031614 |
| NM_001012175 | NM_017262 | NM_031646 |
| NM_001013079 | NM_017312 | NM_031654 |
| NM_001013103 | NM_017319 | NM_031667 |
| NM_001013231 | NM_019133 | NM_031692 |
| NM_001024269 | NM_019164 | NM_031765 |
| NM_001025414 | NM_019224 | NM_031776 |
| NM_001025649 | NM_019306 | NM_031777 |
| NM_001025688 | NM_019326 | NM_031820 |
| NM_001025722 | NM_019343 | NM_031831 |
| NM_001025770 | NM_019378 | NM_031975 |
| NM_001031845 | NM_019621 | NM_032062 |
| NM_001033670 | NM_021681 | NM_032616 |
| NM_001033681 | NM_021739 | NM_033359 |
| NM_001033889 | NM_021760 | NM_053310 |
| NM_001033914 | NM_021835 | NM_053409 |
| NM_001033968 | NM_021847 | NM_053435 |
| NM_001034009 | NM_021859 | NM_053440 |
| NM_001034135 | NM_022185 | NM_053486 |
| NM_012506 | NM_022262 | NM_053573 |
| NM_012527 | NM_022267 | NM_053620 |
| NM_012613 | NM_022382 | NM_053684 |
| NM_012628 | NM_022386 | NM_053788 |
| NM_012660 | NM_022507 | NM_053837 |
| NM_012686 | NM_022675 | NM_053846 |
| NM_053894 | NM_182668 | XM_234909 |
| NM_053955 | NM_182842 | XM_235156 |
| NM_053972 | NM_198749 | XM_238177 |
| NM_053973 | NM_198758 | XM_238280 |
| NM_057116 | NM_207599 | XM_239780 |
| NM_057136 | NM_212458 | XM_242062 |
| NM_057152 | XM_213437 | XM_243390 |
| NM_057207 | XM_213777 | XM_340886 |
| NM_057209 | XM_214313 | XM_340911 |
| NM_057213 | XM_214316 | XM_340967 |
| NM_080583 | XM_214673 | XM_341088 |
| NM_080896 | XM_214720 | XM_341091 |
| NM_130420 | XM_214751 | XM_341157 |
| NM_130428 | XM_214899 | XM_341896 |
| NM_130755 | XM_215371 | XM_341963 |
| NM_133406 | XM_215451 | XM_342244 |
| NM_133414 | XM_215467 | XM_342477 |
| NM_133582 | XM_216835 | XM_342653 |
| NM_134336 | XM_218432 | XM_342808 |
| NM_134376 | XM_218615 | XM_342823 |
| NM_134400 | XM_219529 | XM_342854 |
| NM_134404 | XM_220232 | XM_343581 |
| NM_138921 | XM_222103 | XM_343613 |
| NM_145090 | XM_222662 | XM_343839 |
| NM_145781 | XM_224389 | XM_345870 |
| NM_147209 | XM_226439 | XM_345909 |
| NM_172075 | XM_227282 | XM_345970 |
| NM_175754 | XM_228114 | XM_577103 |
| NM_177481 | XM_233737 | XM_578287 |
| NM_181092 | XM_234514 | |

The genes in this set show abundance of expressed gene product(s) in the Aged Unimpaired population that are significantly decreased, as determined by ANOVA, compared to those abundances in Young and Aged Impaired populations combined. Therefore, compounds useful in treating cognitive impairment selected using these genes should decrease the abundance or attenuate the function of the expressed gene products of these genes.

### Example 41

An ANOVA was conducted on the probe set signal values for all probe sets by combining two groups of animals and comparing them to the third group. An "AI ANOVA" was performed, where Aged Unimpaired were combined with Young and compared to Aged Impaired. All probe sets were considered in the statistical analysis based on the gcRMA method of data extraction from the perfect match sequences for each probe. Any probe sets having a p-value less than 0.05 were considered significantly changed and the genes or plurality of genes selected.

Pearson's correlations comparing probe set signal values to learning indices were then calculated for the aged animals (excluding young) across all present probe sets. Again, correlations representing a p-value of less than 0.05 were considered significantly changed.

### Microarray Results

Table 41 shows the genes that were identified as being associated with cognitive impairment in this experiment. The reference sequence transcript ID (GENBANK® number for the exemplar sequence) was used to identify genes considered significantly changed. When this exemplar ID was not available, a UniGene identifier was used in conjunction with the GENBANK® accession number.

**TABLE 41**

| **DG - AI ANOVA POSITIVE CORRELATION** | | |
|---|---|---|
| **GENBANK® ID** | **GENBANK® ID** | **GENBANK® ID** |
| AA892549 | NM_001009536 | NM_001013874 |
| AF023090 | NM_001009625 | NM_001014772 |
| AI317821 | NM_001009686 | NM_001015005 |
| AI717047 | NM_001009690 | NM_001024256 |
| BG662522 | NM_001009693 | NM_001024261 |
| BI298090 | NM_001009708 | NM_001024746 |
| BM390128 | NM_001009973 | NM_001024773 |
| BM391371 | NM_001011891 | NM_001025123 |
| NM_001001513 | NM_001011893 | NM_001025130 |
| NM_001002815 | NM_001011903 | NM_001025142 |
| NM_001002828 | NM_001011906 | NM_001025152 |
| NM_001004080 | NM_001011910 | NM_001025625 |
| NM_001004081 | NM_001011920 | NM_001025633 |
| NM_001004090 | NM_001011946 | NM_001025664 |
| NM_001004199 | NM_001011958 | NM_001025678 |
| NM_001004209 | NM_001011970 | NM_001025716 |
| NM_001004247 | NM_001011974 | NM_001031641 |
| NM_001004254 | NM_001012004 | NM_001031644 |
| NM_001004255 | NM_001012030 | NM_001033683 |
| NM_001004273 | NM_001012050 | NM_001033696 |
| NM_001005383 | NM_001012051 | NM_001033699 |
| NM_001005539 | NM_001012067 | NM_001033707 |
| NM_001005872 | NM_001012083 | NM_001033715 |
| NM_001005898 | NM_001012106 | NM_001033852 |
| NM_001005902 | NM_001012120 | NM_001033866 |
| NM_001006967 | NM_001012159 | NM_001033868 |
| NM_001006968 | NM_001012161 | NM_001033926 |
| NM_001006987 | NM_001012171 | NM_001033951 |
| NM_001006989 | NM_001012180 | NM_001033968 |
| NM_001007146 | NM_001012217 | NM_001033987 |
| NM_001007616 | NM_001012235 | NM_001034020 |
| NM_001007624 | NM_001013034 | NM_001034068 |
| NM_001007625 | NM_001013046 | NM_001034090 |
| NM_001007654 | NM_001013070 | NM_001034110 |
| NM_001007682 | NM_001013082 | NM_001034998 |
| NM_001007691 | NM_001013086 | NM_001034999 |
| NM_001007712 | NM_001013087 | NM_001035221 |
| NM_001008279 | NM_001013105 | NM_012500 |
| NM_001008319 | NM_001013118 | NM_012512 |
| NM_001008324 | NM_001013121 | NM_012527 |
| NM_001008356 | NM_001013167 | NM_012543 |
| NM_001008374 | NM_001013174 | NM_012569 |
| NM_001008509 | NM_001013206 | NM_012577 |
| NM_001009474 | NM_001013231 | NM_012595 |
| NM_012618 | NM_017317 | NM_022701 |
| NM_012628 | NM_017320 | NM_022799 |
| NM_012663 | NM_017340 | NM_022853 |
| NM_012732 | NM_017343 | NM_022939 |
| NM_012744 | NM_019124 | NM_023972 |
| NK_012749 | NM_019132 | NM_023978 |
| NK_012777 | NM_019141 | NM_024155 |
| NK_012788 | NM_019204 | NM_024359 |
| NM_012816 | NM_019211 | NM_024374 |
| NM_012820 | NM_019226 | NM_024399 |
| NM_012838 | NM_019232 | NM_024404 |
| NM_012884 | NM_019242 | NM_030826 |
| NM_012913 | NM_019253 | NM_030992 |
| NM_012963 | NM_019257 | NM_031022 |
| NM_012971 | NM_019272 | NM_031035 |
| NM_012993 | NM_019289 | NM_031057 |
| NM_013000 | NM_019359 | NM_031099 |
| NM_013013 | NM_019362 | NM_031114 |
| NM_013022 | NM_020308 | NM_031120 |
| NM_013088 | NM_021663 | NM_031147 |
| NM_013091 | NM_021694 | NM_031149 |
| NM_013154 | NM_021703 | NM_031357 |
| NM_013156 | NM_021746 | NM_031509 |
| NM_013164 | NM_021748 | NM_031525 |
| NM_013194 | NM_021766 | NM_031553 |
| NM_013198 | NM_021847 | NM_031554 |
| NM_013226 | NM_021859 | NM_031589 |
| NM_016990 | NM_021863 | NM_031596 |
| NM_017051 | NM_022207 | NM_031632 |
| NM_017052 | NM_022226 | NM_031654 |
| NM_017060 | NM_022265 | NM_031657 |
| NM_017068 | NM_022281 | NM_031664 |
| NM_017109 | NM_022286 | NM_031683 |
| NM_017116 | NM_022382 | NM_031711 |
| NM_017125 | NM_022390 | NM_031729 |
| NM_017132 | NM_022499 | NM_031745 |
| NM_017137 | NM_022500 | NM_031752 |
| NM_017148 | NM_022502 | NM_031770 |
| NM_017181 | NM_022523 | NM_031774 |
| NK_017204 | NM_022526 | NM_031786 |
| NM_017212 | NM_022538 | NM_031789 |
| NM_017216 | NM_022592 | NM_031812 |
| NM_017223 | NM_022595 | NM_031816 |
| NM_017232 | NM_022596 | NM_031827 |
| NM_017234 | NM_022597 | NM_031830 |
| NM_017264 | NM_022601 | NM_031837 |
| NM_017274 | NM_022617 | NM_031970 |
| NM_017288 | NM_022699 | NM_031981 |
| NM_032067 | NM_130409 | NM_199384 |
| NM_032416 | NM_130416 | NM_199388 |
| NM_032619 | NM_133398 | NM_199408 |
| NM_053323 | NM_133401 | NM_206847 |
| NM_053401 | NM_133404 | NM_207591 |
| NM_053411 | NM_133421 | NM_207599 |
| NM_053448 | NM_133551 | NM_212463 |
| NM_053462 | NM_133585 | NM_212491 |
| NM_053467 | NM_134376 | NM_212509 |
| NM_053507 | NM_134390 | XM_213329 |
| NM_053538 | NM_134410 | XM_213342 |
| NM_053555 | NM_134415 | XM_213463 |
| NM_053598 | NM_134449 | XK_213673 |
| NM_053599 | NM_134456 | XM_213677 |
| NM_053600 | NM_134468 | XM_213729 |
| NM_053604 | NM_138508 | XM_213739 |
| NM_053665 | NM_138877 | XM_213824 |
| NM_053698 | NM_138889 | XM_213832 |
| NM_053750 | NM_138896 | XM_213972 |
| NM_053776 | NM_138917 | XM_213993 |
| NM_053777 | NM_139107 | XM_214147 |
| NM_053794 | NM_139110 | XM_214172 |
| NM_053804 | NM_139256 | XM_214279 |
| NM_053851 | NM_139325 | XM_214518 |
| NM_053870 | NM_139329 | XM_214843 |
| NM_053886 | NM_147177 | XM_214859 |
| NM_053911 | NM_147210 | XM_214963 |
| NM_053927 | NM_153469 | XM_214968 |
| NM_053936 | NM_153628 | XM_215028 |
| NM_053959 | NM_153630 | XM_215101 |
| NM_053985 | NM_172009 | XM_215113 |
| NM_054001 | NM_172033 | XM_215177 |
| NM_057100 | NM_172334 | XM_215222 |
| NM_057114 | NM_173116 | XM_215285 |
| NM_057118 | NM_175578 | XM_215674 |
| NM_057131 | NM_175582 | XM_215733 |
| NM_057192 | NM_175765 | XM_215754 |
| NM_057197 | NM_177927 | XM_215935 |
| NM_057204 | NM_181388 | XM_215939 |
| NM_057208 | NM_181475 | XM_216124 |
| NM_057210 | NM_182953 | XM_216230 |
| NM_080480 | NM_183328 | XM_216306 |
| NM_080577 | NM_183402 | XM_216386 |
| NM_080691 | NM_198787 | XM_216407 |
| NM_080767 | NM_199087 | XM_216515 |
| NM_080773 | NM_199108 | XM_216565 |
| NM_080888 | NM_199118 | XM_216629 |
| NM_080910 | NM_199119 | XM_216704 |
| XM_216740 | XM_230637 | XM_341790 |
| XM_216835 | XM_231162 | XM_341796 |
| XM_216872 | XM_232323 | XM_341799 |
| XM_216968 | XM_232745 | XM_341826 |
| XM_217061 | XM_232855 | XM_341867 |
| XM_217195 | XM_233415 | XM_341882 |
| XM_217478 | XM_233761 | XM_341934 |
| XM_217740 | XM_233798 | XM_341940 |
| XM_218336 | XM_234008 | XM_341957 |
| XM_218816 | XM_234205 | XM_342002 |
| XM_218858 | XM_234394 | XM_342409 |
| XM_219001 | XM_235400 | XM_342542 |
| XM_219515 | XM_235426 | XM_342866 |
| XM_219885 | XM_235518 | XM_342918 |
| XM_219948 | XM_235657 | XM_343119 |
| XM_220512 | XM_236189 | XM_343157 |
| XM_220574 | XM_236192 | XM_343268 |
| XM_220775 | XM_236362 | XM_343336 |
| XM_221297 | XM_236468 | XM_343396 |
| XM_221747 | XM_236659 | XM_343564 |
| XM_222661 | XM_236687 | XM_343570 |
| XM_223012 | XM_237415 | XM_343773 |
| XM_223125 | XM_237787 | XM_343776 |
| XM_223597 | XM_238019 | XM_343986 |
| XM_223938 | XM_238155 | XM_344286 |
| XM_224232 | XM_241632 | XM_344403 |
| XM_224630 | XM_242032 | XM_344409 |
| XM_224788 | XM_243652 | XM_344870 |
| XM_225014 | XM_340798 | XM_344970 |
| XM_225259 | XM_340818 | XM_345477 |
| XM_225457 | XM_340825 | XM_345535 |
| XM_225726 | XM_340997 | XM_345870 |
| XM_226237 | XM_341051 | XM_346083 |
| XM_226987 | XM_341052 | XM_573258 |
| XM_227074 | XM_341086 | XM_574670 |
| XM_228044 | XM_341157 | XM_577023 |
| XM_228701 | XM_341704 | |
| XM_230291 | XM_341785 | |

The genes in this set show abundance of expressed gene product(s) in the Aged Impaired population that are significantly increased, as determined by ANOVA, compared to those abundances in Young and Aged Unimpaired populations combined. In addition, the abundance of expressed gene product(s) in the aged animals positively correlate with learning index of the animals, such that poorer learners have higher abundances of expressed gene products of the selected genes. Therefore, compounds useful in treating cognitive impairment selected using these genes should decrease the abundance or attenuate the function of the expressed gene products of these genes.

### Example 42

An ANOVA was conducted on the probe set signal values for all probe sets by combining two groups of animals and comparing them to the third group. An "AI ANOVA" was performed, where Aged Unimpaired were combined with Young and compared to Aged Impaired. All probe sets were considered in the statistical analysis based on the gcRMA method of data extraction from the perfect match sequences for each probe. Any probe sets having a p-value less than 0.05 were considered significantly changed and the genes or plurality of genes selected.

Pearson's correlations comparing probe set signal values to learning indices were then calculated for the aged animals (excluding young) across all present probe sets. Again, correlations representing a p-value of less than 0.05 were considered significantly changed.

### Microarray Results

Table 42 shows the genes that were identified as being associated with cognitive impairment in this experiment. The reference sequence transcript ID (GENBANK® number for the exemplar sequence) was used to identify genes considered significantly changed. When this exemplar ID was not available, a UniGene identifier was used in conjunction with the GENBANK® accession number.

**TABLE 42**

| **DG - AI ANOVA NEGATIVE CORRELATION** | | |
|---|---|---|
| **GENBANK® ID** | **GENBANK® ID** | **GENBANK® ID** |
| BF557865 | NM_031127 | XM_223729 |
| BM391206 | NM_053775 | XM_224733 |
| NM_001004102 | NM_134383 | XM_230288 |
| NM_001009689 | NM_173094 | XM_231453 |
| NM_001013071 | NM_212504 | XM_234508 |
| NM_001024238 | XM_218939 | XM_343446 |
| NM_013111 | XM_219292 | XM_344594 |
| NM_024355 | XM_219879 | XM_345938 |
| NM_031123 | XM_221074 | |

The genes in this set show abundance of expressed gene product(s) in the Aged Impaired population that are significantly decreased, as determined by ANOVA, compared to those abundances in Young and Aged Unimpaired populations combined. In addition, the abundance of expressed gene product(s) in the aged animals negatively correlate with learning index of the animals, such that poorer learners have lower abundances of expressed gene products of the selected genes. Therefore, compounds useful in treating cognitive impairment selected using these genes should increase the abundance or enhance the function of the expressed gene products of these genes.

### Example 43

An ANOVA was conducted on the probe set signal values for all probe sets by combining two groups of animals and comparing them to the third group. An "AI ANOVA" was performed, where Aged Unimpaired were combined with Young and compared to Aged Impaired. All probe sets were considered in the statistical analysis based on the gcRMA method of data extraction from the perfect match sequences for each probe. Any probe sets having a p-value less than 0.05 were considered significantly changed and the genes or plurality of genes selected.

### Microarray Results

Table 43 shows the genes that were identified as being associated with cognitive impairment in this experiment. The reference sequence transcript ID (GENBANK® number for the exemplar sequence) was used to identify genes considered significantly changed. When this exemplar ID was not available, a UniGene identifier was used in conjunction with the GENBANK® accession number.

**TABLE 43**

| **DG - AI ANOVA INCREASE** | | |
|---|---|---|
| **GENBANK® ID** | **GENBANK® ID** | **GENBANK® ID** |
| AI103331 | NM_001013081 | NM_012974 |
| AI235284 | NM_001013135 | NM_013015 |
| AW526364 | NM_001013179 | NM_013070 |
| BI291997 | NM_001013193 | NM_013104 |
| NM_001001511 | NM_001013204 | NM_013107 |
| NM_001001516 | NM_001013207 | NM_013122 |
| NM_001002016 | NM_001013210 | NM_013135 |
| NM_001002802 | NM_001013233 | NM_013137 |
| NM_001002835 | NM_001013246 | NM_013146 |
| NM_001004076 | NM_01015026 | NM_017009 |
| NM_001004099 | NM_001017382 | NM_017024 |
| NM_001004218 | NM_001024268 | NM_017037 |
| NM_001004245 | NM_001024771 | NM_017043 |
| NM_001005528 | NM_001024775 | NM_017062 |
| NM_001005529 | NM_001024782 | NM_017139 |
| NM_001005893 | NM_001025423 | NM_017142 |
| NM_001005907 | NM_001025630 | NM_017200 |
| NM_001006971 | NM_001025721 | NM_017224 |
| NM_001007001 | NM_001030026 | NM_017262 |
| NM_001007557 | NM_001030041 | NM_017351 |
| NM_001007677 | NM_001033653 | NM_019143 |
| NM_001007684 | NM_001033656 | NM_019144 |
| NM_001007749 | NM_001033757 | NM_019219 |
| NM_001007755 | NM_001034004 | NM_019249 |
| NM_001007802 | NM_001034164 | NM_019266 |
| NM_001008315 | NM_012515 | NM_019312 |
| NM_001008725 | NM_012523 | NM_019313 |
| NM_001008767 | NM_012532 | NM_019318 |
| NM_001008768 | NM_012546 | NM_019335 |
| NM_001008829 | NM_012562 | NM_019346 |
| NM_001009172 | NM_012576 | NM_019386 |
| NM_001009502 | NM_012578 | NM_019904 |
| NM_001009623 | NM_012614 | NM_019905 |
| NM_001009668 | NM_012650 | NM_020082 |
| NM_001009669 | NM_012701 | NM_020088 |
| NM_001009683 | NM_012703 | NM_021578 |
| NM_001011919 | NM_012722 | NM_021582 |
| NM_001011985 | NM_012740 | NM_021690 |
| NM_001012147 | NM_012771 | NM_021769 |
| NM_001012190 | NM_012856 | NM_021771 |
| NM_001012464 | NM_012862 | NM_021989 |
| NM_001013047 | NM_012886 | NM_022215 |
| NM_001013059 | NM_012925 | NM_022244 |
| NM_001013072 | NM_012940 | NM_022251 |
| NM_022257 | NM_053554 | NM_172029 |
| NM_022266 | NM_053560 | NM_173118 |
| NM_022270 | NM_053570 | NM_173145 |
| NM_022285 | NM_053583 | NM_175579 |
| NM_022401 | NM_053612 | NM_175756 |
| NM_022504 | NM_053618 | NM_181087 |
| NM_022516 | NM_053654 | NM_181368 |
| NM_022531 | NM_053670 | NM_181377 |
| NM_022677 | NM_053684 | NM_183330 |
| NM_022692 | NM_053796 | NM_184051 |
| NM_022866 | NM_053838 | NM_198786 |
| NM_024129 | NM_053857 | NM_199093 |
| NM_024131 | NM_053882 | NM_199208 |
| NM_024133 | NM_053896 | NM_199270 |
| NM_024353 | NM_053933 | NM_206849 |
| NM_024358 | NM_053936 | NM_207596 |
| NM_024369 | NM_053992 | NM_207598 |
| NM_024372 | NM_057107 | NM_207609 |
| NM_024400 | NM_057200 | NM_212466 |
| NM_030843 | NM_057211 | NM_212505 |
| NM_030847 | NM_080479 | NM_212523 |
| NM_030863 | NM_080584 | NM_212525 |
| NM_030872 | NM_080698 | NM_212547 |
| NM_030987 | NM_080899 | XM_213408 |
| NM_031018 | NM_131911 | XM_213421 |
| NM_031031 | NM_133296 | XM_213460 |
| NM_031118 | NM_133298 | XM_213526 |
| NM_031140 | NM_133305 | XM_213611 |
| NM_031145 | NM_133307 | XM_214344 |
| NM_031242 | NM_133317 | XM_214441 |
| NM_031320 | NM_133522 | XM_214526 |
| NM_031332 | NM_133526 | XM_214583 |
| NM_031511 | NM_133548 | XM_214673 |
| NM_031514 | NM_133598 | XM_214721 |
| NM_031549 | NM_133605 | XM_214769 |
| NM_031560 | NM_133624 | XM_214935 |
| NM_031630 | NM_134419 | XM_214993 |
| NM_031728 | NM_138502 | XM_215076 |
| NM_031739 | NM_138521 | XM_215095 |
| NM_031797 | NM_138542 | XM_215117 |
| NM_031814 | NM_139185 | XM_215372 |
| NM_031818 | NM_139216 | XM_215376 |
| NM_052804 | NM_139255 | XM_215424 |
| NM_053356 | NM_144757 | XM_215576 |
| NM_053455 | NM_145091 | XM_215578 |
| NM_053485 | NM_147205 | XM_216018 |
| NM_053503 | NM_147206 | XM_216688 |
| NM_053536 | NM_172022 | XM_218368 |
| XM_218963 | XM_230647 | XM_342092 |
| XM_218977 | XM_231134 | XM_342271 |
| XM_219045 | XM_231402 | XM_342284 |
| XM_219470 | XM_232283 | XM_342317 |
| XM_219966 | XM_232622 | XM_342432 |
| XM_220333 | XM_233779 | XM_342759 |
| XM_220398 | XM_235024 | XM_342829 |
| XM_220644 | XM_235049 | XM_342905 |
| XM_220810 | XM_235710 | XM_342979 |
| XM_220982 | XM_236458 | XM_343058 |
| XM_221272 | XM_236560 | XM_343065 |
| XM_221380 | XM_237371 | XM_343126 |
| XM_221387 | XM_237754 | XM_343259 |
| XM_221916 | XM_237792 | XM_343334 |
| XM_222636 | XM_238447 | XM_343479 |
| XM_223583 | XM_240178 | XM_343619 |
| XM_223781 | XM_240311 | XM_344421 |
| XM_224296 | XM_242644 | XM_344524 |
| XM_224538 | XM_242982 | XM_345584 |
| XM_224561 | XM_243637 | XM_345702 |
| XM_225196 | XM_243815 | XM_345789 |
| XM_225548 | XM_340939 | XM_345825 |
| XM_225625 | XM_341227 | XM_573544 |
| XM_225631 | XM_341448 | XM_573983 |
| XM_225644 | XM_341509 | XM_574161 |
| XM_225997 | XM_341540 | XM_574618 |
| XM_226016 | XM_341584 | XM_575585 |
| XM_226213 | XM_341784 | XM_576363 |
| XM_227066 | XM_341857 | XM_576401 |
| XM_228197 | XM_341877 | XM_580221 |
| XM_230036 | XM_341880 | |
| XM_230462 | XM_342072 | |

The genes in this set show abundance of expressed gene product(s) in the Aged Impaired population that are significantly increased, as determined by ANOVA, compared to those abundances in Young and Aged Unimpaired populations combined. Therefore, compounds useful in treating cognitive impairment selected using these genes should decrease the abundance or attenuate the function of the expressed gene products of these genes.

### Example 44

An ANOVA was conducted on the probe set signal values for all probe sets by combining two groups of animals and comparing them to the third group. An "AI ANOVA" was performed, where Aged Unimpaired were combined with Young and compared to Aged Impaired. All probe sets were considered in the statistical analysis based on the gcRMA method of data extraction from the perfect match sequences for each probe. Any probe sets having a p-value less than 0.05 were considered significantly changed and the genes or plurality of genes selected.

### Microarray Results

Table 44 shows the genes that were identified as being associated with cognitive impairment in this experiment. The reference sequence transcript ID (GENBANK® number for the exemplar sequence) was used to identify genes considered significantly changed. When this exemplar ID was not available, a UniGene identifier was used in conjunction with the GENBANK® accession number.

**TABLE 44**

| **DG - AI ANOVA DECREASE** | | |
|---|---|---|
| **GENBANK® ID** | **GENBANK® ID** | **GENBANK® ID** |
| NM_001005530 | NM_020097 | XM_219723 |
| NM_001005885 | NM_021597 | XM_220607 |
| NM_001006994 | NM_021702 | XM_221888 |
| NM_001007000 | NM_021760 | XM_221941 |
| NM_001007607 | NM_022615 | XM_221962 |
| NM_001008353 | NM_022688 | XM_222223 |
| NM_001008553 | NM_024364 | XM_222460 |
| NM_001009719 | NM_031082 | XM_222662 |
| NM_001010965 | NM_031318 | XM_224474 |
| NM_001011942 | NM_031571 | XM_224947 |
| NM_001012144 | NM_031613 | XM_226624 |
| NM_001013209 | NM_053301 | XM_233260 |
| NM_001017376 | NM_053340 | XM_233490 |
| NM_001024964 | NM_053360 | XM_233679 |
| NM_001025136 | NM_053441 | XM_234470 |
| NM_001025400 | NM_053883 | XM_236614 |
| NM_001031645 | NM_080397 | XM_237307 |
| NM_001033684 | NM_133315 | XM_239171 |
| NM_012585 | NM_133387 | XM_340856 |
| NM_012934 | NM_133562 | XM_340999 |
| NM_013066 | NM_144758 | XM_341241 |
| NM_017030 | NM_172034 | XM_341709 |
| NM_017093 | NM_173325 | XM_341983 |
| NM_017197 | NM_199081 | XM_342107 |
| NM_017259 | XM_214701 | XM_343817 |
| NM_017290 | XM_215134 | XM_343919 |
| NM_017318 | XM_215883 | XM_347168 |
| NM_017357 | XM_216477 | XM_575397 |
| NM_019264 | XM_217115 | |

The genes in this set show abundance of expressed gene product(s) in the Aged Impaired population that are significantly decreased, as determined by ANOVA, compared to those abundances in Young and Aged Unimpaired populations combined. Therefore, compounds useful in treating cognitive impairment selected using these genes should increase the abundance or enhance the function of the expressed gene products of these genes.

### Example 45

An ANOVA was conducted on the probe set signal values for all probe sets by combining two groups of animals and comparing them to the third group. An "AU ANOVA" was performed, where Aged Impaired were combined with Young and compared to Aged Unimpaired. All probe sets were considered in the statistical analysis based on the gcRMA method of data extraction from the perfect match sequences for each probe. Any probe sets having a p-value less than 0.05 were considered significantly changed and the genes or plurality of genes selected.

Pearson's correlations comparing probe set signal values to learning indices were then calculated for the aged animals (excluding young) across all present probe sets. Again, correlations representing a p-value of less than 0.05 were considered significantly changed.

### Microarray Results

Table 45 shows the genes that were identified as being associated with cognitive impairment in this experiment. The reference sequence transcript ID (GENBANK® number for the exemplar sequence) was used to identify genes considered significantly changed. When this exemplar ID was not available, a UniGene identifier was used in conjunction with the GENBANK® accession number.

**TABLE 45**

| **DG - AU ANOVA POSITIVE CORRELATION** | | |
|---|---|---|
| **GENBANK® ID** | **GENBANK® ID** | **GENBANK® ID** |
| AI501759 | NM_001024874 | NM_022296 |
| BE329232 | NM_001025409 | NM_022498 |
| NM_001003975 | NM_001025660 | NM_023971 |
| NM_001004253 | NM_001025688 | NM_024147 |
| NM_001004269 | NM_001025695 | NM_030989 |
| NM_001004275 | NM_001025735 | NM_031028 |
| NM_001005381 | NM_001025748 | NM_031125 |
| NM_001005876 | NM_001030023 | NM_031137 |
| NM_001006960 | NM_001031648 | NM_031139 |
| NM_001006963 | NM_001031653 | NM_031152 |
| NM_001006981 | NM_001031659 | NM_031515 |
| NM_001007020 | NM_001033065 | NM_031521 |
| NM_001007620 | NM_001033079 | NM_031528 |
| NM_001007714 | NM_001033670 | NM_031600 |
| NM_001007731 | NM_001033702 | NM_031641 |
| NM_001007744 | NM_001033870 | NM_031646 |
| NM_001008277 | NM_001034009 | NM_031768 |
| NM_001008291 | NM_001034026 | NM_031783 |
| NM_001008298 | NM_001034129 | NM_031979 |
| NM-001008338 | NM_001034834 | NM_032066 |
| NM_001008352 | NM_012615 | NM_053291 |
| NM_001008508 | NM_012809 | NM_053404 |
| NM_001008556 | NM_012836 | NM_053440 |
| NM_001008766 | NM_012991 | NM_053457 |
| NM_001008839 | NM_012998 | NM_053508 |
| NM_001008891 | NM_013053 | NM_053561 |
| NM_001009602 | NM_013192 | NM_053620 |
| NM_001009619 | NM_017029 | NM_053770 |
| NM_001009637 | NM_017040 | NM_053779 |
| NM_001009657 | NM_017075 | NM_053811 |
| NM_001009825 | NM_017190 | NM_053818 |
| NM_001011964 | NM_017201 | NM_053842 |
| NM_001011975 | NM_019133 | NM_053850 |
| NM_001011983 | NM_019271 | NM_053874 |
| NM_001012138 | NM_019352 | NM_053928 |
| NM_001012208 | NM_019379 | NM_053965 |
| NM_001012504 | NM_020099 | NM_053999 |
| NM_001013114 | NM_021262 | NM_054004 |
| NM_001013245 | NM_021697 | NM_054006 |
| NM_001017385 | NM_021770 | NM_057132 |
| NM_001017386 | NM_021835 | NM_057141 |
| NM_001024745 | NM_021869 | NM_080478 |
| NM_001024756 | NM_022198 | NM_080887 |
| NM_001024785 | NM_022282 | NM_080895 |
| NM_130406 | XM_213382 | XM_235552 |
| NM_130826 | XM_213437 | XM_235640 |
| NM_130894 | XM_213569 | XM_237999 |
| NM_133310 | XM_214155 | XM_238205 |
| NM_133569 | XM_214238 | XM_238280 |
| NM_133582 | XM_214420 | XM_238380 |
| NM_133594 | XM_214570 | XM_240330 |
| NM_133615 | XM_214625 | XM_340750 |
| NM_138528 | XM_214817 | XM_340775 |
| NM_138840 | XM_215659 | XM_341058 |
| NM_138899 | XM_215701 | XM_341120 |
| NM_138900 | XM_215847 | XM_341653 |
| NM_139109 | XM_215985 | XM_341666 |
| NM_139186 | XM_216041 | XM_341714 |
| NM_139194 | XM_216169 | XM_341856 |
| NM_139254 | XM_216198 | XM_341878 |
| NM_144754 | XM_216367 | XM_341961 |
| NM_145085 | XM_216400 | XM_342004 |
| NM_145878 | XM_216524 | XM_342286 |
| NM_152935 | XM_216801 | XM_342300 |
| NM_153297 | XM_217124 | XM_342489 |
| NM_171994 | XM_217592 | XM_342521 |
| NM_172157 | XM_217806 | XM_342532 |
| NM_173115 | XM_218720 | XM_342632 |
| NM_173290 | XM_218949 | XM_342872 |
| NM_175595 | XM_219693 | XM_343006 |
| NM_175603 | XM_220884 | XM_343034 |
| NM_175838 | XM_220982 | XM_343117 |
| NM_181081 | XM_221100 | XM_343175 |
| NM_182671 | XM_223227 | XM_343459 |
| NM_182820 | XM_223837 | XM_343513 |
| NM_183332 | XM_224169 | XM_343922 |
| NM_184049 | XM_224429 | XM_344113 |
| NM_198771 | XM_225468 | XM_347236 |
| NM_198789 | XM_225711 | XM_577103 |
| NM_199372 | XM_225983 | |
| NM_199380 | XM_232315 | |
| NM_199500 | XM_233231 | |

The genes in this set show abundance of expressed gene product(s) in the Aged Unimpaired population that are significantly decreased, as determined by ANOVA, compared to those abundances in Young and Aged Impaired populations combined. In addition, the abundance of expressed gene product(s) in the aged animals positively correlate with learning index of the animals, such that poorer learners have higher abundances of expressed gene products of the selected genes. Therefore, compounds useful in treating cognitive impairment selected using these genes should decrease the abundance or attenuate the function of the expressed gene products of these genes.

### Example 46

An ANOVA was conducted on the probe set signal values for all probe sets by combining two groups of animals and comparing them to the third group. An "AU ANOVA" was performed, where Aged Impaired were combined with Young and compared to Aged Unimpaired. All probe sets were considered in the statistical analysis based on the gcRMA method of data extraction from the perfect match sequences for each probe. Any probe sets having a p-value less than 0.05 were considered significantly changed and the genes or plurality of genes selected.

Pearson's correlations comparing probe set signal values to learning indices were then calculated for the aged animals (excluding young) across all present probe sets. Again, correlations representing a p-value of less than 0.05 were considered significantly changed.

### Microarray Results

Table 46 shows the genes that were identified as being associated with cognitive impairment in this experiment. The reference sequence transcript ID (GENBANK® number for the exemplar sequence) was used to identify genes considered significantly changed. When this exemplar ID was not available, a UniGene identifier was used in conjunction with the GENBANK® accession number.

**TABLE 46**

| **DG - AU ANOVA NEGATIVE CORRELATION** | | |
|---|---|---|
| **GENBANK® ID** | **GENBANK® ID** | **GENBANK® ID** |
| BF411876 | NM_031635 | XM_215947 |
| NM_001008511 | NM_031698 | XM_215990 |
| NM_001012093 | NM_053416 | XM_220222 |
| NM_001013090 | NM_053621 | XM_220813 |
| NM_001024258 | NM_133593 | XM_221043 |
| NM_001024865 | NM_134326 | XM_226666 |
| NM_001034994 | NM_172039 | XM_233808 |
| NM_013159 | NM_181362 | XM_341215 |
| NM_022202 | NM_212498 | XM_342580 |
| NM_022236 | XM_214253 | XM_344785 |
| NM_022518 | XM_215942 | |

The genes in this set show abundance of expressed gene product(s) in the Aged Unimpaired population that are significantly increased, as determined by ANOVA, compared to those abundances in Young and Aged Impaired populations combined. In addition, the abundance of expressed gene product(s) in the aged animals negatively correlate with learning index of the animals, such that poorer learners have lower abundances of expressed gene products of the selected genes. Therefore, compounds useful in treating cognitive impairment selected using these genes should increase the abundance or enhance the function of the expressed gene products of these genes.

### Example 47

An ANOVA was conducted on the probe set signal values for all probe sets by combining two groups of animals and comparing them to the third group. An "AU ANOVA" was performed, where Aged Impaired were combined with Young and compared to Aged Unimpaired. All probe sets were considered in the statistical analysis based on the gcRMA method of data extraction from the perfect match sequences for each probe. Any probe sets having a p-value less than 0.05 were considered significantly changed and the genes or plurality of genes selected.

### Microarray Results

Table 47 shows the genes that were identified as being associated with cognitive impairment in this experiment. The reference sequence transcript ID (GENBANK® number for the exemplar sequence) was used to identify genes considered significantly changed. When this exemplar ID was not available, a UniGene identifier was used in conjunction with the GENBANK® accession number.

**TABLE 47**

| **DG** - **AU ANOVA INCREASE** | | |
|---|---|---|
| **GENBANK® ID** | **GENBANK® ID** | **GENBANK® ID** |
| NM_001002831 | NM_031610 | XM_217837 |
| NM_001005888 | NM_031742 | XM_218293 |
| NM_001007680 | NM_031751 | XM_218432 |
| NM_001007704 | NM_040669 | XM_221037 |
| NM_001012092 | NM_052807 | XM_222155 |
| NM_001012195 | NM_053355 | XM_224720 |
| NM_001013120 | NM_053442 | XM_225168 |
| NM_001024767 | NM_053644 | XM_225253 |
| NM_001025125 | NM_053826 | XM_226315 |
| NM_001025722 | NM_057196 | XM_226976 |
| NM_001033701 | NM_080897 | XM_231143 |
| NM_012853 | NM_133302 | XM_232253 |
| NM_012935 | NM_133558 | XM_232735 |
| NM_012959 | NM_138922 | XM_233421 |
| NM_013126 | NM_153740 | XM_234238 |
| NM_016994 | NM_172327 | XM_235986 |
| NM_019170 | NM_213562 | XM_340785 |
| NM_021688 | XM_213921 | XM_340879 |
| NM_022269 | XM_214730 | XM_340935 |
| NM_022525 | XM_214906 | XM_341590 |
| NM_022684 | XM_215742 | XM_343146 |
| NM_023095 | XM_215949 | XM_344785 |
| NM_023977 | XM_215984 | XM_577078 |
| NM_024378 | XM_217246 | |
| NM_031569 | XM_217293 | |

The genes in this set show abundance of expressed gene product(s) in the Aged Unimpaired population that are significantly increased, as determined by ANOVA, compared to those abundances in Young and Aged Impaired populations combined. Therefore, compounds useful in treating cognitive impairment selected using these genes should increase the abundance or enhance the function of the expressed gene products of these genes.

### Example 48

An ANOVA was conducted on the probe set signal values for all probe sets by combining two groups of animals and comparing them to the third group. An "AU ANOVA" was performed, where Aged Impaired were combined with Young and compared to Aged Unimpaired. All probe sets were considered in the statistical analysis based on the gcRMA method of data extraction from the perfect match sequences for each probe. Any probe sets having a p-value less than 0.05 were considered significantly changed and the genes or plurality of genes selected.

### Microarray Results

Table 48 shows the genes that were identified as being associated with cognitive impairment in this experiment. The reference sequence transcript ID (GENBANK® number for the exemplar sequence) was used to identify genes considered significantly changed. When this exemplar ID was not available, a UniGene identifier was used in conjunction with the GENBANK® accession number.

**TABLE 48**

| **DG - AU ANOVA DECREASE** | | |
|---|---|---|
| **GENBANK® ID** | **GENBANK® ID** | **GENBANK® ID** |
| AI454679 | NM_012533 | NM_022589 |
| AW142720 | NM_012567 | NM_022600 |
| AW524426 | NM_012582 | NM_022675 |
| BE098467 | NM_012651 | NM_022681 |
| BF282715 | NM_012656 | NM_022858 |
| NM_001001510 | NM_012755 | NM_022863 |
| NM_001001719 | NM_012756 | NM_022936 |
| NM_001001799 | NM_012762 | NM_022951 |
| NM_001004094 | NM_012775 | NM_024000 |
| NM_001004132 | NM_012798 | NM_024146 |
| NM_001004238 | NM_012829 | NM_024158 |
| NM_001004274 | NM_012952 | NM_024383 |
| NM_001005547 | NM_012968 | NM_030836 |
| NM_001007637 | NM_013029 | NM_030861 |
| NM_001007721 | NM_013080 | NM_030862 |
| NM_001008301 | NM_013100 | NM_030868 |
| NM_001008368 | NM_013106 | NM_031007 |
| NM_001008558 | NM_013199 | NM_031030 |
| NM_001009631 | NM_016991 | NM_031034 |
| NM_001009641 | NM_017031 | NM_031063 |
| NM_001009713 | NM_017033 | NM_031132 |
| NM_001010953 | NM_017066 | NM_031333 |
| NM_001010961 | NM_017195 | NM_031351 |
| NM_001011896 | NM_017207 | NM_031518 |
| NM_001011904 | NM_017213 | NM_031594 |
| NM_001011956 | NM_017261 | NM_031692 |
| NM_001011978 | NM_017305 | NM_031701 |
| NM_001011987 | NM_017359 | NM_031707 |
| NM_001012113 | NM_019129 | NM_031831 |
| NM_001012152 | NM_019193 | NM_031855 |
| NM_001012175 | NM_019306 | NM_032613 |
| NM_001013103 | NM_019356 | NM_032616 |
| NM_001013199 | NM_021577 | NM_033359 |
| NM_001017537 | NM_021751 | NM_053363 |
| NM_001024747 | NM_021763 | NM_053375 |
| NM_001024784 | NM_021851 | NM_053428 |
| NM_001024906 | NM_022193 | NM_053438 |
| NM_001025421 | NM_022197 | NM_053506 |
| NM_001025753 | NM_022209 | NM_053601 |
| NM_001033066 | NM_022217 | NM_053655 |
| NM_001033693 | NM_022249 | NM_053854 |
| NM_001034131 | NM_022278 | NM_053866 |
| NM_001034198 | NM_022288 | NM_053973 |
| NM_012520 | NM_022548 | NM_057201 |
| NM_057205 | XM_215416 | XM_233955 |
| NM_131906 | XM_215612 | XM_233970 |
| NM_131907 | XM_216047 | XM_234156 |
| NM_131914 | XM_216563 | XM_234328 |
| NM_133560 | XM_216725 | XM_235164 |
| NM_133600 | XM_216755 | XM_235179 |
| NM_134336 | XM_216784 | XM_235338 |
| NM_134346 | XM_216859 | XM_236992 |
| NM_134367 | XM_216964 | XM_237179 |
| NM_139060 | XM_217078 | XM_237790 |
| NM_139113 | XM_217367 | XM_238127 |
| NM_139324 | XM_217464 | XM_238151 |
| NM_173126 | XM_217732 | XM_238177 |
| NM_173154 | XM_218382 | XM_243623 |
| NM_175758 | XM_219374 | XM_340884 |
| NM_175761 | XM_220224 | XM_341354 |
| NM_182842 | XM_220753 | XM_341612 |
| NM_198749 | XM_221120 | XM_342149 |
| NM_198764 | XM_221369 | XM_342174 |
| NM_199099 | XM_221566 | XM_342281 |
| NM_199256 | XM_222568 | XM_342396 |
| NM_199382 | XM_222578 | XM_342503 |
| NM_206950 | XM_222868 | XM_342533 |
| NM_212521 | XM_223496 | XM_342692 |
| NM_212532 | XM_224350 | XM_342900 |
| NM_214457 | XM_224417 | XM_343081 |
| XM_213433 | XM_224732 | XM_343114 |
| XM_213777 | XM_225923 | XM_343546 |
| XM_213779 | XM_226014 | XM_343588 |
| XM_214030 | XM_230495 | XM_343764 |
| XM_214400 | XM_231148 | XM_344135 |
| XM_214451 | XM_231655 | XM_344405 |
| XM_214522 | XM_232640 | XM_345867 |
| XM_214751 | XM_232809 | XM_573442 |
| XM_214775 | XM_232941 | XM_574644 |
| XM_215389 | XM_233297 | |

The genes in this set show abundance of expressed gene product(s) in the Aged Unimpaired population that are significantly decreased, as determined by ANOVA, compared to those abundances in Young and Aged Impaired populations combined. Therefore, compounds useful in treating cognitive impairment selected using these genes should decrease the abundance or attenuate the function of the expressed gene products of these genes.

While this invention has been particularly shown and described with references to preferred elements thereof, it will be understood by those skilled in the art that various changes in form and details may be made therein without departing from the scope of the invention encompassed by the appended claims.

### CLAUSES:

1. A method of identifying a gene or a plurality of genes associated with cognitive impairment in a mammal comprising the steps of:
   (a) providing an AI population of aged mammals with cognitive impairment;
   (b) providing an AU population of aged mammals without cognitive impairment;
   (c) providing a Y population of young mammals;
      (d)determining the abundance of a plurality of expressed gene products in one or more of the CA1, CA3, and DG hippocampal tissue of each mammal in the AI, AU and Y populations; and
   (e) selecting a gene or a plurality of genes based on a significant increase or decrease in the relative abundance of the gene's expressed gene product in the AI population of mammals relative to the combined AU and Y populations or in the AU population of mammals relative to the combined AI and Y populations.
2. A method of identifying a gene or a plurality of genes associated with cognitive impairment in a mammal comprising the steps of:
   (a) providing an AI population of aged mammals with cognitive impairment;
   (b) providing an AU population of aged mammals without cognitive impairment;
   (c) providing a Y population of young mammals;
      (d)determining the abundance of a plurality of expressed gene products in one or more of the CA1, CA3, and DG hippocampal tissue of each mammal in the AI, AU and Y populations;
   (e) selecting a gene or a plurality of genes based on a significant increase or decrease in the relative abundance of the gene's expressed gene product in the AI population of mammals relative to the combined AU and Y populations or in the AU population of mammals relative to the combined AI and Y populations;
   (f) correlating the increased or decreased abundance of a selected gene's expressed gene products with the level of cognitive impairment in each mammal of the AU and AI populations; and
   (g) selecting a gene or a plurality of genes based on a significant correlation between the relative abundance of the gene's expressed gene product and the level of cognitive impairment in the mammals.
3. The method according to clause 1 or 2, wherein the significance of the increase or decrease in the relative abundance is determined using a parametric test.
4. The method according to clause 3, wherein the parametric test is an analysis of variance (ANOVA).
5. The method according to clause 1 or 2, wherein the significance of the increase or decrease in relative abundance is determined using a non-parametric test.
6. The method according to clause 5, wherein the non-parametric test is selected from the group consisting of a Mann-Whitney U test, a Wilcoxon ranksum test, a Wilcoxon matched pairs signed-rank test, a Mann-Whitney-Wilcoxon test, a Kruskal-Wallis analysis of variance by ranks, a Friedman two way analysis of variance, and a Kolmogorov-Smirnov test.
7. The method according to clause 2, wherein the significance of the correlation is determined using a parametric Pearson correlation coefficient.
8. The method according to clause 2, wherein the significance of the correlation is determined using a nonparametric Spearman rank correlation coefficient.
9. The method according to clause 1 or 2, wherein the abundance of the plurality of expressed gene products is determined by a method selected from the group consisting of microarray analysis, macroarray analysis, in situ hybridization histochemistry, fluorescent in situ hybridization (FISH), immunocytochemistry (ICC), enzyme linked immunosorbent assay (ELISA), immuneprecipitation, quantitative polymerase chain reaction (PCR), serial analysis of gene expression (SAGE) analysis, radioimmunoassay (RIA) and blot analysis.
10. The method according to any one of clauses 1 to 9, wherein the mammal is an outbred strain of rat.
11. The method according to any one of clauses 1 to 10, wherein the expressed gene products are RNAs.
12. The method according to any one of clauses 1 to 11, wherein the expressed gene products are proteins, polypeptides or peptides.
13. A method of identifying a compound useful in the treatment of cognitive impairment comprising the steps of:
   (a) determining the abundance or function of an expressed gene product in a mammalian cell of the mammalian cell's homologue of a gene or a plurality of genes identified by the method of clause 1 or 2 to increase in abundance in the AI population relative to the combined AU and Y populations or to decrease in abundance in the AU population relative to the combined AI and Y populations or of a gene or genes selected from the group consisting of each of the individual genes listed in Tables 1, 3, 5, 8, 9, 11, 13, 16, 17, 19, 21, 24, 25, 27, 29, 32, 33, 35, 37, 40, 41, 43, 45 and 48 in the presence or absence of a candidate compound; and
   (b) identifying a compound from any of the candidate compound(s) that significantly decreases that abundance or attenuates the function of that gene or its expressed gene product in the mammalian cell to which the candidate compound is administered.
14. A method of identifying a compound useful in the treatment of cognitive impairment comprising the steps of:
   (a) determining the abundance or function of an expressed gene product in a mammalian cell of the mammalian cell's homologue of a gene or a plurality of genes identified by the method of clause 1 or 2 to decrease in abundance in the AI population relative to the combined AU and Y populations or to increase in abundance in the AU population relative to the combined AI and Y populations or of a gene or genes selected from the group consisting of each of the individual genes listed in Tables 2, 4, 6, 7, 10, 12, 14, 15, 18, 20, 22, 23, 26, 28, 30, 31, 34, 36, 38, 39, 42, 44, 46 and 47 in the presence or absence of a candidate compound; and
   (b) identifying a compound from any of the candidate compound(s) that significantly increases that abundance or enhances the function of that gene or its expressed gene product in the mammalian cell to which the candidate compound is administered.
15. The method according to clause 13 or 14, wherein the mammalian cell is selected from the group consisting of a neuronal cell and a glial cell.
16. The method according to clause 15, wherein the mammalian cell is a hippocampal cell.
17. The method according to clause 16, wherein the hippocampal cell is selected from the group of a CA1 cell, a CA3 cell or a DG cell.
18. A method of identifying a compound useful in the treatment of cognitive impairment comprising:
   (a) determining the abundance or function of an expressed gene product, in the hippocampus of a mammal, of the mammal's homologue of a gene or a plurality of genes identified by the method of clause 1 or 2 to increase in abundance in the AI population relative to the combined AU and Y populations or to decrease in abundance in the AU population relative to the combined AI and Y populations or a gene or genes selected from the group consisting of each of the individual genes listed in Tables 1, 3, 5, 8, 9, 11, 13, 16, 17, 19, 21, 24, 25, 27, 29, 32, 33, 35, 37, 40, 41, 43, 45 and 48 in the presence or absence of a candidate compound; and
   (b) identifying a compound from any of the candidate compound(s) that significantly decreases that abundance or attenuates the function of that gene or its expressed gene product in the mammal to whom the candidate compound is administered.
19. A method of identifying a compound useful in the treatment of cognitive impairment comprising:
   (a) determining the abundance or function of an expressed gene product, in the hippocampus of a mammal, of the mammal's homologue of a gene or a plurality of genes identified by the method of clause 1 or 2 to decrease in abundance in the AI population relative to the combined AU and Y populations or to increase in abundance in the AU population relative to the combined AI and Y populations or a gene or genes selected from the group consisting of each of the individual genes listed in Tables 2, 4, 6, 7, 10, 12, 14, 15, 18, 20, 22, 23, 26, 28, 30, 31, 34, 36, 38, 39, 42, 44, 46 and 47 in the presence or absence of a candidate compound; and
   (b) identifying a compound from any of the candidate compound(s) that significantly increases that abundance or enhances the function of that gene or its expressed gene product in the mammal to whom the candidate compound is administered.
20. The method according to clause 18 or 19, wherein the mammal is an aged mammal.
21. The method according to clause 20, wherein the aged mammal is cognitively impaired.
22. A method of identifying a compound useful in the treatment of cognitive impairment in aging comprising:
   (a) determining the abundance or function of an expressed gene product, in the hippocampus of an aged mammal with cognitive impairment, of the mammal's homologue of a gene or a plurality of genes identified by the method of clause 1 or 2 to increase in abundance in the AI population relative to the combined AU and Y populations or to decrease in abundance in the AU population relative to the combined AI and Y populations or a gene or genes selected from the group consisting of each of the individual genes listed in Tables 1, 3, 5, 8, 9, 11, 13, 16, 17, 19, 21, 24, 25, 27, 29, 32, 33, 35, 37, 40, 41, 43, 45 and 48 in the presence or absence of a candidate compound; and
   (b) identifying a compound from among the candidate compound(s) that significantly decreases that abundance or attenuates the function of that gene or its expressed gene product in the aged mammal with cognitive impairment to whom the compound is administered relative to a member of the group consisting of an aged mammal without cognitive impairment, a young mammal, an aged cognitively impaired mammal in the absence of the compound, and two or more of them.
23. A method of identifying a compound useful in the treatment of cognitive impairment in aging comprising:
   (a) determining the abundance or function of an expressed gene product, in the hippocampus of an aged mammal with cognitive impairment, of the mammal's homologue of a gene or a plurality of genes identified by the method of clause 1 or 2 to decrease in abundance in the AI population relative to the combined AU and Y populations or to increase in abundance in the AU population relative to the combined AI and Y populations or a gene or genes selected from the group consisting of each of the individual genes listed in Tables 2, 4, 6, 7, 10, 12, 14, 15, 18, 20, 22, 23, 26, 28, 30, 31, 34, 36, 38, 39, 42, 44, 46 and 47 in the presence or absence of a candidate compound; and
   (b) identifying a compound from among the candidate compound(s) that significantly increases that abundance or enhances the function of that gene or its expressed gene product in the aged mammal with cognitive impairment to whom the compound is administered relative to a member of the group consisting of an aged mammal without cognitive impairment, a young mammal, an aged cognitively impaired mammal in the absence of the compound, and two or more of them.
24. The method according to clause 22 or 23, wherein the compound does not significantly alter that abundance or function in a member of the group consisting of a young mammal, an aged cognitively unimpaired mammal and both, to whom the candidate compound is administered.
25. A method of identifying a compound useful in the treatment of cognitive impairment comprising:
   (a) determining the cognitive status of a mammal in the presence or absence of a candidate compound believed to decrease the abundance or attenuate the function of an expressed gene product of a gene or a plurality of genes identified by the method of clause 1 or 2 to increase in abundance in the AI population relative to the combined AU and Y populations or to decrease in abundance in the AU population relative to the combined AI and Y populations or a gene or genes selected from the group consisting of each of the individual genes listed in Tables 1, 3, 5, 8, 9, 11, 13, 16, 17, 19, 21, 24, 25, 27, 29, 32, 33, 35, 37, 40, 41, 43, 45 and 48; and
   (b) identifying the compound from among the candidate compound(s) that beneficially alters the cognitive status of the mammal to whom the compound is administered.
26. A method of identifying a compound useful in the treatment of cognitive impairment comprising:
   (a) determining the cognitive status of a mammal in the presence or absence of a candidate compound believed to increase the abundance or enhance the function of an expressed gene product of a gene or a plurality of genes identified by the method of clause 1 or 2 to decrease in abundance in the AI population relative to the combined AU and Y populations or to increase in abundance in the AU population relative to the combined AI and Y populations or a gene or genes selected from the group consisting of each of the individual genes listed in Tables 2, 4, 6, 7, 10, 12, 14, 15, 18, 20, 22, 23, 26, 28, 30, 31, 34, 36, 38, 39, 42, 44, 46 and 47; and
   (b) identifying the compound from among the candidate compound(s) that beneficially alters the cognitive status of the mammal to whom the compound is administered.
27. The method according to clause 25 or 26, wherein the mammal is an aged mammal.
28. The method according to clause 27, wherein the aged mammal is cognitively impaired.
29. A method of identifying a compound useful in the treatment of cognitive impairment in aging comprising:
   (a) determining the cognitive status of an aged mammal with cognitive impairment, in the presence or absence of a candidate compound believed to decrease the abundance or attenuate the function of an expressed gene product of a gene or a plurality of genes identified by the method of clause 1 or 2 to increase in abundance in the AI population relative to the combined AU and Y populations or to decrease in abundance in the AU population relative to the combined AI and Y populations or a gene or genes selected from the group consisting of each of the individual genes listed in Tables 1, 3, 5, 8, 9, 11, 13, 16, 17, 19, 21, 24, 25, 27, 29, 32, 33, 35, 37, 40, 41, 43, 45 and 48; and
   (b) identifying a compound from among the candidate compound(s) that beneficially alters the cognitive function in the aged mammal with cognitive impairment to whom the compound is administered relative to a member of the group consisting of an aged mammal without cognitive impairment, a young mammal, an aged cognitively impaired mammal to whom the compound is not administered, and two or more of them.
30. A method of identifying a compound useful in the treatment of cognitive impairment in aging comprising:
   (a) determining the cognitive status of an aged mammal with cognitive impairment, in the presence or absence of a candidate compound believed to increase the abundance or enhance the function of an expressed gene product of a gene or a plurality of genes identified by the method of clause 1 or 2 to decrease in abundance in the AI population relative to the combined AU and Y populations or to increase in abundance in the AU population relative to the combined AI and Y populations or a gene or genes selected from the group consisting of each of the individual genes listed in Tables 2, 4, 6, 7, 10, 12, 14, 15, 18, 20, 22, 23, 26, 28, 30, 31, 34, 36, 38, 39, 42, 44, 46 and 47; and
   (b) identifying a compound from among the candidate compound(s) that beneficially alters the cognitive function in the aged mammal with cognitive impairment to whom the compound is administered relative to a member of the group consisting of an aged mammal without cognitive impairment, a young mammal, an aged cognitively impaired mammal to whom the compound is not administered, and two or more of them.
31. The method according to clause 29 or 30, wherein the compound does not significantly alter the cognitive status of a member of the group consisting of a young mammal, an aged cognitively unimpaired mammal, and both, to whom the compound is administered.
32. The method according to any one of clauses 25 to 31, wherein cognitive function is hippocampal-dependent function.
33. The method according to clause 32, wherein the hippocampal-dependent function is selected from the group consisting of spatial memory acquisition, long-term spatial memory, and spatial memory retrieval.
34. The method according to any one of clauses 19 to 33, wherein the mammal is an outbred strain of rat.
35. The method according to any one of clauses 19 to 33, wherein the mammal is a human.
36. The method according to any one of clauses 19 to 24, wherein the abundance is determined in CA1, CA3 or DG hippocampal tissue.
37. The method according to clause 1, wherein the identified gene(s) are selected from the group consisting of each of the individual genes listed in Tables 1-48.
38. The method according to clause 2, wherein the identified gene(s) are selected from the group consisting of each of the individual genes listed in Tables 1, 2, 5, 6, 9, 10, 13, 14, 17, 18, 21, 22, 25, 26, 29, 30, 33, 34, 37, 38, 41, 42, 45 and 46.
39. The method according to any one of clauses 14, 19, 23, 26 and 30 wherein the expressed gene product is the product of the GABA-5 α5 receptor gene (GENBANK® accession number NM_017295) or its homologues.
40. A method for treating cognitive impairment in a mammal in need of such treatment, the method comprising the step of treating the mammal with GABA-5 α5 receptor agonist.
41. The method according to clause 40, wherein the mammal is a human.
42. The method according to clause 40 and 41, wherein the agonist is selected from the group consisting of 1-methyl-7-acetyleno-5-phenyl-1,3-dihydro-benzo[e]-1,4-diazepin-2-one, S(CH₂)₃0H, and 8-ethylthio-3-methyl-5-(1-oxidopyridin-2-yl)-3,4-dihydronaphthalen-1(2H)-one.
43. A pharmaceutical composition for treating cognitive impairment, the composition comprising pharmaceutically effective amount of a GABA5 α5 receptor agonist and a pharmaceutically acceptable carrier.
44. The pharmaceutical composition according to clause 43, wherein the agonist is selected from the group consisting of 1-methyl-7-acetyleno-5-phenyl-1,3-dihydro-benzo[e]-1,4-diazepin-2-one, S(CH₂)₃0H, and 8-ethylthio-3-methyl-5-(1-oxidopyridin-2-yl)-3,4-dihydronaphthalen-1(2H)-one.

## Claims

1. A method of identifying a gene or a plurality of genes associated with cognitive impairment in a mammal comprising the steps of:
(a) providing an AI population of aged mammals with cognitive impairment;
(b) providing an AU population of aged mammals without cognitive impairment;
(c) providing a Y population of young mammals;
(d) determining the abundance of a plurality of expressed gene products in one or more of the CA1, CA3, and DG hippocampal tissue of each mammal in the AI, AU and Y populations; and
(e) selecting a gene or a plurality of genes based on a significant increase or decrease in the relative abundance of the gene's expressed gene product in the AI population of mammals relative to the combined AU and Y populations or in the AU population of mammals relative to the combined AI and Y populations;
and optionally
(f) correlating the increased or decreased abundance of a selected gene's expressed gene products with the level of cognitive impairment in each mammal of the AU and AI populations; and
(g) selecting a gene or a plurality of genes based on a significant correlation between the relative abundance of the gene's expressed gene product and the level of cognitive impairment in the mammals.

2. The method according to claim 1, wherein the significance of the increase or decrease in the relative abundance is determined using a parametric test; preferably wherein the parametric test is an analysis of variance (ANOVA);
or wherein the significance of the increase or decrease in relative abundance is determined using a non-parametric test; preferably wherein the non-parametric test is selected from the group consisting of a Mann-Whitney U test, a Wilcoxon ranksum test, a Wilcoxon matched pairs signed-rank test, a Mann-Whitney-Wilcoxon test, a Kruskal-Wallis analysis of variance by ranks, a Friedman two way analysis of variance, and a Kolmogorov-Smirnov test.

3. The method according to claim 1, wherein the significance of the correlation is determined using a parametric Pearson correlation coefficient;
or wherein the significance of the correlation is determined using a nonparametric Spearman rank correlation coefficient.

4. The method according to claim 1, wherein the abundance of the plurality of expressed gene products is determined by a method selected from the group consisting of microarray analysis, macroarray analysis, in situ hybridization histochemistry, fluorescent in situ hybridization (FISH), immunocytochemistry (ICC), enzyme linked immunosorbent assay (ELISA), immuneprecipitation, quantitative polymerase chain reaction (PCR), serial analysis of gene expression (SAGE) analysis, radioimmunoassay (RIA) and blot analysis.

5. The method according to any one of claims 1 to 4, wherein the expressed gene products are RNAs; or wherein the expressed gene products are proteins, polypeptides or peptides.

6. A method of identifying a compound useful in the treatment of cognitive impairment comprising the steps of:
(a) determining the abundance or function of an expressed gene product in a mammalian cell of the mammalian cell's homologue of a gene or a plurality of genes identified by the method of claim 1 to increase in abundance in the AI population relative to the combined AU and Y populations or to decrease in abundance in the AU population relative to the combined AI and Y populations or of a gene or genes selected from the group consisting of each of the individual genes listed in Tables 1, 3, 5, 8, 9, 11, 13, 16, 17, 19, 21, 24, 25, 27, 29, 32, 33, 35, 37, 40, 41, 43, 45 and 48 in the presence or absence of a candidate compound; and
(b) identifying a compound from any of the candidate compound(s) that significantly decreases that abundance or attenuates the function of that gene or its expressed gene product in the mammalian cell to which the candidate compound is administered.

7. A method of identifying a compound useful in the treatment of cognitive impairment comprising the steps of:
(a) determining the abundance or function of an expressed gene product in a mammalian cell of the mammalian cell's homologue of a gene or a plurality of genes identified by the method of claim 1 to decrease in abundance in the AI population relative to the combined AU and Y populations or to increase in abundance in the AU population relative to the combined AI and Y populations or of a gene or genes selected from the group consisting of each of the individual genes listed in Tables 2, 4, 6, 7, 10, 12, 14, 15, 18, 20, 22, 23, 26, 28, 30, 31, 34, 36, 38, 39, 42, 44, 46 and 47 in the presence or absence of a candidate compound; and
(b) identifying a compound from any of the candidate compound(s) that significantly increases that abundance or enhances the function of that gene or its expressed gene product in the mammalian cell to which the candidate compound is administered.

8. The method according to claim 6 or 7, wherein the mammalian cell is selected from the group consisting of a neuronal cell and a glial cell; preferably wherein the mammalian cell is a hippocampal cell such as a CA1 cell, a CA3 cell or a DG cell.

9. A method of identifying a compound useful in the treatment of cognitive impairment comprising:
(a) determining the abundance or function of an expressed gene product, in the hippocampus of a mammal, of the mammal's homologue of a gene or a plurality of genes identified by the method of claim 1 to increase in abundance in the AI population relative to the combined AU and Y populations or to decrease in abundance in the AU population relative to the combined AI and Y populations or a gene or genes selected from the group consisting of each of the individual genes listed in Tables 1, 3, 5, 8, 9, 11, 13, 16, 17, 19, 21, 24, 25, 27, 29, 32, 33, 35, 37, 40, 41, 43, 45 and 48 in the presence or absence of a candidate compound; and
(b) identifying a compound from any of the candidate compound(s) that significantly decreases that abundance or attenuates the function of that gene or its expressed gene product in the mammal to whom the candidate compound is administered;
preferably wherein the mammal is an aged mammal and optionally wherein the aged mammal is cognitively impaired.

10. A method of identifying a compound useful in the treatment of cognitive impairment comprising:
(a) determining the abundance or function of an expressed gene product, in the hippocampus of a mammal, of the mammal's homologue of a gene or a plurality of genes identified by the method of claim 1 to decrease in abundance in the AI population relative to the combined AU and Y populations or to increase in abundance in the AU population relative to the combined AI and Y populations or a gene or genes selected from the group consisting of each of the individual genes listed in Tables 2, 4, 6, 7, 10, 12, 14, 15, 18, 20, 22, 23, 26, 28, 30, 31, 34, 36, 38, 39, 42, 44, 46 and 47 and in the presence or absence of a candidate compound; and
(b) identifying a compound from any of the candidate compound(s) that significantly increases that abundance or enhances the function of that gene or its expressed gene product in the mammal to whom the candidate compound is administered;
preferably wherein the mammal is an aged mammal and optionally wherein the aged mammal is cognitively impaired.

11. A method of identifying a compound useful in the treatment of cognitive impairment in aging comprising:
(a) determining the abundance or function of an expressed gene product, in the hippocampus of an aged mammal with cognitive impairment, of the mammal's homologue of a gene or a plurality of genes identified by the method of claim 1 to increase in abundance in the AI population relative to the combined AU and Y populations or to decrease in abundance in the AU population relative to the combined AI and Y populations or a gene or genes selected from the group consisting of each of the individual genes listed in Tables 1, 3, 5, 8, 9, 11, 13, 16, 17, 19, 21, 24, 25, 27, 29, 32, 33, 35, 37, 40, 41, 43, 45 and 48 in the presence or absence of a candidate compound; and
(b) identifying a compound from among the candidate compound(s) that significantly decreases that abundance or attenuates the function of that gene or its expressed gene product in the aged mammal with cognitive impairment to whom the compound is administered relative to a member of the group consisting of an aged mammal without cognitive impairment, a young mammal, an aged cognitively impaired mammal in the absence of the compound, and two or more of them;
preferably wherein the compound does not significantly alter that abundance or function in a member of the group consisting of a young mammal, an aged cognitively unimpaired mammal and both, to whom the candidate compound is administered.

12. A method of identifying a compound useful in the treatment of cognitive impairment in aging comprising:
(a) determining the abundance or function of an expressed gene product, in the hippocampus of an aged mammal with cognitive impairment, of the mammal's homologue of a gene or a plurality of genes identified by the method of claim 1 to decrease in abundance in the AI population relative to the combined AU and Y populations or to increase in abundance in the AU population relative to the combined AI and Y populations or a gene or genes selected from the group consisting of each of the individual genes listed in Tables 2, 4, 6, 7, 10, 12, 14, 15, 18, 20, 22, 23, 26, 28, 30, 31, 34, 36, 38, 39, 42, 44, 46 and 47 in the presence or absence of a candidate compound; and
(b) identifying a compound from among the candidate compound(s) that significantly increases that abundance or enhances the function of that gene or its expressed gene product in the aged mammal with cognitive impairment to whom the compound is administered relative to a member of the group consisting of an aged mammal without cognitive impairment, a young mammal, an aged cognitively impaired mammal in the absence of the compound, and two or more of them;
preferably wherein the compound does not significantly alter that abundance or function in a member of the group consisting of a young mammal, an aged cognitively unimpaired mammal and both, to whom the candidate compound is administered.

13. A method of identifying a compound useful in the treatment of cognitive impairment comprising:
(a) determining the cognitive status of a mammal in the presence or absence of a candidate compound believed to decrease the abundance or attenuate the function of an expressed gene product of a gene or a plurality of genes identified by the method of claim 1 to increase in abundance in the AI population relative to the combined AU and Y populations or to decrease in abundance in the AU population relative to the combined AI and Y populations or a gene or genes selected from the group consisting of each of the individual genes listed in Tables 1, 3, 5, 8, 9, 11, 13, 16, 17, 19, 21, 24, 25, 27, 29, 32, 33, 35, 37, 40, 41, 43, 45 and 48; and
(b) identifying the compound from among the candidate compound(s) that beneficially alters the cognitive status of the mammal to whom the compound is administered;
preferably wherein the mammal is an aged mammal and optionally wherein the aged mammal is cognitively impaired.

14. A method of identifying a compound useful in the treatment of cognitive impairment comprising:
(a) determining the cognitive status of a mammal in the presence or absence of a candidate compound believed to increase the abundance or enhance the function of an expressed gene product of a gene or a plurality of genes identified by the method of claim 1 to decrease in abundance in the AI population relative to the combined AU and Y populations or to increase in abundance in the AU population relative to the combined AI and Y populations or a gene or genes selected from the group consisting of each of the individual genes listed in Tables 2, 4, 6, 7, 10, 12, 14, 15, 18, 20, 22, 23, 26, 28, 30, 31, 34, 36, 38, 39, 42, 44, 46 and 47; and
(b) identifying the compound from among the candidate compound(s) that beneficially alters the cognitive status of the mammal to whom the compound is administered;
preferably wherein the mammal is an aged mammal and optionally wherein the aged mammal is cognitively impaired.

15. A method of identifying a compound useful in the treatment of cognitive impairment in aging comprising:
(a) determining the cognitive status of an aged mammal with cognitive impairment, in the presence or absence of a candidate compound believed to decrease the abundance or attenuate the function of an expressed gene product of a gene or a plurality of genes identified by the method of claim 1 to increase in abundance in the AI population relative to the combined AU and Y populations or to decrease in abundance in the AU population relative to the combined AI and Y populations or a gene or genes selected from the group consisting of each of the individual genes listed in Tables 1, 3, 5, 8, 9, 11, 13, 16, 17, 19, 21, 24, 25, 27, 29, 32, 33, 35, 37, 40, 41, 43, 45 and 48; and
(b) identifying a compound from among the candidate compound(s) that beneficially alters the cognitive function in the aged mammal with cognitive impairment to whom the compound is administered relative to a member of the group consisting of an aged mammal without cognitive impairment, a young mammal, an aged cognitively impaired mammal to whom the compound is not administered, and two or more of them;
preferably wherein the compound does not significantly alter the cognitive status of a member of the group consisting of a young mammal, an aged cognitively unimpaired mammal, and both, to whom the compound is administered.

16. A method of identifying a compound useful in the treatment of cognitive impairment in aging comprising:
(a) determining the cognitive status of an aged mammal with cognitive impairment, in the presence or absence of a candidate compound believed to increase the abundance or enhance the function of an expressed gene product of a gene or a plurality of genes identified by the method of claim 1 to decrease in abundance in the AI population relative to the combined AU and Y populations or to increase in abundance in the AU population relative to the combined AI and Y populations or a gene or genes selected from the group consisting of each of the individual genes listed in Tables 2, 4, 6, 7, 10, 12, 14, 15, 18, 20, 22, 23, 26, 28, 30, 31, 34, 36, 38, 39, 42, 44, 46 and 47; and
(b) identifying a compound from among the candidate compound(s) that beneficially alters the cognitive function in the aged mammal with cognitive impairment to whom the compound is administered relative to a member of the group consisting of an aged mammal without cognitive impairment, a young mammal, an aged cognitively impaired mammal to whom the compound is not administered, and two or more of them;
preferably wherein the compound does not significantly alter the cognitive status of a member of the group consisting of a young mammal, an aged cognitively unimpaired mammal, and both, to whom the compound is administered.

17. The method according to any one of claims 13 to 16, wherein cognitive function is hippocampal-dependent function; preferably wherein the hippocampal-dependent function is selected from the group consisting of spatial memory acquisition, long-term spatial memory, and spatial memory retrieval.

18. The method according to any one of claims 1 to 17, wherein the mammal is an outbred strain of rat; or wherein the mammal is a human.

19. The method according to any one of claims 10 to 12, wherein the abundance is determined in CA1, CA3 or DG hippocampal tissue.

20. The method according to claim 1, wherein the identified gene(s) are selected from the group consisting of each of the individual genes listed in Tables 1-48; and/or claim 1, wherein the identified gene(s) are selected from the group consisting of each of the individual genes listed in Tables 1, 2, 5, 6, 9, 10, 13, 14, 17, 18, 21, 22, 25, 26, 29, 30, 33, 34, 37, 38, 41, 42, 45 and 46.

21. The method according to any one of claims 7, 10, 12, 14 and 16 wherein the expressed gene roduct is the product of the GABA-A α5 receptor gene (GENBANK® accession number NM_017295) or its homologues.

22. A GABA-A α5 receptor agonist for use in the treatment of cognitive impairment in a mammal in need of such treatment; preferably wherein the mammal is a human.

23. A pharmaceutical composition for treating cognitive impairment, the composition comprising pharmaceutically effective amount of a GABAA α5 receptor agonist and a pharmaceutically acceptable carrier.

24. The GABA-A α5 receptor agonist for use according to claim 22 or the pharmaceutical composition according to claim 23, wherein the agonist is selected from the group consisting of 1-methyl-7-acetyleno-5-phenyl-1,3- dihydro-benzo[e]-1,4-diazepin-2-one, S(CH₂)₃0H, and 8-ethylthio-3-methyl-5-(1-oxidopyridin-2-yl)-3,4- dihydronaphthalen-1(2H)-one.


SEQUENCE LISTING

<110> THE JOHNS HOPKINS UNIVERSITY

<120> METHODS FOR CHARACTERIZING AND TREATING COGNITIVE IMPAIRMENT IN
      AGING AND DISEASE

<130> P32019EP-D1-PCT

<140> Not yet assigned
<141> 2006-08-03

<150> EP06800760.8
<151> 2006-08-03

<150> US 60/705,642
<151> 2005-08-03

<150> US 60/705,560
<151> 2005-08-03

<150> US 60/705,559
<151> 2005-08-03

<150> US 60/705,702
<151> 2005-08-03

<150> US 60/705,691
<151> 2005-08-03

<150> US 60/705,686
<151> 2005-08-03

<150> US 60/705,700
<151> 2005-08-03

<150> US 60/705,698
<151> 2005-08-03

<150> US 60/705,662
<151> 2005-08-03

<150> US 60/705,697
<151> 2005-08-03

<150> US 60/705,659
<151> 2005-08-03

<150> US 60/705,683
<151> 2005-08-03

<150> US 60/705,699
<151> 2005-08-03

<150> US 60/705,417
<151> 2005-08-03

<150> US 60/705,418
<151> 2005-08-03

<150> US 60/705,511
<151> 2005-08-03

<150> US 60/705,701
<151> 2005-08-03

<150> US 60/705,661
<151> 2005-08-03

<150> US 60/705,594
<151> 2005-08-03

<150> US 60/705,695
<151> 2005-08-03

<150> US 60/705,512
<151> 2005-08-03

<150> US 60/705,416
<151> 2005-08-03

<150> US 60/705,591
<151> 2005-08-03

<150> US 60/705,703
<151> 2005-08-03

<160> 3

<170> PatentIn version 3.5

<210> 1
<211> 2564
<212> DNA
<213> Rattus norvegicus

<400> 1
cagccggccg ctcagggggc taaggaggtc tggagggctc ggcgcgactg agggccggac 60

cactagaggt atcgggtggc gctgcggcag cagcaggtgc gggtggcgga ggctgcggct 120

ccagtgccat cccttattcc acctgcgctg tcagcatgca ccttgcagaa ccagaagcag 180

cttgctggct ttgaacgtgt ggcaaatatt tcagaaagct tcaagatcaa gttggaggaa 240

aggacggttt ttcttctaaa ttcatctgct tcaactatta ttcttactgg gaatggacaa 300

tggaatgctc tctagattta tcatgaccaa aacgctcctt gtcttctgca tttccatgac 360

cttatccagt cactttggct tttcacaaat gccaactagt tctgtacaag atgagaccaa 420

tgacaacatc acaatattca ccaggatctt ggacgggctc ttggatggct atgacaacag 480

actgcggcct gggctgggag agcgaatcac gcaggtgcga acagacatct atgttaccag 540

ctttggccca gtgtccgaca cggaaatgga atataccata gatgtatttt tccgtcaaag 600

ctggaaagat gaaaggctgc ggtttaaggg gcctatgcaa cgtctccctc tcaacaacct 660

tcttgccagg aaaatctgga ccccagacac attcttccac aatgggaaga agtccattgc 720

gcacaacatg acgacaccca acaagctgct gaggctggag gatgatggca cacttctcta 780

caccatgcgc ctgacgatct ctgctgagtg tccaatgcag cttgaggact ttccgatgga 840

tgcccatgct tgtcccctga aatttggcag ttatgcttac cctaattcgg aagttgtcta 900

tgtttggacc aatggttcca ccaagtctgt ggtggtggca gaagatggct ccagactcaa 960

ccagtaccac ctcatggggc agacagtagg cactgagaac atcagcacca gcacaggtga 1020

atatacaatc atgactgctc attttcacct gaagaggaag atcgggtact ttgtcatcca 1080

gacgtacctt ccctgcatca tgacagtcat cttatcccag gtgtcttttt ggcttaatcg 1140

agaatctgtc ccagctagga cagtttttgg agtgaccaca gtgctgacca tgacaaccct 1200

cagcatcagt gcccggaatt cgctgcccaa agtggcctat gccacagcca tggactggtt 1260

cattgctgtc tgctatgcat ttgtcttctc tgccctgatt gaatttgcca cagtcaacta 1320

ctttacaaag agaggatggg cctgggatgg caagaaggcc ttggaagcag ctaaaatcaa 1380

gaaaaaagaa cgtgaactca tactaaataa gtcaacaaat gcttttacaa ctgggaagtt 1440

gacccatcct ccaaacatcc caaaggagca gcttccaggc gggactggga atgctgtggg 1500

tacagcctca atcagagcat ctgaggagaa gacttctgag agtaaaaaga cctacaacag 1560

catcagcaag atcgacaaaa tgtcccggat tgtgttcccc attttgtttg gcactttcaa 1620

tctagtttac tgggcaacat atttgaatag ggagcccgtg ataaaagggg ctacctctcc 1680

aaagtaagac aggaaaccat acttgcacag aaatgaacct gaggagaggt caagctcaca 1740

gagactattt gggcgcctgt ctttcaggaa attttgcatg tttaataata tgtacaaata 1800

atattgcctt gatgtttcta tgtgtaactt caatgtttca aggatgtccc ttaataaacc 1860

aagcaaatgg ccttctacaa caacgggagg caatgactga ctctcagatg ctcagcgtcc 1920

taacatcaat agtttacaaa caagataagt atatttttaa ctgttctggt atatgacgtt 1980

tttatacttc gaatgccatt tcgtaccatt tttcccagcc aacagaacat tttagggaat 2040

ccctgtgatg accacttgac aggtgaaaaa gcaaagatcc tcgggtacac aaagtccatg 2100

aagagcaaac tgtggacatt taagtccagt acgaattgcc tttaacaatt cttcttgttc 2160

tgaaattaga aaaatactgc atgaactgac attaagaagt agataagcaa acatttatgc 2220

agacaaattt aatgacaagc ccatagtgtc ttagattagt agatcaaata attccccaag 2280

gaaaagaaat caactgattc aaaattaatt ttgttgtttt ttgtgaaaaa tgaatttatt 2340

tctcaccccg ccccaacacc tttacaacct taataatgac taagaaagca aaatcttaaa 2400

accttaaagc aacaaggcct tggtctttgg tggtggtatt ctgtggccat tgtttctgac 2460

cctgggtcct cttgctgctg cttcagcgct gagaaattgt aattgagtta ttttctgttt 2520

tatttccctg tacatatttc atggttggat tatcgctctg ttag 2564

<210> 2
<211> 464
<212> PRT
<213> Rattus norvegicus

<400> 2
Met Asp Asn Gly Met Leu Ser Arg Phe Ile Met Thr Lys Thr Leu Leu
1               5                   10                  15

Val Phe Cys Ile Ser Met Thr Leu Ser Ser His Phe Gly Phe Ser Gln
            20                  25                  30

Met Pro Thr Ser Ser Val Gln Asp Glu Thr Asn Asp Asn Ile Thr Ile
        35                  40                  45

Phe Thr Arg Ile Leu Asp Gly Leu Leu Asp Gly Tyr Asp Asn Arg Leu
    50                  55                  60

Arg Pro Gly Leu Gly Glu Arg Ile Thr Gln Val Arg Thr Asp Ile Tyr

65                  70                  75                  80

Val Thr Ser Phe Gly Pro Val Ser Asp Thr Glu Met Glu Tyr Thr Ile
                85                  90                  95

Asp Val Phe Phe Arg Gln Ser Trp Lys Asp Glu Arg Leu Arg Phe Lys
            100                 105                 110

Gly Pro Met Gln Arg Leu Pro Leu Asn Asn Leu Leu Ala Arg Lys Ile
        115                 120                 125

Trp Thr Pro Asp Thr Phe Phe His Asn Gly Lys Lys Ser Ile Ala His
    130                 135                 140

Asn Met Thr Thr Pro Asn Lys Leu Leu Arg Leu Glu Asp Asp Gly Thr
145                 150                 155                 160

Leu Leu Tyr Thr Met Arg Leu Thr Ile Ser Ala Glu Cys Pro Met Gln
                165                 170                 175

Leu Glu Asp Phe Pro Met Asp Ala His Ala Cys Pro Leu Lys Phe Gly
            180                 185                 190

Ser Tyr Ala Tyr Pro Asn Ser Glu Val Val Tyr Val Trp Thr Asn Gly
        195                 200                 205

Ser Thr Lys Ser Val Val Val Ala Glu Asp Gly Ser Arg Leu Asn Gln
    210                 215                 220

Tyr His Leu Met Gly Gln Thr Val Gly Thr Glu Asn Ile Ser Thr Ser
225                 230                 235                 240

Thr Gly Glu Tyr Thr Ile Met Thr Ala His Phe His Leu Lys Arg Lys
                245                 250                 255

Ile Gly Tyr Phe Val Ile Gln Thr Tyr Leu Pro Cys Ile Met Thr Val
            260                 265                 270

Ile Leu Ser Gln Val Ser Phe Trp Leu Asn Arg Glu Ser Val Pro Ala
        275                 280                 285

Arg Thr Val Phe Gly Val Thr Thr Val Leu Thr Met Thr Thr Leu Ser
    290                 295                 300

Ile Ser Ala Arg Asn Ser Leu Pro Lys Val Ala Tyr Ala Thr Ala Met
305                 310                 315                 320

Asp Trp Phe Ile Ala Val Cys Tyr Ala Phe Val Phe Ser Ala Leu Ile
                325                 330                 335

Glu Phe Ala Thr Val Asn Tyr Phe Thr Lys Arg Gly Trp Ala Trp Asp
            340                 345                 350

Gly Lys Lys Ala Leu Glu Ala Ala Lys Ile Lys Lys Lys Glu Arg Glu
        355                 360                 365

Leu Ile Leu Asn Lys Ser Thr Asn Ala Phe Thr Thr Gly Lys Leu Thr
    370                 375                 380

His Pro Pro Asn Ile Pro Lys Glu Gln Leu Pro Gly Gly Thr Gly Asn
385                 390                 395                 400

Ala Val Gly Thr Ala Ser Ile Arg Ala Ser Glu Glu Lys Thr Ser Glu
                405                 410                 415

Ser Lys Lys Thr Tyr Asn Ser Ile Ser Lys Ile Asp Lys Met Ser Arg
            420                 425                 430

Ile Val Phe Pro Ile Leu Phe Gly Thr Phe Asn Leu Val Tyr Trp Ala
        435                 440                 445

Thr Tyr Leu Asn Arg Glu Pro Val Ile Lys Gly Ala Thr Ser Pro Lys
    450                 455                 460

<210> 3
<211> 24
<212> DNA
<213> Artificial Sequence

<220>
<223> Description of Artificial Sequence: Synthetic
      oligonucleotide

<400> 3
tttttttttt tttttttttt tttt                                              24